# EUROPEAN PATENT APPLICATION

(11) **EP 1 529 778 A1**
(43) Date of publication of application: **11.05.2005**
(21) Application number: 04256810.5
(22) Date of filing: 04.11.2004
(51) Int. Cl.: C07D 263/42, C07D 239/46, C07D 403/12, C07F 7/18

(54) **Production method of pyrimidine derivative, intermediate therefor**

(30) Priority: 04.11.2003 JP 2003374045
(71) Applicant: Ajinomoto Co., Inc., Tokyo 104-8315 (JP)
(72) Inventor: Takahashi, Daisuke c/o Ajinomoto Co, Inc, Kawasaki-shi Kanagawa 210-8681 (JP); Izawa, Kunisuke c/o Ajinomoto Co, Inc, Kawasaki-shi Kanagawa 210-8681 (JP)
(74) Representative: Nicholls, Kathryn Margaret

(57) **Abstract**

The present invention relates to a production method of compound (XV), which includes hydrolysis of compound (I) to give compound (II), then reaction with reagent (III) to give compound (IV), then reaction with compound (V) to give compound (VI), then condensation with compound (XII) to give compound (XI), and preferably deprotection by an enzyme reaction. This method is an advantageous production method of a pyrimidine derivative and a synthetic intermediate useful as an enzyme inhibitor. 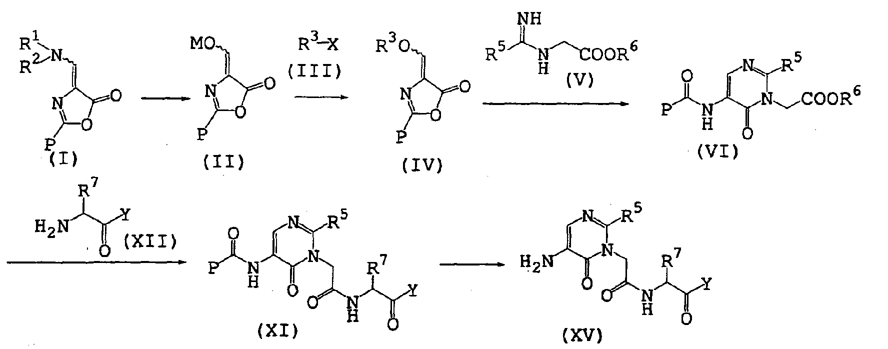 wherein P is an alkyl group and the like, M is sodium and the like, R¹ and R² are each an alkyl group and the like, R³ is an alkyl group optionally having substituent(s) and the like, X is a halogen atom and the like, R⁵ is an alkyl group optionally having substituent(s) and the like, R⁶ is a hydrogen atom and the like, R⁷ is an aralkyl group optionally having substituent(s) and the like, and Y is a heteroaryl group optionally having substituents and the like.

## Description

### TECHNICAL FIELD OF THE INVENTION

The present invention relates to a novel production method of a pyrimidine derivative useful as an enzyme inhibitor such as an elastase inhibitor, a chymase inhibitor and the like. More particularly, the present invention relates to a novel intermediate to be used in the production method and a production method thereof.

### BACKGROUND OF THE INVENTION

A group of compounds represented by the formula (XVI): 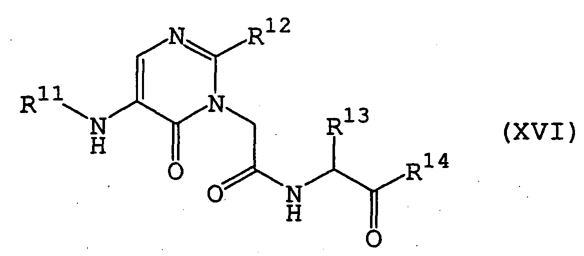 wherein R¹¹ is a hydrogen atom, an acyl group, a formyl group and the like, R¹² is a phenyl group optionally having substituent(s), an alkyl group and the like, R¹³ is an alkyl group optionally having substituent(s), an aryl group optionally having substituent(s), an aralkyl group optionally having substituent(s) and the like, and R¹⁴ is a heterocyclic group optionally having substituent(s), an acyl group, a haloalkyl group and the like have been reported to be useful as a pharmaceutical product, particularly an enzyme inhibitor such as an elastase inhibitor, a chymase inhibitor and the like for the prophylaxis or treatment of rheumatism, a chronic obstructive pulmonary disease and the like (WO98/2480 and WO98/09949).

As a production method of a compound represented by the formula (6), which is one of the compounds represented by the formula (XVI), a method depicted in the following reaction scheme has been reported (WO02/051815). 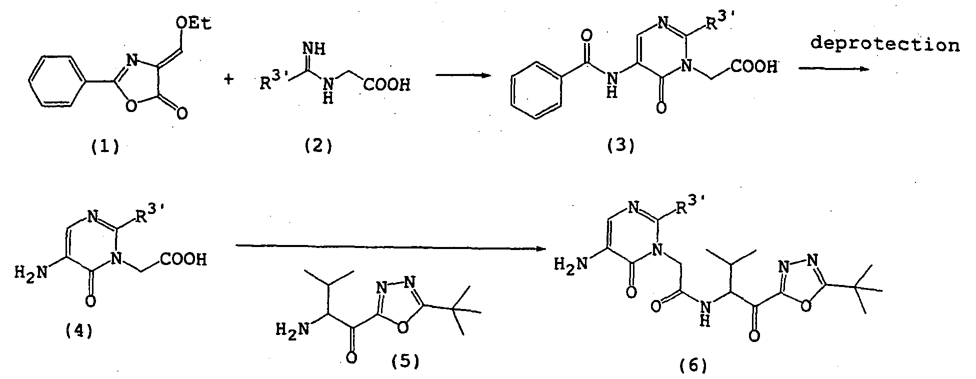 wherein R³' is (1) C1-4 alkyl group, (2) Cycl or (3) C1-4 alkyl group substituted by Cycl (wherein Cycl is a C3-10 monocyclic or bicyclic carbon ring, a 3- to 10-membered monocyclic or bicyclic heterocycle containing 1 to 4 nitrogen atoms, 1 or 2 oxygen atoms and/or 1 or 2 sulfur atoms, etc.).

This method is associated with a problem of decreased yield and degraded quality of the object compound represented by the formula (6), because, when a compound represented by the formula (4) and a compound represented by the formula (5) are subjected to an amidation reaction, a side reaction of an amidation reaction with itself to produce a dimer of the compound represented by the formula (4) occurs due to the presence of an amino group at the 5-position of a pyrimidine ring of the compound represented by the formula (4).

In the above-mentioned method, the production of a dimer can be avoided when the amidation reaction of a compound represented by the formula (3) with a compound represented by the formula (5) is carried out without deprotection of a benzoyl group of a compound represented by the formula (3) and the benzoyl group is thereafter deprotected. However, deprotection of the benzoyl group after the amidation reaction requires severe conditions involving a strong base and refluxing for a long time (WO02/051815, Example 4 etc.). Under such conditions, a pyrimidine compound represented by the formula (6) cannot be obtained easily since 1,3,4-oxadiazole ring in a compound represented by the formula (6) is decomposed. To avoid dimerization reaction in the above-mentioned method, therefore, protection of an amino group of a compound represented by the formula (4) in advance with a protecting group capable of being deprotected under mild conditions may be performed. However, addition of further steps of protection and deprotection that have become necessary is not preferable.

In protection of amino group by acylation, it is generally known that hydrolysis of aliphatic acyl such as acetyl can proceed better under mild conditions than aromatic acyl such as benzoyl (Theodora W. Grrene, Peter G. M. Wuts, Protective Groups in Organic Synthesis, 2nd edn., A Wiley Interscience Publication, 1991, p. 349). Therefore, if a compound represented by the formula (1), wherein the 2-position of an azlactone ring is an aliphatic group, can be obtained as a starting material, a compound represented by the formula (3), wherein the 5-position amino group is protected by an aliphatic acyl group, can be obtained. Therefore, the compound may be amidated with a compound represented by the formula (5) without deprotecting the aliphatic acyl group, and deprotected under milder conditions free of decomposition.

However, an efficient production method of a compound represented by the formula (1), wherein the 2-position of an azlactone ring is an aliphatic group, has not been reported yet.

The present inventors have tried synthesis of a compound represented by the formula (8), wherein the 2-position of an azlactone ring is a methyl group, using the Erlenmeyer method shown in the following reaction scheme known as a general production method of azlactone compounds.

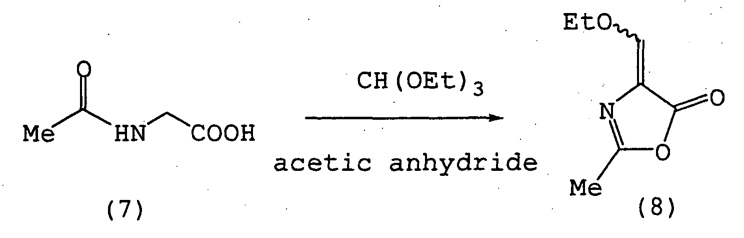

However, this method allowed generation of a lot of by-products, and an azlactone compound represented by the formula (8) was obtained only in an extremely low yield.

### SUMMARY OF THE INVENTION

The problem to be soled by the present invention is provision of an advantageous production method of a pyrimidine derivative represented by the following formula (XV) or an N-protected form thereof (e.g., N-formyl form, see WO98/09949), which are useful as an enzyme inhibitor. Another object is to provide a novel synthetic intermediate necessary for the production method and a production method thereof.

The present inventors have conducted intensive studies in an attempt to solve the above-mentioned problems and found a method of advantageously producing a novel compound represented by the following formula (IV), and provided a novel and advantageous production method using the compound as a starting material or an intermediate compound, a pyrimidine derivative represented by the formula (XV) or an N-protected form thereof.

Therefore, the present invention provides the following.
(1) A production method of a compound represented by the formula (IV): 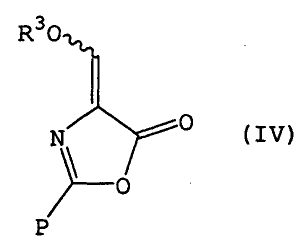 wherein P is a hydrogen atom, an alkyl group, an alkenyl group, an aralkyl group optionally having substituent(s) or a haloalkyl group and R³ is an alkyl group optionally having substituent(s), an aryl group optionally having substituent(s), an aralkyl group optionally having substituent(s), a trialkylsilyl group, -COR^{4a} or -SO₂R^{4b} wherein R^{4a} and R^{4b} are the same or different and each independently is an alkyl group optionally having substituent(s) or an aryl group optionally having substituent(s) (hereinafter to be also referred to as compound (IV)),
   which comprises hydrolyzing a compound represented by the formula (I): 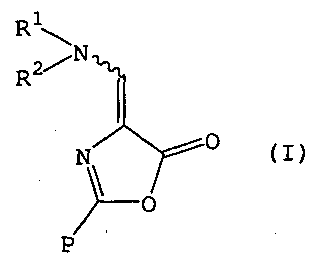 wherein R¹ and R² are the same or different and each is an alkyl group or optionally form an aliphatic heterocycle together with the adjacent nitrogen atom, a wavy line shows a cis form, a trans form or a mixture thereof and P is as defined above (hereinafter to be also referred to as compound (I)), or a salt thereof, in the presence of an alkali metal hydroxide to give a compound represented by the formula (II): 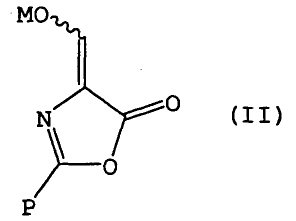 wherein M is a hydrogen atom, sodium, potassium or lithium and P and a wavy line are as defined above (hereinafter to be also referred to as compound (II)); and reacting the obtained compound (II) with a reagent represented by the formula: R³-X (III) wherein R³ is as defined above and X is a halogen atom, -OSO₃R⁴ or -OCOR^{4a}, wherein R⁴ is an alkyl group optionally having substituent(s) or an aryl group optionally having substituent(s) and R^{4a} is as defined above (hereinafter to be also referred to as reagent (III)).
(2) A production method of a compound represented by the formula (VI): 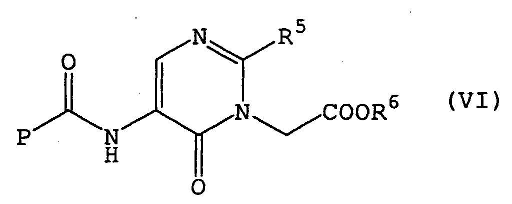 wherein R⁵ is an alkyl group optionally having substituent(s) or a phenyl group optionally having substituent(s), R⁶ is a hydrogen atom, an alkyl group or an aralkyl group and P is as defined above (hereinafter to be also referred to as compound (VI)), or a salt thereof, which comprises the following Steps (a) , (b) and (c):
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with a compound represented by the formula (V): 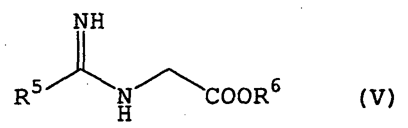 wherein each symbol is as defined above (hereinafter to be also referred to as compound (V)), or a salt thereof, to give compound (VI) or a salt thereof.
(3) A production method of a compound represented by the formula (VII) : 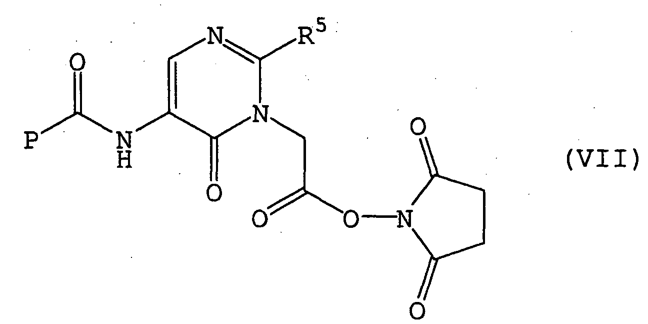 wherein each symbol is as defined above (hereinafter to be also referred to as compound (VII)), which comprises the following Steps (a) , (b) , (c) and (d) ;
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof:
   Step (d): when R⁶ of the obtained compound (VI) is other than a hydrogen atom, converting R⁶ to a hydrogen atom, and condensing the compound or a salt thereof with N-hydroxysuccinimide to give compound (VII).
(4) The production method of any of the above-mentioned (1)-(3), wherein P is a methyl group, an ethyl group or a benzyl group.
(5) The production method of any of the above-mentioned (1)-(3), wherein R³ is a methyl group, an ethyl group, a benzyl group, a methanesulfonyl group or a trimethylsilyl group.
(6) A production method of a compound represented by the formula (IX): 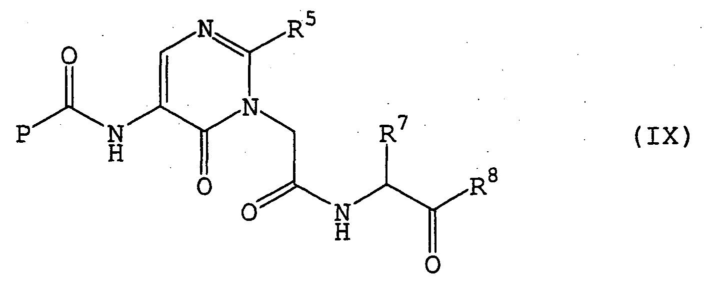 wherein R⁷ is a hydrogen atom, an alkyl group optionally having substituent(s), an aralkyl group optionally having substituent(s) or an aryl group optionally having substituent(s), R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, and other symbols are as defined above (hereinafter to be also referred to as compound (IX)), or a salt thereof, which comprises the following Steps (a), (b), (c), (d) and (e);
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof;
   Step (d): when R⁶ of the obtained compound (VI) is other than a hydrogen atom, converting R⁶ to a hydrogen atom, and condensing the compound or a salt thereof with N-hydroxysuccinimide to give compound (VII);
   Step (e): reacting the obtained compound (VII) with a compound represented by the formula (VIII): 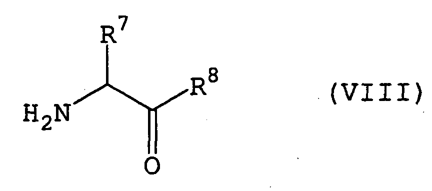 wherein each symbol is as defined above (hereinafter to be also referred to as compound (VIII)), or a salt thereof, to give compound (IX) or a salt thereof.
(7) A production method of compound (IX) or a salt thereof, which comprises the following Steps (a), (b), (c) and (f);
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof;
   Step (f): when R⁶ of the obtained compound (VI) is other than a hydrogen atom, converting R⁶ to a hydrogen atom, and condensing the compound or a salt thereof with compound (VIII) or a salt thereof to give compound (IX) or a salt thereof.
(8) The production method of the above-mentioned (6) or (7), wherein P is a methyl group, an ethyl group or a benzyl group.
(9) The production method of the above-mentioned (6) or (7), wherein R³ is a methyl group, an ethyl group, a benzyl group, a methanesulfonyl group or a trimethylsilyl group.
(10) A production method of a compound represented by the formula (X): 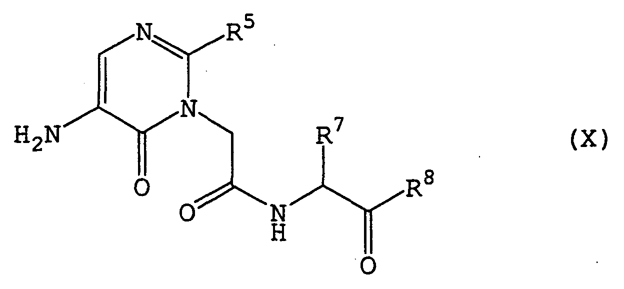 wherein each symbol is as defined above (hereinafter to be also referred to as compound (X)), or a salt thereof, which comprises deprotecting the obtained compound (IX) or a salt thereof by the production method of any of the above-mentioned (6)-(9).
(11) The production method of the above-mentioned (10), which comprises deprotection by an enzyme reaction.
(12) The production method of the above-mentioned (10), which comprises deprotection under acidic conditions.
(13) A production method of a compound represented by the formula (XV): 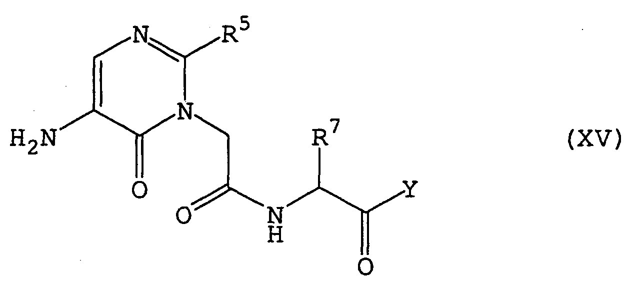 wherein Y is a heterocyclic group optionally having substituent(s), an acyl group optionally having substituent(s) or a haloalkyl group and other symbols are as defined above (hereinafter to be also referred to as compound (XV)) or a salt thereof,
   which comprises the following Steps (j), (k) and (1);
   Step (j): protecting an amino group of the obtained compound (X) or a salt thereof by a method of any of the above-mentioned (10)-(12) to give a compound represented by the formula (XIII): 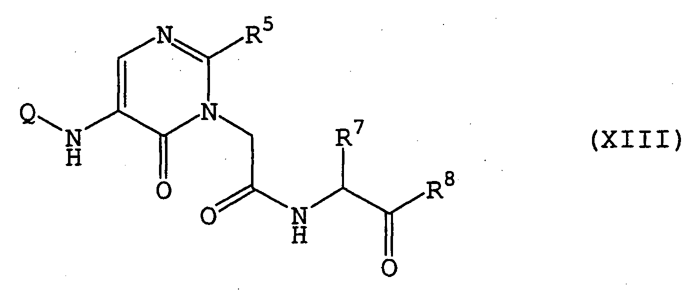 wherein Q is an amino-protecting group other than an acyl group and other symbols are as defined above (hereinafter to be also referred to as compound (XIII)) or a salt thereof;
   Step (k): converting a group represented by R⁸ of the obtained compound (XIII) or a salt thereof to a group represented by Y to give a compound represented by the formula (XIV): 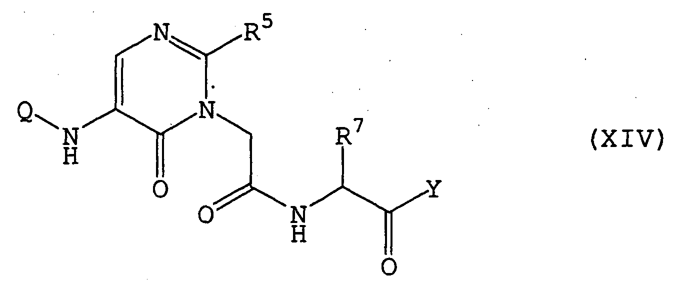 wherein each symbol is as defined above (hereinafter to be also referred to as compound (XIV)) or a salt thereof;
   Step (1): deprotecting the obtained compound (XIV) or a salt thereof to give compound (XV) or a salt thereof.
(14) A production method of a compound represented by the formula (XI): 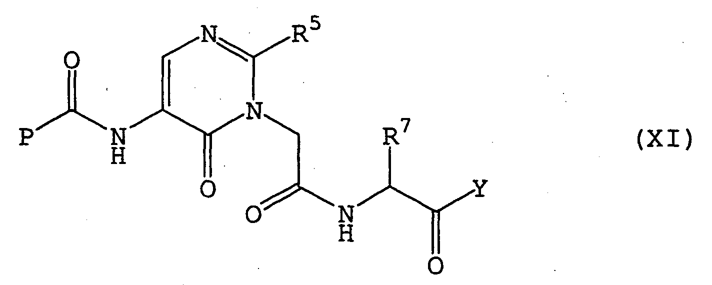 wherein each symbol is as defined above (hereinafter to be also referred to as compound (XI)), or a salt thereof, which comprises the following Steps (a), (b), (c), (d), (e) and (g);
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof;
   Step (d): when R⁶ of the obtained compound (VI) is other than a hydrogen atom, converting R⁶ to a hydrogen atom, and condensing the compound or a salt thereof with N-hydroxysuccinimide to give compound (VII);
   Step (e): reacting the obtained compound (VII) with compound (VIII) or a salt thereof to give compound (IX) or a salt thereof;
   Step (g): converting a group represented by R⁸ of the obtained compound (IX) or a salt thereof to a group represented by Y to give compound (XI) or a salt thereof.
(15) A production method of compound (XI) or a salt thereof, which comprises the following Steps (a), (b), (c), (f) and (g);
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof;
   Step (f): when R⁶ of the obtained compound (VI) is other than a hydrogen atom, converting R⁶ to a hydrogen atom, and condensing the compound or a salt thereof with compound (VIII) or a salt thereof to give compound (IX) or a salt thereof;
   Step (g): converting a group represented by R⁸ of the obtained compound (IX) or a salt thereof to a group represented by Y to give compound (XI) or a salt thereof.
(16) A production method of compound (XI) or a salt thereof, which comprises the following Steps (a), (b), (c) and (h);
   Step (a): hydrolyzing compound (I) or a salt thereof in the presence of an alkali metal hydroxide to give compound (II);
   Step (b): reacting the obtained compound (II) with reagent (III) to give compound (IV);
   Step (c): reacting the obtained compound (IV) with compound (V) or a salt thereof to give compound (VI) or a salt thereof;
   Step (h): when R⁶ of the obtained compound (VI) is other than a hydrogen atom, converting R⁶ to a hydrogen atom, and condensing the compound or a salt thereof with a compound of the formula (XII) : 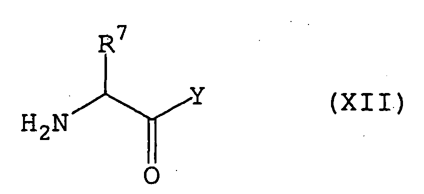 wherein each symbol is as defined above (hereinafter to be also referred to as compound (XII)) or a salt thereof to give compound (XI) or a salt thereof.
(17) The production method of any of the above-mentioned (14)-(16), wherein P is a methyl group, an ethyl group or a benzyl group.
(18) The production method of any of the above-mentioned (14)-(16), wherein R³ is a methyl group, an ethyl group, a benzyl group, a methanesulfonyl group or a trimethylsilyl group.
(19) A production method of compound (XV) or a salt thereof, which comprises deprotecting the obtained compound (XI) or a salt thereof by a production method of any of the above-mentioned (14)-(18).
(20) The production method of the above-mentioned (19), which comprises deprotection by an enzyme reaction.
(21) The production method of the above-mentioned (19), which comprises deprotection under acidic conditions.
(22) A compound represented by the formula (IV'): 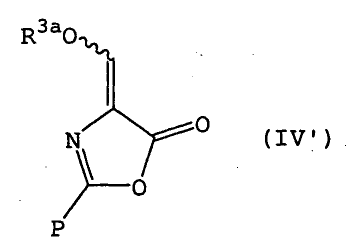 wherein P and a wavy line are as defined above, R^{3a} is an alkyl group optionally having substituent(s), provided that P is not an alkyl group and a benzyl group, an aryl group optionally having substituent(s), an aralkyl group optionally having substituent(s), a trialkylsilyl group, -COR^{4a} or -SO₂R^{4b} wherein R^{4a} and R^{4b} are the same or different and each independently is an alkyl group optionally having substituent(s) or an aryl group optionally having substituent(s) (hereinafter to be also referred to as compound (IV')).
(23) The compound of the above-mentioned (22), wherein P is a methyl group, an ethyl group or a benzyl group and R^{3a} is a methyl group, an ethyl group, a benzyl group, a methanesulfonyl group or a trimethylsilyl group.
(24) A compound represented by the formula (VII): 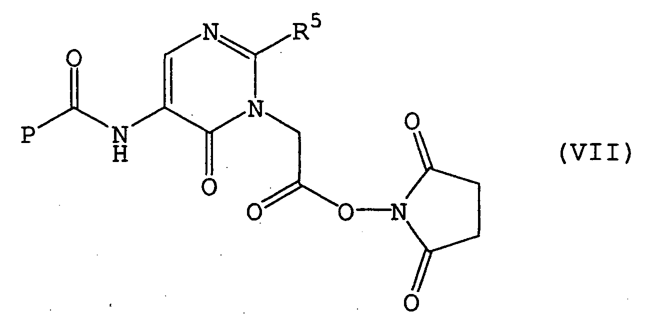 wherein each symbol is as defined above.
(25) The compound of the above-mentioned (24), wherein P is a methyl group, an ethyl group or a benzyl group, and R⁵ is a methyl group or a phenyl group optionally having substituent(s).
(26) A compound represented by the formula (IX): 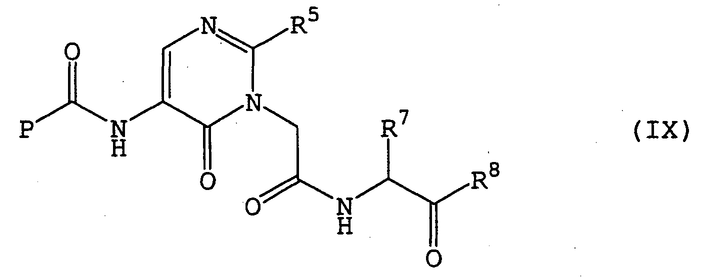 wherein each symbol is as defined above, or a salt thereof.
(27) The compound the above-mentioned (26) wherein P is a methyl group, an ethyl group or a benzyl group, R⁵ is a methyl group or a phenyl group optionally having substituent(s), R⁷ is a methyl group, an isopropyl group or a benzyl group, and R⁸ is a hydrogen atom, a hydroxyl group, a methoxy group, an ethoxy group or -N(Me)-OMe, or a salt thereof.
(28) A compound represented by the formula (X): 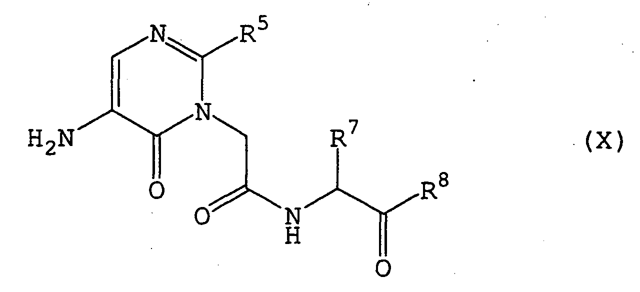 wherein each symbol is as defined above, or a salt thereof.
(29) The compound of the above-mentioned (28), wherein R⁵ is a methyl group or a phenyl group optionally having substituent(s), R⁷ is a methyl group, an isopropyl group or a benzyl group, R⁸ is a hydrogen atom, a hydroxyl group, a methoxy group, an ethoxy group or -N(Me)-OMe, or a salt thereof.
(30) A production method of compound (X) or a salt thereof, which comprises deprotecting compound (IX) or a salt thereof by an enzyme reaction.
(31) A production method of compound (XV) or a salt thereof, which comprises deprotecting compound (XI) or a salt thereof by an enzyme reaction.
(32) The production method of the above-mentioned (30) or (31), wherein P is a benzyl group optionally having substituent(s).
(33) The production method of the above-mentioned (30) or (31), wherein the enzyme is penicillin amidase.

### BRIEF DESCRIPTION OF THE DRAWINGS

Fig. 1 is a chart showing the results of thermal analyses (DSC and TG) of compound of Example 2.
Fig. 2 is a chart showing the results of thermal analyses (DSC and TG) of compound of Example 3.

### DETAILED DESCRIPTION OF THE INVENTION

The present invention is explained in detail in the following.

The definitions of symbols used in the present invention are as follows.

The "alkyl group" for P, R¹, R², R⁶, R⁹ or R¹⁰ is a linear or branched chain alkyl group preferably having 1-20, more preferably 1-7, carbon atoms, such as methyl group, ethyl group, n-propyl group, isopropyl group, n-butyl group, isobutyl group, sec-butyl group, tert-butyl group, lauryl group and the like. Of these, methyl group and ethyl group are preferable.

The "alkyl group optionally having substituent(s)" for R³, R⁴, R^{4a}, R^{4b}, R⁵ or R⁷ is the above-mentioned alkyl group optionally substituted by one or more of the following substituents. As the substituent here, for example, a nitro group, a linear or branched chain alkoxy group (carbon atoms: 1-6, ex.: methoxy group), a halogen atom (ex.: chlorine atom, fluorine atom etc.), a hydroxyl group and the like can be mentioned.

The "alkenyl group" for P is a linear or branched chain alkenyl group preferably having 2-20, more preferably 2-7, carbon atoms, such as vinyl group, allyl group, homoallyl group, oleyl group and the like, with preference given to vinyl group and allyl group.

The "aryl group optionally having substituent(s)" for R³, R⁴, R^{4a}, R^{4b} or R⁷ is an aryl group preferably having 6-20, more preferably 6-8, carbon atoms. The aryl group is optionally substituted by one or more of the following substituents. As the substituent in this context, for example, a nitro group, a linear or branched chain alkoxy group (carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), a hydroxyl group and the like can be mentioned, and as the substituent of the "phenyl group optionally having substituent(s)" for R⁵, similar ones can be mentioned. Specific examples of an aryl group optionally having substituent(s) and a phenyl group optionally having substituent(s) include phenyl group, o-, m- or p-nitrophenyl group, o-, m- or p-methoxyphenyl group, o-, m- or p-chlorophenyl group, o-, m- or p-fluorophenyl group, o-, m- or p-tolyl group and the like, with preference given to phenyl group, p-fluorophenyl group and p-tolyl group.

As the "aryl group optionally having substituent(s)" for R³, 4-nitrophenyl group, 2-nitrophenyl group, 2,4-dinitrophenyl group and the like are preferable.

The "aralkyl group" for R⁶ is an aralkyl group wherein the aryl moiety is an aryl group preferably having 6-12, more preferably 6-8, carbon atoms, and the alkyl moiety is a linear or branched chain alkyl group preferably having 1-6, more preferably 1-3, carbon atoms. Specific examples of aralkyl group preferably include benzyl group.

The "aralkyl group optionally having substituent(s)" for P, R³ or R⁷ is the above-mentioned aralkyl group optionally substituted by one or more of the following substituents, and the substituent in this context includes, for example, a nitro group, a linear or branched chain alkoxy group (carbon number: 1-6, e.g.: methoxy group), a halogen atom (e.g.: chlorine atom, fluorine atom etc.), a hydroxyl group and the like.

The "haloalkyl group" for P and Y is a straight chain or branched chain alkyl group preferably having 1-5, more preferably 1-3, carbon atoms, which is substituted by one or more halogen atoms (fluorine atom, chlorine atom, bromine atom, iodine atom). For example, trifluoromethyl group, trichloromethyl group, chloromethyl group and the like can be mentioned, with preference given to trifluoromethyl group.

The "halogen atom" for X is a chlorine atom, a bromine atom or an iodine atom, with preference given to a chlorine atom and a bromine atom.

As the "aliphatic heterocycle" optionally formed by R¹ and R² together with the adjacent nitrogen atom is a 5- or 6-membered aliphatic heterocycle containing a carbon atom and at least one nitrogen atom, and optionally containing 1 to 3 hetero atoms selected from oxygen atom, sulfur atom and nitrogen atom. For example, pyrrolidine, piperidine, morpholine, thiomorpholine, piperazine and the like can be mentioned.

The "trialkylsilyl group" for R³ is a silyl group substituted by the same or different alkyl groups defined above, such as trimethylsilyl group, tert-butyldimethylsilyl group, triisopropylsilyl group and the like, with preference given to trimethylsilyl group.

The "alkoxy group" for R⁸ is a linear or branched chain alkoxy preferably having 1-20, more preferably 1-7, carbon atoms, such as methoxy group, ethoxy group, n-propoxy group, isopropoxy group, n-butoxy group, isobutoxy group, sec-butoxy group, tert-butoxy group, lauryloxy group and the like. Of these, methoxy group and ethoxy group are preferable.

The "aralkyloxy group" for R⁸ is an aralkyloxy group having the aralkyl moiety defined above, such as benzyloxy group, p-nitrobenzyloxy group and the like.

The "hetero ring group" of the "heterocyclic group optionally having substituent(s)" for Y is a 5- or 6-membered heterocyclic group, containing, besides carbon atom, 1-5 hetero atoms selected from oxygen atom, sulfur atom and nitrogen atom, or a condensed hetero ring group thereof and the like. For example, pyrrolyl group (1-, 2- or 3-pyrrolyl group), furyl group (2- or 3-furyl group), thienyl group (2- or 3-thienyl group), imidazolyl group (1-, 2-, 4- or 5-imidazolyl group), oxazolyl group (2-, 4- or 5-oxazolyl group), thiazolyl group (2-, 4- or 5-thiazolyl group), pyrazolyl group (1-, 3-, 4- or 5-pyrazolyl group), isoxazolyl group (3-, 4- or 5-isoxazolyl group), isothiazolyl group (3-, 4- or 5-isothiazolyl group), oxadiazolyl group (1,2,4-oxadiazol-3 or 5-yl group, 1,3,4-oxadiazol-2-yl group, 1,2,5-oxadiazol-3-yl group), thiadiazolyl group, (1,2,4-thiadiazol-3 or 5-yl group, 1,3,4-thiadiazol-2-yl group, 1,2,5-thiadiazol-3-yl group), triazolyl group (1,2,4-triazol-1, 3, 4 or 5-yl group, 1,2,3-triazol-1, 2 or 4-yl group), indolyl group (1-, 2-, 3-, 4-, 5-, 6- or 7-indolyl group), benzofuryl group (2-, 3-, 4-, 5-, 6- or 7-benzofuryl group), benzothienyl group (2-, 3-, 4-, 5-, 6- or 7-benzothienyl group), benzimidazolyl group (1-, 2-, 4-, 5-, 6-or 7-benzimidazolyl group), benzoxazolyl group (2-, 4-, 5-, 6-or 7-benzoxazolyl group), benzothiazolyl group (2-, 4-, 5-, 6-or 7-benzothiazolyl group), pyridyl group (2-, 3- or 4-pyridyl group), 1-oxidopyridyl group (1-oxido-2-, 3- or 4-pyridyl group), pyrimidinyl group (2-, 4- or 5-pyrimidinyl group), tetrazolyl group (1H-tetrazol-1 or 5-yl group, 2H-tetrazol-2 or 5-yl group), quinolyl group (2-, 3-, 4-, 5-, 6-, 7- or 8-quinolyl group) or isoquinolyl group (1-, 3-, 4-, 5-, 6-, 7- or 8-isoquinolyl group), pyrrolidinyl group (1-, 2- or 3-pyrrolidinyl group), pyrazolidinyl group (1-, 3- or 4-pyrazolidinyl group), imidazolidinyl group (1-, 2- or 4-imidazolidinyl group), pyrrolinyl group (1-, 2- or 3-pyrrolinyl group), pyrazolinyl group (1-, 3- or 4-pyrazolinyl group), imidazolinyl group (1-, 2-, 4- or 5-imidazolinyl group), tetrahydrofuryl group (2- or 3-tetrahydrofuryl group), tetrahydrothienyl group (2- or 3-tetrahydrothienyl group), thiazolidinyl group (2-, 3-, 4- or 5-thiazolidinyl group), piperidinyl group (1-, 2-, 3- or 4-piperidinyl group), piperazinyl group (1- or 2-piperazinyl group), tetrahydropyranyl group (2-, 3- or 4-tetrahydropyranyl group), morpholinyl group (2-, 3- or 4-morpholinyl group), thiomorpholinyl group (2-, 3- or 4-thiomorpholinyl group), dioxolanyl group (2- or 4-dioxolanyl group), homopiperidinyl group (1-, 2-, 3- or 4-homopiperidinyl group), homopiperazinyl group (1-, 2-, 5- or 6-homopiperazinyl group), indolinyl group (1-, 2-, 3-, 4-, 5-, 6- or 7-indolinyl group), chromanyl group (2-, 3-, 4-, 5-, 6-, 7- or 8-chromanyl group) and the like can be mentioned, oxadiazolyl group, thiazolyl group and benzoxazolyl group are preferable and 1,3,4-oxadiazol-2-yl group, 2-thiazolyl group and 2-benzoxazolyl group are more preferable.

The hetero ring group is optionally substituted by one or more of the following substituents. As the substituent here, for example, a nitro group, a linear or branched chain alkoxy group (preferable carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), alkoxycarbonyl group (e.g.: methoxycarbonyl group, ethoxycarbonyl group etc.) and the like can be mentioned.

Specific examples of the heterocyclic group optionally having substituent(s) include 5-tert-butyl-1,3,4-oxadiazol-2-yl group, 2-thiazolyl group, 5-methoxycarbonylbenzoxazol-2-yl group and the like, with preference given to 5-tert-butyl-1,3,4-oxadiazol-2-yl group.

The "acyl group" of the "acyl group optionally having substituent(s)" for Y is a linear or branched chain acyl group preferably having 1-20 carbon atoms, such as acetyl group, propionyl group, butyryl group, isobutyryl group and the like. The acyl group is optionally substituted by one or more of the following substituents. As the substituent here, for example, a nitro group, a linear or branched chain alkoxy group (preferable carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), a 2-pyridyloxy group and the like can be mentioned. Specific examples of the "acyl group optionally having substituent(s)" preferably include 4-(2-pyridyloxy)butyryl group.

The "amino-protecting group other than acyl group" for Q is not particularly limited as long as it is other than acyl group. For example, benzyloxycarbonyl group (Cbz group), tert-butoxycarbonyl group (Boc group), methoxycarbonyl group (Moc group), 9-fluorenylmethoxycarbonyl group (Fmoc group) and the like can be mentioned. Of these, Cbz group, Boc group and the like permitting deprotection under mild conditions are preferable.

Preferable embodiment of each symbol is explained in the following.

P is preferably an alkyl group or an aralkyl group optionally having substituent(s), and methyl group, ethyl group and benzyl group are more preferable.

As an alkyl group optionally having substituent(s) for R³, methyl group and ethyl group are preferable, as an aryl group optionally having substituent(s) for R³, nitrophenyl group is preferable, as an aralkyl group optionally having substituent(s) for R³, benzyl group and chlorobenzyl group are preferable, as a trialkylsilyl group for R³, trimethylsilyl group is preferable, as -COR^{4a} for R³, acetyl group and propionyl group are preferable, as -SO₂R^{4b} for R³, methanesulfonyl group and p-toluenesulfonyl group are preferable.

As M, sodium or potassium is preferable.

As R⁵, a phenyl group, a p-fluorophenyl group or a methyl group is preferable.

R⁶ is preferably a hydrogen atom or an alkyl group, and a hydrogen atom, a methyl group or a tert-butyl group is more preferable.

R⁷ is preferably an alkyl group optionally having substituent(s) or an aralkyl group optionally having substituent(s), and methyl group, isopropyl group or benzyl group is more preferable.

R⁸ is preferably a hydrogen atom, hydroxyl group, methoxy group, tert-butoxy group, benzyloxy group or -N(R⁹)-OR¹⁰ (each symbol is as defined above), and hydrogen atom, methoxy group, benzyloxy group or -N(Me)-OMe is more preferable.

Y is preferably 5-tert-butyl-1,3,4-oxadiazol-2-yl group, 2-thiazolyl group, trifluoromethyl group, 4-(2-pyridyloxy)butyryl group or 5-methoxycarbonylbenzoxazol-2-yl group.

When compounds represented by the formulas (I), (V), (VI), (VIII), (IX), (X), (XI), (XII), (XIII), (XIV) and (XV) have an acidic group, salts such as alkali metal salt (e.g., potassium salt, sodium salt, lithium salt etc.), organic amine salt (e.g., triethylamine salt, dicyclohexylamine salt etc.) and the like can be formed, and when the compounds have a basic group, inorganic acid salt (e.g., hydrochloride, sulfate etc.), organic acid salt (e.g., acetate, trifluoroacetate, tosylate, mesylate etc.) and the like can be formed.

The production method of the present invention is shown in the following reaction schemes. 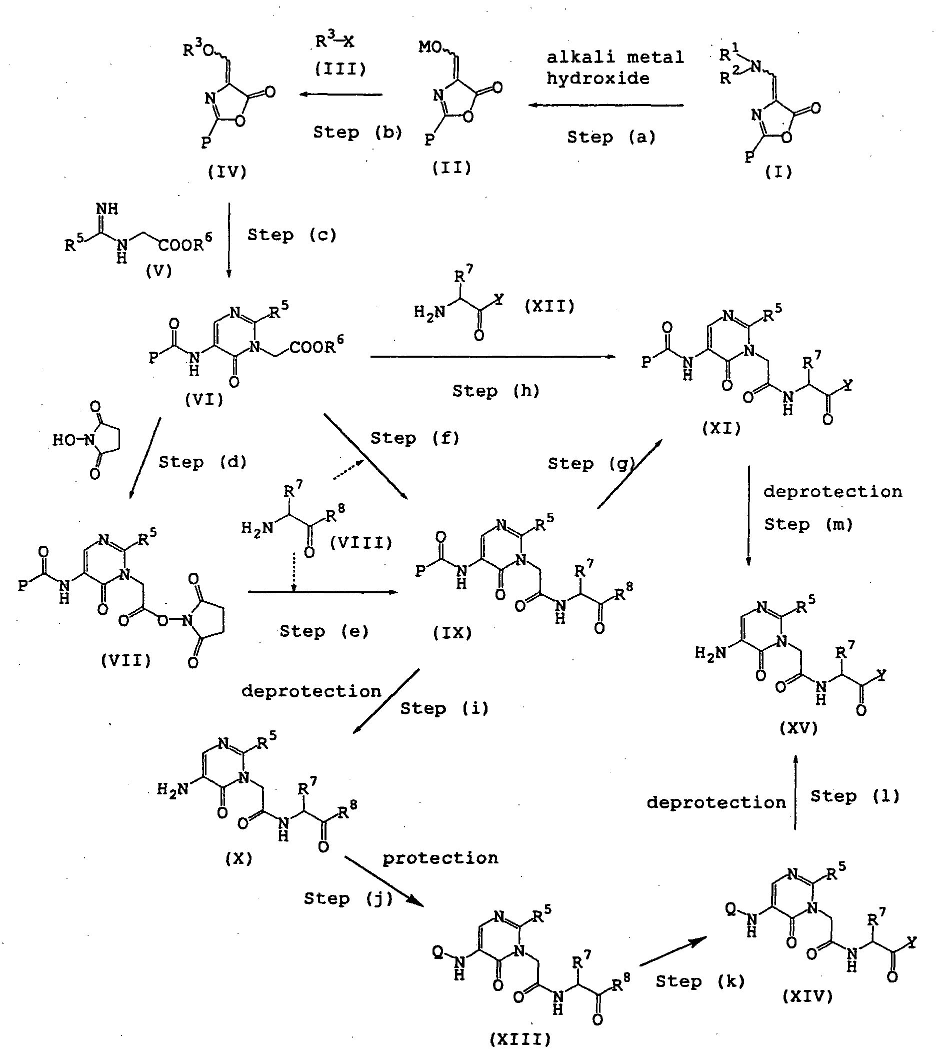 wherein each symbol is as defined above.

In other words, the present invention relates to a production method of a compound represented by the formula (IV), (VI), (VII), (IX), (X), (XI) or (XV), which partially includes Step (a)-(m) shown in the above-mentioned reaction scheme, and a preferable route for producing a desired compound can be appropriately selected.

The production method of the present invention includes novel synthesis routes, Step (a) and Step (b), for producing compound (IV), which is a novel azlactone derivative, and compound (IV) can be produced efficiently in a high yield by these Steps.

Using compound (IV) as a starting material of pyrimidine compound (XV) or (X), which is an enzyme inhibitor or a useful intermediate therefore, the reaction can proceed via compound (VI), (VII), (IX) and (XI), which are synthetic intermediates wherein the 5-position amino group of pyrimidine ring is protected by aliphatic acyl permitting deprotection under mild conditions, deprotection can be performed without decomposition and the like in Step (i) or (m).

As a result, deprotection prior to Steps (d), (e), (f), (g) and (h) is not necessary, and side reactions such as dimerization by condensation reaction in the form of a free amino group and the like can be avoided. In addition, since the protecting group does not need to be changed to avoid dimerization, compound (X) or compound (XV) having high quality can be efficiently produced in a smaller number of steps.

In the present invention, moreover, it has been found that deprotection can be performed by an enzyme reaction in deprotection Step (i) or (m) by using compound (IV) as a starting material. Since the enzyme reaction proceeds in a high yield under mild conditions by stirring at room temperature in around a neutral range, it can be preferably applied to the present invention.

It has been further found that deprotection proceeds in a high yield under mild conditions of weak acidic near room temperature without accompanying side reactions such as decomposition and the like.

Steps (a)-(m) are explained in the following.

### 1. Step (a)

In Step (a), compound (I) is hydrolyzed in the presence of an alkali metal hydroxide to give compound (II). To be specific, for example, the reaction can be carried out by adding an alkali metal hydroxide, preferably an aqueous solution thereof, to compound (I) in a solvent. The order of addition may be reverse or simultaneous.

As a production method of compound (II), a method of producing 4-hydroxymethylene-2-alkyl-2-oxazolin-5-one by reacting 4-(N,N-dimethylaminomethylene)-2-alkyl-2-oxazolin-5-one with sodium ethoxide in an ethanol solvent is known (Ind. J. Chem., 39B, 688-693 (2000)), but this method is associated with problems in that an open-ring form is by-produced, a decomposed product is generated during neutralization and the like.

In Step (a), the use of an alkali metal hydroxide is advantageous because compound (II) can be synthesized in a high yield under mild conditions.

Since compound (II) produced in Step (a) is stable in the form of a salt, M is preferably an alkali metal such as sodium, potassium and lithium, and sodium is more preferable.

As the alkali metal hydroxide to be used in Step (a), sodium hydroxide, potassium hydroxide, lithium hydroxide and the like can be mentioned, with preference given to sodium hydroxide. While alkali metal hydroxide can be used in the form of a solid, it is preferably used as an aqueous solution. In this case, the concentration is in the range of 0.1N-12N.

The amount of alkali metal hydroxide to be used is generally 0.9-1.5 equivalents, preferably 1-1.2 equivalents, relative to compound (I).

As the solvent to be used in Step (a), any solvent can be used as long as it does not inhibit the reaction, and, for example, water, acetates (e.g., ethyl acetate, isopropyl acetate, isobutyl acetate, n-butyl acetate etc.), acetonitrile, tetrahydrofuran (THF), N,N-dimethylformamide and the like can be mentioned. These may be used in combination of two or more kinds thereof. A mixed solvent of water and an organic solvent miscible with water, such as acetonitrile, acetone and the like, is more preferable and a mixed solvent of acetonitrile and water is particularly preferable. The amount of solvent to be used is generally 3- to 50-fold weight, preferably 10- to 20-fold weight, relative to compound (I). When an aqueous solution of alkali metal hydroxide is to be used, the amount of water is included in the amount of solvent.

The use of an alcohol solvent (e.g., ethanol, isopropyl alcohol, n-butanol etc.) is not preferable because a side reaction such as ring opening of azlactone ring of compound (I) and the like tends to occur.

Step (a) is conducted in the range of generally from -10°C to the refluxing temperature of the solvent to be used (preferably 0°C to 20°C). This reaction completes in the above-mentioned temperature range generally for 30 min to one night (preferably 2 hr-20 hr).

After the completion of the reaction of Step (a), compound (II) is present in the form of an alkali metal salt (M=alkali metal). Therefore, when a free form (M=hydrogen atom) of compound (II) is to be isolated, an acid (e.g., hydrogen chloride, sulfuric acid etc.) is added to the reaction mixture to adjust to pH 3.5-4.5, and a free form of compound (II) can be isolated by a general isolation and purification method, such as concentration, crystal precipitation or silica gel column chromatography of a reaction mixture, but the method is not limited to these.

For isolation in the form of an alkali metal salt, an alkali metal salt of compound (II) can be isolated by a general isolation and purification method, such as concentration or crystal precipitation of a reaction mixture.

Moreover, an alkali metal salt of compound (II) easily becomes a hydrate, and therefore, when subjected to Step (b) as it is in the form of a hydrate, an adverse influence such as decomposition due to an influence of water and the like may occur. To prevent this, it is preferable to yield the compound as an anhydrous crystal by high temperature slurry washing in an organic solvent, high temperature drying and the like.

Of compounds (II) obtained in Step (a), those in the form of an alkali metal salt are novel compounds.

Compound (I), which is a starting material in Step (a), can be produced by a known method.

For example, as described in Reference Examples 1-3 to be mentioned later, compound (I) can be produced by reacting N-acylglycine represented by the formula: PC(=O)NHCH₂COOH wherein P is as defined above, with formamide represented by the formula: R¹R²NCHO wherein each symbol is as defined above and phosphorus oxychloride (Ind. J. Chem., 39B, 688-693 (2000)).

In addition, as described in Reference Example 4 to be mentioned later, compound (I) can be produced by reacting N-acylglycine represented by the formula: PC(=O)NHCH₂COOH wherein P is as defined above with formamide dimethylacetal represented by the formula: R¹R²NCH(OMe)₂ wherein each symbol is as defined above and N,N'-dicyclohexylcarbodiimide (J. Heterocyclic Chem., 34, 247 (1997)).

### 2. Step (b)

In Step (b), compound (II) is reacted with reagent (III) to give compound (IV). To be specific, for example, the reaction can be carried out by adding a reagent (III) to compound (II) in a solvent. The order of addition may be reverse or simultaneous.

Compound (IV) wherein the 2-position of azlactone ring is an aliphatic group when produced by an Erlenmeyer method, which is a general production method of an azlactone compound, is associated with many by-products, extremely low yield and difficult isolation. According to Step (a) and Step (b) of the present invention, compound (IV) can be advantageously produced in a high yield from known compound (I) under mild conditions.

As the solvent to be used in Step (b), any solvent can be used as long as it does not inhibit the reaction, and, for example, acetates (e.g., ethyl acetate, isopropyl acetate, isobutyl acetate, n-butyl acetate etc.), acetonitrile, THF, N,N-dimethylformamide, halogen solvents (e.g., chloroform, dichloromethane etc.), acetone and the like can be mentioned. These may be used in combination of two or more kinds thereof, and a single or mixed solvent of N,N-dimethylformamide, acetonitrile and chloroform is preferable. The amount of solvent to be used is generally 3- to 50-fold weight, preferably 5- to 20-fold weight, relative to compound (II).

As reagent (III) to be used for Step (b), any can be used as long as it can be defined by R³ and X in the formula (III). Specific examples include, but not limited to, dimethyl sulfate, diethyl sulfate, methyl iodide, ethyl iodide, benzyl chloride, benzyl bromide, benzyl iodide, p-toluenesulfonyl chloride, methanesulfonyl chloride, trimethylsilyl chloride, acetyl chloride, acetic anhydride and the like. From the aspects of the yield and cost, dimethyl sulfate, diethyl sulfate, benzyl bromide and trimethylsilyl chloride are preferable.

When compound (II) is a free form (M=hydrogen atom), a base is preferably added during the reaction. As such a base, inorganic base (e.g., potassium carbonate, sodium carbonate, sodium hydride, potassium hydride etc.) and organic base (e.g., triethylamine, diisopropylethylamine, pyridine, N-methylmorpholine etc.) can be mentioned, with preference given to organic base, particularly triethylamine and diisopropylethylamine.

The amount of the base to be used is generally 0.5-5 equivalents, preferably 1-1.5 equivalents, relative to compound (II).

While the amount of reagent (III) to be used varies depending on the kind of the reagent to be used, it is generally 0.9-2 equivalents, preferably 1-1.5 equivalents, relative to compound (II). When an acetic anhydride is used beyond the above-mentioned amount of use, such portion functions as a solvent.

Step (b) is performed in the range of generally from 0°C to the refluxing temperature of the solvent to be used (preferably 0°C to 80°C). The reaction completes in the above-mentioned temperature range generally in 30 min - one night (preferably 1 hr-20 hr) .

Isolation and purification of compound (IV) can be carried out by a conventional method. For example, after the completion of the reaction, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.), partitioned and the obtained organic layer is concentrated to isolate compound (IV). Furthermore, compound (IV) can be purified by, but not limited to, adding a crystal precipitation solvent such as ethers (e.g., diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.) to allow crystal precipitation or subjecting the compound to silica gel column chromatography.

Compound (IV) can be subjected to a next step without isolation as a reaction mixture containing compound (IV).

In Step (b), a part of the obtained compound (IV), namely, compound (IV'), is a novel compound, which is useful as an intermediate for an enzyme inhibitor of the present invention, and also can be used as a synthetic intermediate for various amino acid derivatives and heterocycle derivatives (International Journal of Peptide & protein Research, 45(3), 266-71 (1995); J. C. S. Parkin. Transaction 1, (17), 2813-16 (1998) and the like).

### 3. Step (c)

In Step (c), compound (IV) is reacted with compound (V) or a salt thereof to give compound (VI). This reaction is carried out in a solvent. To be specific, for example, compound (IV) or a solution thereof is added to compound (V) or a solution thereof (in the case of a solution, it is preferably added dropwise) and the mixture is stirred with heating. The order of addition may be reverse or simultaneous.

When R⁶ is an alkyl group or aralkyl group, compound (V) tends to be decomposed when it is in a free form, and as a solid, it is generally isolated in the form of a stable salt. As a salt of compound (V), for example, acid addition salts such as hydrochloride, sulfate, trifluoroacetate and the like can be mentioned. When R⁶ is a hydrogen atom, compound (V) exists stably by forming a salt with an imino group in a molecule, and it also exists stably as a base addition salt such as sodium salt, potassium salt and the like. Accordingly, when compound (V) in a free form is reacted with compound (IV), a base (e.g., alkoxide compound such as sodium ethoxide, potassium butoxide, sodium methoxide and the like) in an amount necessary for converting compound (V) to a base addition salt is preferably used.

In Step (c), these acid addition salts of compound (V) are preferably neutralized using a base in a solvent, once converted into a free form and then reacted with compound (IV). It is also possible to carry out reaction without once converting to a free form before reaction, such as by dissolving a salt of compound (V) and compound (IV) in a solvent and adding a base. In this case, however, the yield of the object compound (VI) decreases. The base to be used for neutralization is free of particular limitation, and, for example, sodium hydroxide, potassium hydroxide, sodium carbonate, potassium carbonate, triethylamine, sodium methoxide, sodium ethoxide and the like can be mentioned. These bases may be used in the form of an aqueous solution. Neutralization is generally performed in a solvent and as the solvent, toluene, ethyl acetate and the like can be used. In this case, it is preferable to add water to dissolve the base, and a free form of compound (V) extracted into an organic layer can be used upon partitioning. The amount of the base to be use is not particularly limited as long as it can convert a salt of compound (V) into a free form. From economical aspects, the amount is preferably not more than 3 equivalents relative to compound (V).

The solvent usable in Step (c) may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, alcohols (e.g., ethanol, isopropyl alcohol, n-butanol etc.), N,N-dimethylformamide and the like can be mentioned. These may be used in combination of two or more kinds thereof, and acetonitrile, toluene, ethanol, isopropyl alcohol and ethyl acetate are preferable. A single or mixed solvent of acetonitrile, toluene and isopropyl alcohol is more preferable, and a single or mixed solvent of acetonitrile and toluene is particularly preferable. The amount of solvent to be used is generally 2- to 50-fold weight, preferably 5- to 20-fold weight, relative to compound (IV).

The amount of compound (V) to be use is generally 0.9-3 equivalents, preferably 1-1.3 equivalents, relative to compound (IV).

When the reaction mixture of Step (b) is to be used as compound (IV), the amount of each reagent to be used is within the above-mentioned range, based on compound (II) used in Step (b) .

The reaction of compound (IV) with compound (V) is carried out within the range of from generally 10°C to the refluxing temperature of the solvent to be used (preferably 20°C to 80°C). The reaction completes within the above-mentioned temperature range for generally 1 hr-20 hr (preferably 3 hr-15 hr).

Compound (VI) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (VI). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (VI), but the method is not limited to these.

### 4. Step (d)

In Step (d), when R⁶ of compound (VI) is other than a hydrogen atom, compound (VI) or a salt thereof is converted to a carboxylic acid form by hydrolysis and the like and condensed with N-hydroxysuccinimide to give compound (VII).

Compound (VII) is a novel compound, can be amidated with various amines as it is as in Step (e) to be mentioned later. Therefore, it is convenient as a versatile intermediate. It provides an advantage that it can be condensed with amines having a free carboxyl group, such as amino acid, without protection of carboxyl group.

When R⁶ of compound (VI) is other than a hydrogen atom, namely, an alkyl group or an aralkyl group, it is first converted to a carboxylic acid form (R⁶=hydrogen atom, hereinafter also to be simply referred to as a carboxylic acid form) by hydrolysis, acid treatment, hydrogenation and the like. The hydrolysis, acid treatment, hydrogenation and the like can be performed by a method known to those of ordinary skill in the art. For example, hydrolysis can be performed by reacting a compound with a base (e.g., sodium hydroxide, potassium hydroxide etc.) or an acid (e.g., hydrogen chloride, sulfuric acid etc.) in alcohol such as methanol or ethanol or a mixed solvent with water, but the method is not limited to these.

For example, when R⁶ is a tert-butyl group and the like, an acid treatment thereof gives a carboxylic acid form, which can be, for example, performed by a treatment with an acid (e.g., trifluoroacetic acid and the like) in an organic solvent.

When R⁶ is benzyl and the like, a carboxylic acid form can be obtained by hydrogenolysis. For example, it can be performed by catalytic reduction in the presence of a catalyst such as palladium on carbon and the like.

A carboxylic acid form and N-hydroxysuccinimide can be condensed by a method known to those of ordinary skill in the art, and the method is not particularly limited. For example, a carboxylic acid form is reacted with a chlorinating agent such as thionyl chloride, phosphorus pentachloride and the like to give an acid chloride, which is then condensed with N-hydroxysuccinimide. Since primary amide exists in a molecule of compound (VI), a method comprising activating a carboxylic acid form with a condensation agent and then condensing with N-hydroxysuccinimide is preferable.

While Step (d) is explained in the following by referring to preferable embodiments, Step (d) is not limited to this embodiment.

In a preferable embodiment, a carboxylic acid form is activated with an activating agent in a solvent and then condensed with N-hydroxysuccinimide.

As the activating agent, for example, chloroformates (e.g., ethyl chloroformate, isobutyl chloroformate, methyl chloroformate etc.), carbodiimides (e.g., N,N'-dicyclohexylcarbodiimide (DCC), N-(3-dimethylaminopropyl)-N'-ethylcarbodiimide (EDC) etc.), carbodiimidazole (CDI), diphenylphosphoryl azide (DPPA) and the like can be mentioned, but the activating agent is not limited to these. Of these, chloroformate, DCC, EDC and the like are preferable, ethyl chloroformate and isobutyl chloroformate are particularly preferable.

The amount of the activating agent to be used is generally 0.9-1.5 equivalents, preferably 1-1.3 equivalents, relative to the carboxylic acid form.

For activation of a carboxylic acid form, a base may be added as necessary. As such base, inorganic base (e.g., sodium carbonate, potassium carbonate etc.), organic base (e.g., triethylamine, diisopropylethylamine, N-methylmorpholine, pyridine, piperidine etc.) can be mentioned, organic base, particularly N-methylmorpholine, triethylamine and pyridine are preferable.

The amount of the base to be used is generally 0.9-2 equivalents, preferably 1-1.5 equivalents, relative to a carboxylic acid form.

The solvent to be used for the activation of a carboxylic acid form may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, dichloromethane, methyl-tert-butyl ether and the like can be mentioned. These may be used in combination of two or more kinds thereof, a single or mixed solvent of acetonitrile, THF, ethyl acetate and the like is more preferable. The amount of the solvent to be used is generally 3- to 50-fold weight, preferably 5- to 20-fold weight, relative to a carboxylic acid form.

A carboxylic acid form is activated within the range of generally from -40°C to the refluxing temperature of the solvent to be used (preferably -10°C to 10°C). The activation completes within the above-mentioned temperature range, generally 5 min-3 hr (preferably 10 min - 1 hr).

After activation of a carboxylic acid form, N-hydroxysuccinimide is added to a reaction mixture. The order of addition may be reverse or simultaneous.

The amount of N-hydroxysuccinimide to be used is generally 0.9-2 equivalents, preferably 1-1.3 equivalents, relative to a carboxylic acid form.

The condensation with N-hydroxysuccinimide is performed within the range of generally from -50 to 40°C (preferably -20°C to 20°C). The condensation completes within the above-mentioned temperature range, generally 1 hr-20 hr (preferably 2 hr-5 hr) .

Compound (VII) can be isolated and purified by a conventional method. After the completion of the reaction, for example, the reaction mixture is washed successively with an aqueous acidic solution (e.g., hydrogen chloride, sulfuric acid etc.), an aqueous alkali solution (e.g., saturated aqueous sodium hydrogen carbonate, brine etc.) and partitioned, and the obtained organic layer is concentrated to isolate compound (VII). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (VII), but the method is not limited to these.

### 5. Step (e)

In Step (e), compound (VII) is reacted with compound (VIII) or a salt thereof to give compound (IX) or a salt thereof. To be specific, for example, compound (VII) or a solution thereof is added to a solution of compound (VIII) in a solvent. The order of addition may be reverse or simultaneous.

Step (e) is advantageous in that the reaction can be performed without side reactions such as self-condensation of compound (VIII) and the like even when R⁸ of compound (VIII) is a hydroxyl group.

Therefore, as compound (VIII) in Step (e), compound (VIII) wherein R⁸ is a hydroxyl group, namely, a free amino acid, can be preferably used.

The solvent usable in Step (e) may be any as long as it does not inhibit the reaction, and, for example, water, acetates (e.g., ethyl acetate, isopropyl acetate, isobutyl acetate, n-butyl acetate etc.), acetonitrile, THF, N,N-dimethylformamide, acetone and the like can be mentioned. These may be used in combination of two or more kinds thereof, and a single or mixed solvent of water, acetonitrile, THF and the like is more preferable. The amount of the solvent to be used is generally 2- to 50-fold weight, preferably 5- to 20-fold weight, relative to compound (VII).

When compound (VIII) is a free amino acid, it is preferably used after preparing an aqueous alkali solution (e.g., aqueous sodium hydroxide solution, aqueous potassium hydroxide solution etc.) at pH 5-10. The amount of water in the aqueous solution is included in the amount of the solvent to be used.

The amount of compound (VIII) to be used is generally 0.8-2 equivalents, preferably 1-1.5 equivalents, relative to compound (VII).

Step (e) is performed within the range of generally from -20°C to the refluxing temperature of the solvent to be used (preferably 0°C to 30°C). The reaction completes within the above-mentioned temperature range, generally 30 min to one night (preferably 1 hr-10 hr).

When R⁸ of compound (IX) is a hydroxyl group, it is present in the form of a carboxylic acid salt. Therefore, after adding an acid (e.g., hydrogen chloride, sulfuric acid etc.) to the reaction mixture to adjust its pH to not more than 3, for example, the reaction mixture is concentrated or a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify a free form of compound (IX), but the method is not limited to these.

When R⁸ of compound (IX) is other than a hydroxyl group, isolation and purification can be performed by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (IX). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (IX), but the method is not limited to these.

### 6. Step (f)

In Step (f), when R⁶ of compound (VI) is other than a hydrogen atom, compound (VI) or a salt thereof is converted to a carboxylic acid form, and the carboxylic acid form is condensed with compound (VIII) or a salt thereof to give compound (IX) or a salt thereof.

Compound (IX) synthesized in Step (e) and Step (f) is a novel compound, and since a group represented by R⁸ can be converted to a group represented by Y in the state where an amino group of compound (VIII) is protected in Step (g) to be mentioned later, it is useful as a synthetic intermediate capable of efficiently producing compound (XV), which is an enzyme inhibitor.

In Step (f), when R⁸ of compound (VIII) is a hydroxyl group, it is not preferable because side reactions such as self-condensation and the like can occur.

Therefore, Step (f) can be preferably applied when R⁸ of compound (VIII) is other than a hydroxyl group, namely, a hydrogen atom, an alkoxy group, an aralkyloxy group or -N(R⁹)-OR¹⁰ wherein each symbol is as defined above.

When R⁶ of compound (VI) is other than a hydrogen atom, namely, an alkyl group or an aralkyl group, it is first converted to a carboxylic acid form by a method similar to that in Step (d).

A carboxylic acid form and compound (VIII) can be condensed by a method of condensing a carboxylic acid form with N-hydroxysuccinimide in Step (d), where compound (VIII) or a salt thereof is used instead of N-hydroxysuccinimide under similar conditions.

In other words, in a preferable embodiment, a carboxylic acid form is activated with an activating agent in a solvent and condensed with compound (VIII) or a salt thereof to yield compound (IX).

This embodiment is explained in the following but Step (f) is not limited to this embodiment.

As the activating agent, one similar to that used in Step (d) can be mentioned, with preference given to chloroformates, particularly ethyl chloroformate, methyl chloroformate, isobutyl chloroformate and the like.

The amount of the activating agent to be used is generally 0.9-1.5 equivalents, preferably 1-1.2 equivalents, relative to a carboxylic acid form.

During activation of a carboxylic acid form, a base may be added as necessary. As such base, a base similar to that used in Step (d) can be mentioned, with preference given to N-methylmorpholine, triethylamine, pyridine and piperidine.

The amount of the base to be used is generally 0.9-2 equivalents, preferably 1-1.2 equivalents, relative to a carboxylic acid form.

The solvent to be used for the activation of a carboxylic acid form may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, halogen solvents (e.g., dichloromethane, chloroform etc.), ether solvents (e.g., diethyl ether, methyl-tert-butyl ether etc.) and the like and the like can be mentioned. These may be used in combination of two or more kinds thereof, and a single or mixed solvent of acetonitrile, THF, ethyl acetate, methyl-tert-butyl ether and the like are more preferable. The amount of solvent to be used is generally 3- to 50-fold weight, preferably 5- to 20-fold weight, relative to a carboxylic acid form.

A carboxylic acid form is activated at a temperature within the range of generally -50°C to 40°C (preferably -20°C to 20°C). The activation completes within the above-mentioned temperature range generally for 5 min-5 hr (preferably 15 min-2 hr) .

After the activation of a carboxylic acid form, compound (VIII) is added to the reaction mixture. The order of addition may be reverse or simultaneous.

The amount of compound (VIII) to be used is generally 0.9-3 equivalents, preferably 1-1.5 equivalents, relative to a carboxylic acid form.

When compound (VIII) is in the form of a salt, a base similar to that used for activating a carboxylic acid form is preferably added concurrently for neutralization.

The amount of the base to be used is generally 0.9-2 equivalents, preferably 1-1.5 equivalents, relative to compound (VIII) .

The condensation with compound (VIII) is conducted within the temperature range of generally -50°C to 40°C (preferably -20°C to 20°C). The condensation completes within the above-mentioned temperature range generally for 0.5 hr-20 hr (preferably 1 hr-3 hr).

Compound (IX) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (IX). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like is added to allow crystal precipitation, or the reaction mixture is subjected to silica gel column chromatography to purify compound (IX), but the method is not limited to these.

Compound (VIII), which is a starting material of Step (e) and Step (f), is a commercially available amino acid, or can be produced by esterifying it by a known method or condensing it with a hydroxylamine derivative represented by the formula: NH(R⁹)-OR¹⁰ wherein each symbol is as defined above by a known method.

### 7. Step (g)

In Step (g), group represented by R⁸ of compound (IX) or a salt thereof is converted to a group represented by Y to give compound (XI) or a salt thereof.

Step (g) can be one of advantageous reaction routes, since compound (XV) can be produced efficiently, because compound (XI) can be produced from compound (IX) by converting a group represented by R⁸ to a group represented by Y in the state where an amino group of compound (VIII) is protected, as mentioned above.

Since Step (g) varies in the method of converting a group represented by R⁸ to a group represented by Y depending on the embodiment of Y, each case is explained in the following. 7-1. When Y is a heterocyclic group optionally having substituent(s) (hereinafter to be also referred to as Step (g-1)).

In Step (g-1), as shown in the reaction scheme in the following, a group represented by R⁸ is converted to a heterocyclic group. To be specific, compound (IX) is reacted with a reagent represented by the formula (XVIIa)): Y¹-M¹ wherein Y¹ is a heterocyclic group optionally having substituent(s) and M¹ is lithium or MgX¹ wherein X¹ is a chlorine atom, a bromine atom or an iodine atom (hereinafter to be also referred to as reagent (XVIIa)) in a solvent to give a compound represented by the formula (XI) wherein Y is a heterocyclic group optionally having substituent(s) (hereinafter to be also referred to as compound (XIa)). 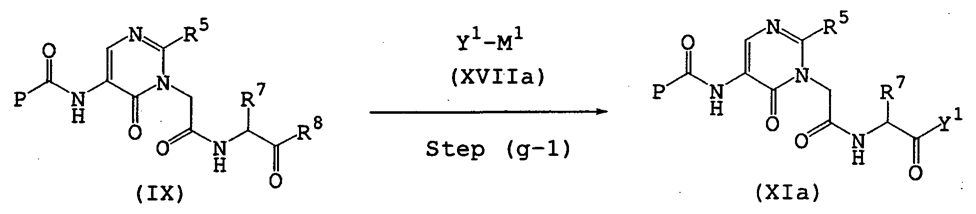 wherein each symbol is as defined above.

In Step (g-1), R⁸ of compound (IX) is preferably a group represented by -N(R⁹)-OR¹⁰ wherein each symbol is as defined above so as to prevent further reaction of compound (XIa) with reagent (XVIIa).

The reagent (XVIIa) can be obtained by reacting a compound represented by the formula: Y¹-H or Y¹-X¹ wherein each symbol is as defined above with alkyl lithium (e.g., n-butyl lithium, methyl lithium etc.) or lithium amide (e.g., lithium diisopropylamide etc.) in an ether solvents (e.g., THF, diethyl ether, methyl-tert-butyl ether (MTBE) etc.) at -90°C to 20°C, or a compound represented by the formula: Y¹-X¹ wherein each symbol is as defined above with magnesium under the general conditions for the production of Grignard reagent.

The solvent usable in Step (g-1) may be any as long as it does not inhibit the reaction, and, for example, ether solvents (e.g., THF, diethyl ether, MTBE etc.), hydrocarbon solvents (e.g., n-hexane, toluene etc.) and the like can be mentioned. Two or more kinds thereof may be used in combination. The amount of the solvent to be used is generally 5- to 50-fold weight, preferably 8- to 20-fold weight, relative to compound (IX).

The amount of reagent (XVIIa) to be used is generally 1-10 equivalents, preferably 1-6 equivalents, relative to compound (IX).

Step (g-1) is performed within the range of generally at -90°C to 30°C (preferably -80°C to -20°C). The reaction completes within the above-mentioned temperature range generally for 10 min-5 hr (preferably 30 min-2 hr).

When R⁸ is a hydrogen atom, a reduced form of compound (XIa) (alcohol form) is obtained, which can be introduced into compound (XIa) by oxidization by a known method (e.g., Dess-Martin reagent, Swern oxidization, Pfitzner-Moffatt oxidation etc.).
7-2. When R⁸ is a hydrogen atom and Y is a heterocyclic group represented by the formula (XVIII): 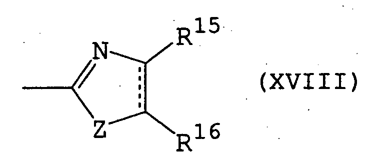 wherein a dotted line shows a double bond or a single bond, Z is an oxygen atom or a sulfur atom, and R¹⁵ and R¹⁶ are the same or different and each is a group corresponding to a substituent of a heterocyclic group, or may form, together with the adjacent carbon atom, a homocyclic ring or heterocycle optionally having substituent(s) (hereinafter to be also referred to as heterocyclic group (XVIII)) (hereinafter to be also referred to as Step (g-2)).

When R⁸ is a hydrogen atom, R⁸ (hydrogen atom) of compound (IX) can be converted to a heterocyclic group (XVIII) by the following steps (g-2a)-(g-2e) according to the method described in Journal of Medicinal Chemistry, 44(8), 1286-1296 (2001).

A compound represented by the formula (XIb) wherein Y is a heterocyclic group (XVIII) (hereinafter to be also referred to as compound (XIb)) can be obtained as shown in the following reaction scheme,
Step (g-2a): a compound represented by the formula (IXa) wherein R⁸ is a hydrogen atom (hereinafter to be also referred to as compound (IXa)) or a salt thereof is converted to cyanohydrin represented by the formula (XIX) (hereinafter to be also referred to as cyanohydrin (XIX));
Step (g-2b): the obtained cyanohydrin (XIX) or a salt thereof is converted to imidate represented by the formula (XXI) (hereinafter to be also referred to as imidate (XXI)) or a salt thereof;
Step (g-2c): the obtained imidate (XXI) or a salt thereof is reacted with a compound represented by the formula (XVIIIa) (hereinafter to be also referred to as compound (XVIIIa)) or a salt thereof to give a compound represented by the formula (XXII) (hereinafter to be also referred to as compound (XXII)) or a salt thereof;
or,
Step (g-2d): compound (IXa) or a salt thereof is reacted with a compound represented by the formula (XVIIIb) (hereinafter to be also referred to as compound (XVIIIb)) to give compound (XXII) or a salt thereof;
Step (g-2e): the obtained compound (XXII) or a salt thereof is oxidized. 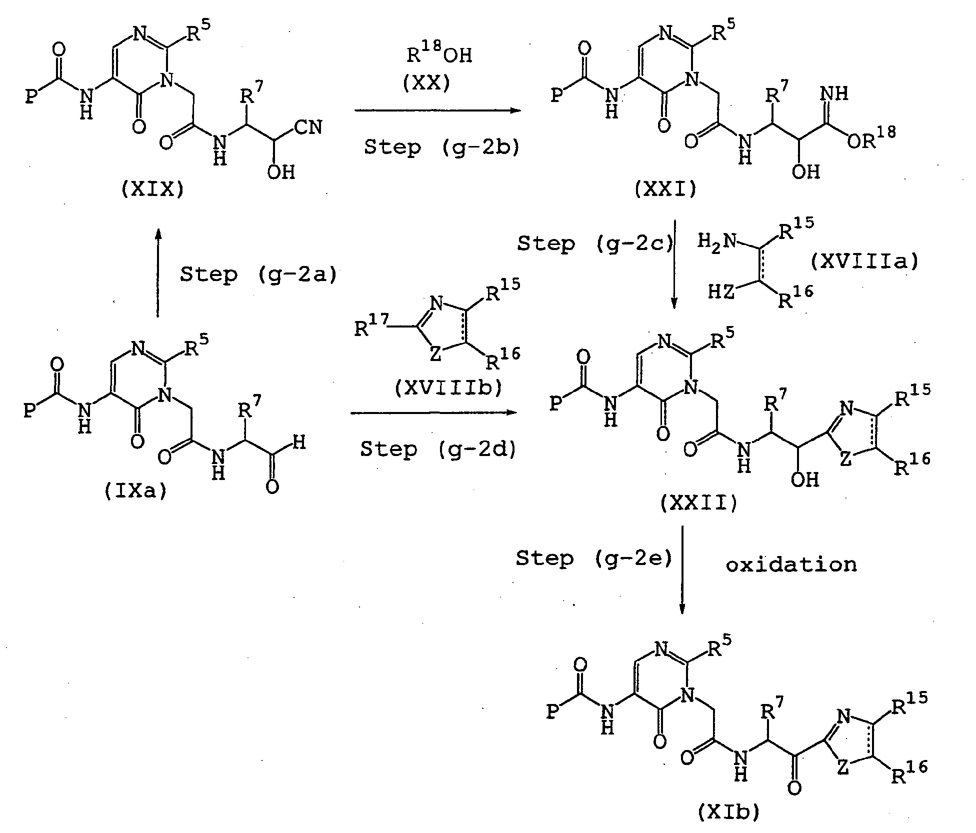 wherein R¹⁷ is a trialkylsilyl group, R¹⁸ is an alkyl group, and other symbols and dotted line are as defined above.

The "group corresponding to a substituent of heterocyclic group" for R¹⁵ or R¹⁶ corresponds to the substituent of the "heterocyclic group optionally having substituent(s)" for Y. To be specific, for example, a nitro group, a linear or branched chain alkoxy group (carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), alkoxycarbonyl group (e.g.: methoxycarbonyl group, ethoxycarbonyl group etc.) and the like can be mentioned.

As the homocyclic ring optionally formed by R¹⁵ and R¹⁶ in combination is, for example, benzene, naphthalene, cyclohexane and the like can be mentioned.

As the heterocycle optionally formed by R¹⁵ and R¹⁶ in combination is, for example, pyridine, piperidine and the like can be mentioned.

As the substituent that the homocyclic ring and heterocycle optionally has, those similar to the above-mentioned "group corresponding to the substituent of a heterocyclic group" can be mentioned.

The "trialkylsilyl group" for R¹⁷ is a silyl group substituted by the same or different alkyl group and, for example, a trimethylsilyl group and the like can be mentioned.

The "alkyl group" for R¹⁸ is a linear or branched chain alkyl preferably having 1 to 3 carbon atoms, and, for example, methyl group, ethyl group, n-propyl group and the like can be mentioned, methyl group and ethyl group are preferable.

### 7-2-1. Step (g-2a)

Step (g-2a) is realized by, for example, reacting compound (IXa) with cyanohydrins and a base in a solvent.

The solvent to be used in Step (g-2a) may be any as long as it does not inhibit the reaction, and, for example, dichloromethane, THF, diethyl ether and the like can be mentioned. Two or more kinds thereof may be used in combination. The amount of the solvent to be used is generally 5- to 50-fold weight, preferably 8- to 20-fold weight, relative to compound (IXa).

As the cyanohydrins, for example, acetone cyanohydrin and the like can be mentioned. The amount of cyanohydrins to be used is generally 0.8-5 equivalents, preferably 1-3 equivalents, relative to compound (IXa).

As the base, for example, triethylamine, pyridine, N-methylmorpholine and the like can be mentioned, with preference given to triethylamine. The amount of the base to be used is generally 0.5-3 equivalents, preferably 0.6-2 equivalents, relative to compound (IXa).

Step (g-2a) is performed within the range of generally 0°C to 50°C (preferably 10°C to 30°C). The reaction completes within the above-mentioned temperature range, generally 30 min-24 hr (preferably 1 hr-8 hr).

The cyanohydrin (XIX) obtained in Step (g-2a) can be isolated and purified by a conventional method. After the completion of the reaction, for example, the reaction mixture is washed with brine and the like, and partitioned, and the obtained organic layer is concentrated to isolate cyanohydrin (XIX). Furthermore, crystal precipitation is performed, or the reaction mixture is subjected to silica gel column chromatography to purify cyanohydrin (XIX), but the method is not limited to these.

### 7-2-2. Step (g-2b)

Step (g-2b) is realized by, for example, reacting cyanohydrin (XIX) with alcohol represented by the formula (XX): R¹⁸OH wherein R¹⁸ is as defined above (hereinafter to be also referred to as alcohol (XX)) and hydrogen chloride in the presence or absence of a solvent.

As the solvent usable in Step (g-2b) may be any as long as it does not inhibit the reaction, and, for example, chloroform, dichloromethane, THF and the like can be mentioned. Two or more kinds thereof may be used in combination. When a solvent is used, the amount thereof to be used is generally 5-to 50-fold weight, preferably 8- to 20-fold weight, relative to cyanohydrin (XIX).

As alcohol (XX), ethanol, propanol and methanol can be specifically mentioned, with preference given to methanol and ethanol. The amount of alcohol (XX) to be used is generally 0.1-50 equivalents, preferably 1-20 equivalents, relative to cyanohydrin (XIX).

Hydrogen chloride may be introduced into the reaction system in the form of a gas, or added in the form of a solution of alcohol (XX), or generated in the system by adding acetyl chloride, trimethylsilyl chloride and the like. The amount of hydrogen chloride to be used is generally 2-50 equivalents, preferably 5-30 equivalents, relative to cyanohydrin (XIX).

Step (g-2b) is performed within the range of generally -20°C to 30°C (preferably 0°C to 20°C). The reaction completes within the above-mentioned temperature range, generally 30 min-24 hr (preferably 1 hr-16 hr).

Imidate (XXI) obtained in Step (g-2b) can be isolated and purified by a conventional method, or can be subjected to the next step without particular purification.

### 7-2-3. Step (g-2c)

Step (g-2c) can be realized by, for example, reacting imidate (XXI) with compound (XVIIIa) in a solvent.

The solvent usable in Step (g-2c) may be any as long as it does not inhibit the reaction, and, for example, ethanol, acetonitrile, THF and the like can be mentioned. Two or more kinds thereof may be used in combination. The amount of solvent to be used is generally 5- to 50-fold weight, preferably 8- to 20-fold weight, relative to imidate (XXI).

As compound (XVIIIa), 2-aminophenol, 2-aminothiophenol, 2-amino-3-hydroxypyridine, methyl 3-amino-4-hydroxybenzoate, aminoethanol and the like can be specifically mentioned, with preference given to methyl 3-amino-4-hydroxybenzoate. The amount of compound (XVIIIa) to be used is generally 0.8-2 equivalents, preferably 1-1.5 equivalents, relative to imidate (XXI).

In Step (g-2c), a base may be added to promote the reaction. As the base, for example, triethylamine, diisopropylethylamine, N-methylmorpholine and the like can be mentioned, with preference given to triethylamine. The amount of the base to be used is generally 0.5-5 equivalents, preferably 1-3 equivalents, relative to imidate (XXI).

Step (g-2c) is performed within the range of generally from 0°C to the refluxing temperature of the solvent to be used (preferably 30°C to refluxing temperature). The reaction completes within the above-mentioned temperature range, generally 30 min-24 hr (preferably 1 hr-8 hr).

### 7-2-4. Step (g-2d)

Step (g-2d) can be realized by, for example, reacting compound (IXa) with compound (XVIIIb) in a solvent as shown in the following scheme to give a compound represented by the formula (XXII') (hereinafter to be also referred to as compound (XXII')), and deprotecting silyl ether of the obtained compound (XXII' ) . 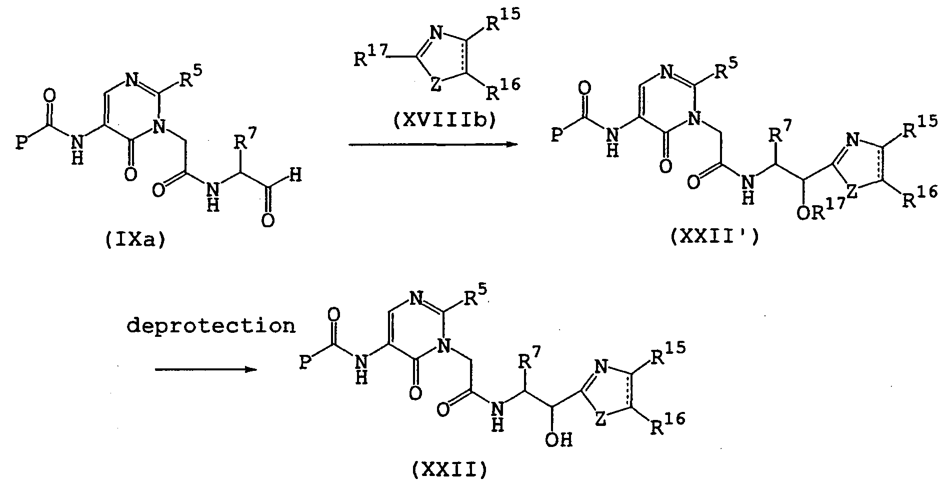 wherein each symbol and dotted line are as defined above.

The solvent usable in Step (g-2d) may be any as long as it does not inhibit the reaction, and, for example, dichloromethane, chloroform, THF and the like can be mentioned. Two or more kinds thereof may be used in combination. The amount of solvent to be used is generally 5- to 50-fold weight, preferably 8- to 20-fold weight, relative to compound (IXa).

As compound (XVIIIb), 2-trimethylsilylthiazole, 2-trimethylsilyloxazole and the like can be specifically mentioned, with preference given to 2-trimethylsilylthiazole. The amount of compound (XVIIIb) to be used is generally 0.8-3 equivalents, preferably 1-1.5 equivalents, relative to compound (IXa) .

Compound (XVIIIb) can be obtained by the method described in J. Org. Chem. 58, 3196 (1993), or a commercially available product may be used.

Step (g-2d) is performed within the range of generally 0°C to 50°C (preferably 10°C to 30°C). The reaction completes within the above-mentioned temperature range, generally 30 min-24 hr (preferably 1 hr-8 hr).

For deprotection of the obtained compound (XXII'), an acid (e.g., hydrogen chloride, methanesulfonic acid etc.) or a fluoride salt (e.g., tetra-n-butylammonium fluoride etc.) can be added to the above-mentioned reaction solution.

The amount of the acid or fluoride salt to be used is generally 1-10 equivalents, preferably 1-3 equivalents, relative to compound (IXa).

The deprotection is performed within the range of generally 0°C to 50°C (preferably 10°C to 30°C). The reaction completes within the above-mentioned temperature range, generally 30 min-24 hr (preferably 30 min-8 hr).

Compound (XXII) obtained in Step (g-2c) or Step (g-2d) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XXII). Furthermore, crystal precipitation may be performed, or the reaction mixture is subjected to silica gel column chromatography to purify compound (XXII), but the method is not limited to these.

### 7-2-5. Step (g-2e)

In Step (g-2e), compound (XXII) is oxidized by various known methods (e.g., Dess-Martin reagent, Swern oxidization, Pfitzner-Moffatt oxidation etc.) to give compound (XIb).

Pfitzner-Moffatt oxidation (J. Med. Chem., 30, 1617-1622 (1987)), which is a preferable embodiment, is explained in the following, but Step (g-2e) is not limited to this method.

The Pfitzner-Moffatt oxidization in Step (g-2e) can be realized by, for example, reacting compound (XXII) with a carbodiimide condensation agent and an acid in dimethyl sulfoxide and other solvent.

The solvent usable in the oxidization reaction may be any as long as it does not inhibit the reaction, and, for example, toluene, chloroform, dichloromethane and the like can be mentioned. Two or more kinds thereof may be used in combination. The amount of solvent to be used is generally 5-to 50-fold weight, preferably 8- to 20-fold weight, relative to compound (XXII).

The amount of dimethyl sulfoxide to be used is generally 2-100 equivalents, preferably 2-80 equivalents, relative to compound (XXII).

As the carbodiimide condensation agent, for example, N,N'-dicyclohexylcarbodiimide (DCC), N-(3-dimethylaminopropyl)-N'-ethylcarbodiimide hydrochloride (EDC) and the like can be mentioned, with preference given to EDC. The amount of the carbodiimide condensation agent to be used is generally 1-5 equivalents, preferably 1-3 equivalents, relative to compound (XXII) .

As the acid, for example, dichloroacetic acid, chloroacetic acid and the like can be mentioned, with preference given to dichloroacetic acid. The amount of the acid to be used is generally 0.8-10 equivalents, preferably 1-3 equivalents, relative to compound (XXII).

The oxidization reaction is performed within the range of generally 0°C to 30°C (preferably 0°C to 20°C). The reaction completes within the above-mentioned temperature range, generally 30 min-24 hr (preferably 1 hr-5 hr).

### 7-3. When R⁸ is a hydrogen atom and Y is an acyl group optionally having substituent(s) (hereinafter to be also referred to as Step (g-3))

When R⁸ is a hydrogen atom, R⁸ (hydrogen atom) of compound (IX) can be converted to an acyl group optionally having substituents by the following Steps (g-3a)-(g-3g) according to the method described in WO98/09949.

A compound represented by the formula (XIc), wherein Y is an acyl group optionally having substituent(s) (hereinafter to be also referred to as compound (XIc)) can be obtained as shown in the following reaction scheme,
Step (g-3a): compound (IXa) or a salt thereof is converted to cyanohydrin (XIX) or a salt thereof by a method similar to that in Step (g-2a);
Step (g-3b): the obtained cyanohydrin (XIX) or a salt thereof is hydrolyzed to give a compound represented by the formula (XXIII) (hereinafter to be also referred to as compound (XXIII)) or a salt thereof;
Step (g-3c): the obtained compound (XXIII) or a salt thereof is reacted with N,O-dimethylhydroxylamine or a salt thereof to give a compound represented by the formula (XXIV) (hereinafter to be also referred to as compound (XXIV)) or a salt thereof;
Step (g-3d): an oxazolidine ring is formed on the obtained compound (XXIV) or a salt thereof to give a compound represented by the formula (XXV) (hereinafter to be also referred to as compound (XXV)) or a salt thereof;
Step (g-3e): the obtained compound (XXV) or a salt thereof is reacted with a reagent represented by the formula (XVIIb) (hereinafter to be also referred to as reagent (XVIIb) to give a compound represented by the formula (XXVI) (hereinafter to be also referred to as compound (XXVI)) or a salt thereof;
Step (g-3f): the oxazolidine ring of the obtained compound (XXVI) or a salt thereof is opened to give a compound represented by the formula (XXVII) (hereinafter to be also referred to as compound (XXVII)) or a salt thereof;
Step (g-3g): the obtained compound (XXVII) or a salt thereof is oxidized. 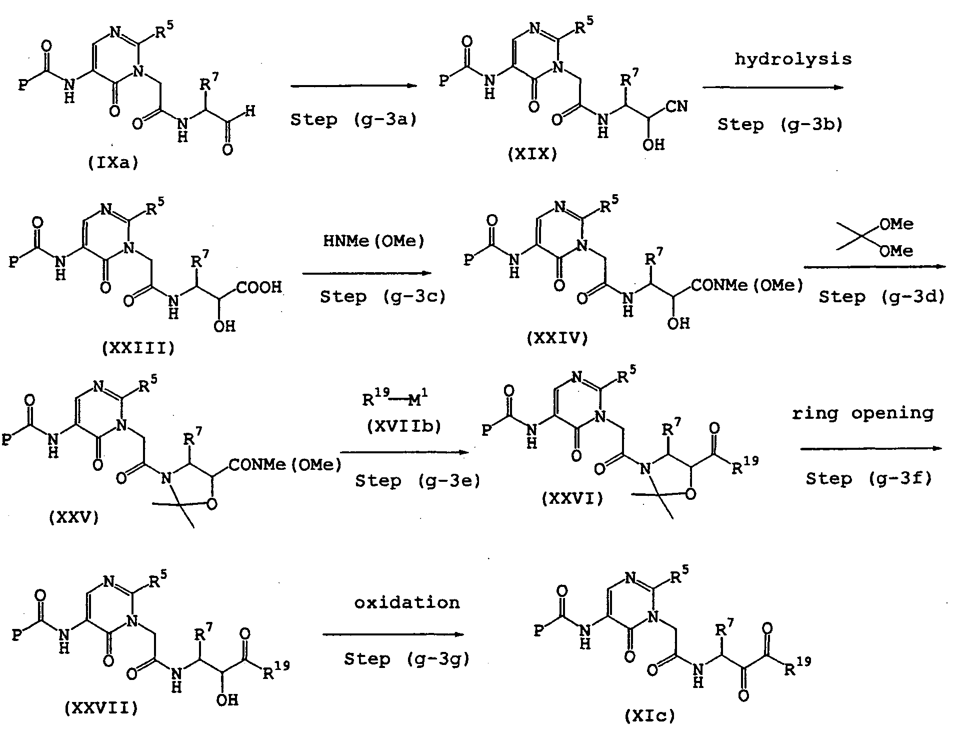 wherein R¹⁹ is a group formed by removing carbonyl group from an acyl group optionally having substituent(s) for Y, and other symbols are as defined above.

As the "group formed by removing carbonyl group from an acyl group optionally having substituent(s)" for R¹⁹ is, for example, 3-(2-pyridyloxy)propyl group and the like can be mentioned.

### 7-3-1. Step (g-3a)

In Step (g-3a), cyanohydrin (XIX) can be obtained from compound (IXa) by a method similar to that in Step (g-2a).

### 7-3-2. Step (g-3b)

Step (g-3b) can be realized by, for example, hydrolyzing cyanohydrin (XIX) with an acid in a solvent.

The solvent usable in Step (g-3b) may be any as long as it does not inhibit the reaction, and, for example, 1,4-dioxane, THF, MTBE and the like can be mentioned. Two or more kinds thereof may be used in combination. The amount of solvent to be used is generally 5- to 50-fold weight, preferably 8- to 20-fold weight, relative to cyanohydrin (XIX).

As the acid, for example, hydrogen chloride, sulfuric acid, p-toluenesulfonic acid and the like can be mentioned, with preference given to hydrogen chloride. The amount of the acid to be used is generally 3-100 equivalents, preferably 5-30 equivalents, relative to cyanohydrin (XIX).

Step (g-3b) is performed within the range of generally from 0°C to the refluxing temperature of the solvent to be used (preferably 5°C to 60°C). The reaction completes within the above-mentioned temperature range, generally 30 min-24 hr (preferably 1 hr-16 hr).

Compound (XXIII) obtained Step (g-3c) can be isolated and purified by a conventional method.

After the completion of the reaction, for example, the reaction mixture is washed with an aqueous acidic solution (e.g., hydrogen chloride, sulfuric acid etc.), and partitioned, and the obtained organic layer is concentrated to isolate compound (XXIII). Furthermore, crystal precipitation may be performed, or the reaction mixture is subjected to silica gel column chromatography to purify compound (XXIII), but the method is not limited to these.

### 7-3-3. Step (g-3c)

Step (g-3c) can be realized by, for example, condensing compound (XXIII) with N,O-dimethylhydroxylamine using a condensation agent in a solvent.

The solvent usable in Step (g-3c) may be any as long as it does not inhibit the reaction, and, for example, dichloromethane, THF, diethyl ether and the like can be mentioned. Two or more kinds thereof may be used in combination. The amount of the solvent to be used is generally 5- to 50-fold weight, preferably 8- to 20-fold weight, relative to compound (XXIII).

As the condensation agent, for example, carbodiimide condensation agents (e.g., DCC, EDC etc.), chloroformates (e.g., methyl chloroformate, ethyl chloroformate, isobutyl chloroformate etc.) and the like can be mentioned.

The condensation reaction is performed within the range of generally -20°C to 20°C (preferably 0°C to 10°C). The reaction completes within the above-mentioned temperature range, generally 15 min-16 hr (preferably 0.5 hr-2 hr).

Compound (XXIV) obtained in Step (g-3c) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XXIV). Furthermore, crystal precipitation may be performed, or the reaction mixture is subjected to silica gel column chromatography to purify compound (XXIV), but the method is not limited to these.

### 7-3-4. Step (g-3d)

Step (g-3d) can be realized by, for example, reacting compound (XXIV) with acetone dimethylacetal in a solvent or without solvent in the presence of an acid.

The solvent usable in Step (g-3d) may be any as long as it does not inhibit the reaction, and, for example, acetone, ethyl acetate, THF and the like can be mentioned. Two or more kinds thereof may be used in combination. The amount of the solvent to be used is generally 5- to 50-fold weight, preferably 8- to 20-fold weight, relative to compound (XXIV).

The amount of acetone dimethylacetal to be used is generally 1-5 equivalents, preferably 1-3 equivalents, relative to compound (XXIV).

As the acid, for example, p-toluenesulfonic acid, methanesulfonic acid, sulfuric acid and the like can be mentioned, with preference given to p-toluenesulfonic acid. The amount of the acid to be used is generally 0.1-3 equivalents, preferably 0.3-2 equivalents, relative to compound (XXIV) .

Step (g-3d) is performed within the range of generally -20°C to 40°C (preferably 0°C to 20°C). The reaction completes within the above-mentioned temperature range, generally 30 min-24 hr (preferably 1 hr-16 hr).

Compound (XXV) obtained in Step (g-3d) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XXV). Furthermore, crystal precipitation may be performed, or the reaction mixture is subjected to silica gel column chromatography to purify compound (XXV), but the method is not limited to these.

### 7-3-5. Step (g-3e)

Step (g-3e) can be realized by, for example, reacting compound (XXV) with reagent (XVIIb) in a solvent.

The solvent usable in Step (g-3e) may be any as long as it does not inhibit the reaction, and, for example, ether solvents (e.g., THF, diethyl ether, MTBE etc.), halogen solvents (e.g., chloroform, dichloromethane etc.) and the like can be mentioned. Two or more kinds thereof may be used in combination. The amount of solvent to be used is generally 5-to 50-fold weight, preferably 8- to 20-fold weight, relative to compound (XXV).

The reagent (XVIIb) can be prepared from a compound represented by the formula R¹⁹-X wherein each symbol is as defined above by a method similar to that for reagent (XVIIa) in the aforementioned Step (g-1). For example, 3-(2-pyridyloxy)propylmagnesium bromide, 3-(2-pyridyloxy)propyllithium and the like are preferable. The amount of reagent (XVIIb) to be used is generally 1-5 equivalents, preferably 1-3 equivalents, relative to compound (XXV).

Step (g-3e) is performed within the range of generally -80°C to 40°C (preferably -78°C to 20°C). The reaction completes within the above-mentioned temperature range, generally 15 min-24 hr (preferably 1 hr-16 hr).

Compound (XXVI) obtained in Step (g-3e) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XXVI). Furthermore, crystal precipitation may be performed, or the reaction mixture is subjected to silica gel column chromatography to purify compound (XXVI), but the method is not limited to these.

### 7-3-6. Step (g-3f)

Step (g-3f) can be realized by, for example, reacting compound (XXVI) with an acid in a solvent.

The solvent usable in Step (g-3f) may be any as long as it does not inhibit the reaction, and, for example, water, methanol, ethanol and the like can be mentioned. Two or more kinds thereof may be used in combination. The amount of solvent to be used is generally 5- to 50-fold weight, preferably 8- to 20-fold weight, relative to compound (XXVI).

As the acid, for example, hydrogen chloride, sulfuric acid, p-toluenesulfonic acid, methanesulfonic acid and the like can be mentioned, with preference given to p-toluenesulfonic acid and sulfuric acid. The amount of the acid to be used is generally 0.2-30 equivalents, preferably 1-20 equivalents, relative to compound (XXVI).

Step (g-3f) is performed within the range of generally from 20°C to the refluxing temperature of the solvent to be used. The reaction completes within the above-mentioned temperature range, generally 30 min-24 hr (preferably 3 hr-16 hr).

Compound (XXVII) obtained in Step (g-3f) can be isolated and purified by a conventional method.

After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XXVII). Furthermore, crystal precipitation may be performed, or the reaction mixture is subjected to silica gel column chromatography to purify compound (XXVII), but the method is not limited to these.

### 7-3-7. Step (g-3g)

In Step (g-3g), compound (XXVII) can be introduced into compound (XIc) by a method similar to that in Step (g-2e) (e.g., Dess-Martin reagent, Swern oxidization, Pfitzner-Moffatt oxidation etc.).

### 7-4. When R⁸ is a hydroxyl group and Y is a trifluoromethyl group (hereinafter to be also referred to as Step (g-4))

When R⁸ is a hydroxyl group, R⁸ (hydroxyl group) of compound (IX) can be converted to a trifluoromethyl group by the following Steps (g-4a)-(g-4d) according to the method described in Tetrahedron Lett., 36(42), 7761-7764 (1995).

A compound represented by the formula (XId) wherein Y is a trifluoromethyl group (hereinafter to be also referred to as compound (XId)) can be obtained as shown in the following scheme,
Step (g-4a): a compound represented by the formula (IXb) wherein R⁸ is a hydroxyl group (hereinafter to be also referred to as compound (IXb)) or a salt thereof is reacted with aldehyde represented by the formula (XXVIII) : R²⁰CHO wherein R²⁰ is a hydrogen atom, alkyl group (e.g., methyl group, ethyl group etc.), an aryl group optionally having substituent(s) (e.g., p-methoxyphenyl group etc.) and the like (hereinafter to be also referred to as aldehyde (XXVIII)) to give a compound represented by the formula (XXIX) (hereinafter to be also referred to as compound (XXIX)) or a salt thereof;
Step (g-4b): the obtained compound (XXIX) or a salt thereof is reacted with trimethyl(trifluoromethyl)silane to give a compound represented by the formula (XXX) (hereinafter to be also referred to as compound (XXX)) or a salt thereof;
Step (g-4c): a trimethylsilyl group of the obtained compound (XXX) or a salt thereof is deprotected to give a compound represented by the formula (XXXI) (hereinafter to be also referred to as compound (XXXI)) or a salt thereof;
Step (g-4d): an oxazolidine ring of the obtained compound (XXXI) or a salt thereof is opened. 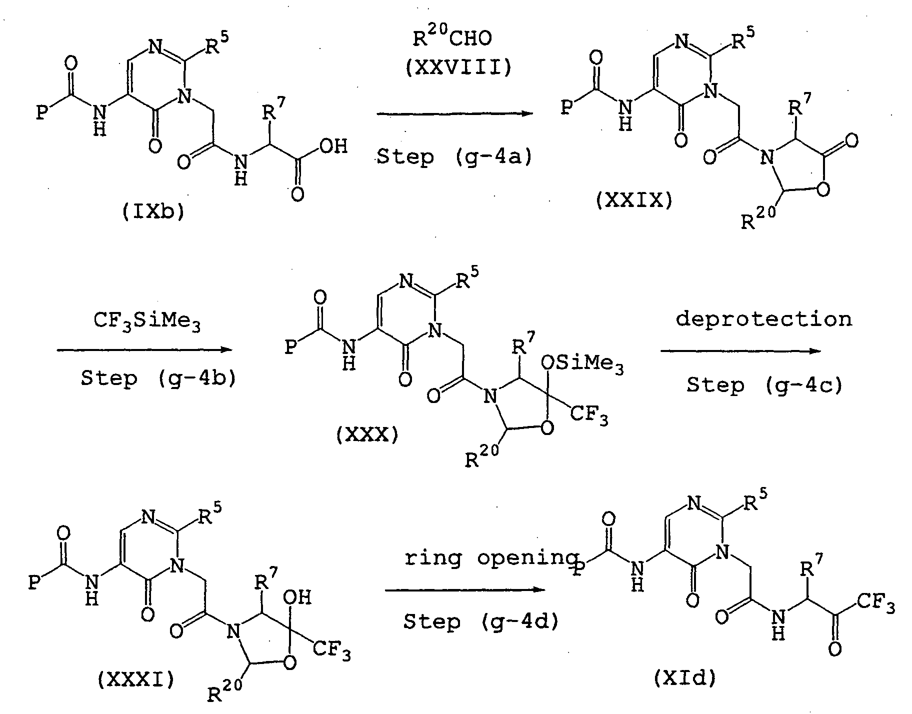 wherein each symbol is as defined above.

### 7-4-1. Step (g-4a)

Step (g-4a) can be realized by, for example, subjecting compound (IXb) and aldehyde (XXVIII) to a dehydration reaction in a solvent in the presence of an acid. The dehydration reaction can be performed by, for example, distilling water and the solvent from the mixture and returning only the solvent by an azeotropic reaction using a Dean-Stark tube.

As the solvent usable in Step (g-4a), for example, benzene, toluene, chloroform, diethyl ether, acetonitrile and the like can be mentioned. Two or more kinds thereof may be used in combination. The amount of the solvent to be used is generally 5- to 50-fold weight, preferably 8- to 20-fold weight, relative to compound (IXb).

As the acid, for example, p-toluenesulfonic acid, methanesulfonic acid and the like can be mentioned, with preference given to p-toluenesulfonic acid. The amount of the acid to be used is generally 0.01-5 equivalents, preferably 0.03-1 equivalent, relative to compound (IXb).

As aldehyde (XXVIII), for example, paraformaldehyde, acetaldehyde and the like can be mentioned, with preference given to paraformaldehyde. The amount of aldehyde (XXVIII) to be used is generally 1-5 equivalents, preferably 1-3 equivalents, relative to compound (IXb).

Step (g-4a) is generally performed at the refluxing temperature of the solvent to be used. The reaction completes within the above-mentioned temperature range, generally 30 min-24 hr (preferably 1 hr-16 hr).

Compound (XXIX) obtained in Step (g-4a) can be isolated and purified by a conventional method. After the completion of the reaction, for example, the reaction mixture is washed with an aqueous acidic solution (e.g., hydrogen chloride, sulfuric acid etc.), and partitioned, and the obtained organic layer is concentrated to isolate compound (XXIX). Furthermore, crystal precipitation may be performed, or the reaction mixture is subjected to silica gel column chromatography to purify compound (XXIX), but the method is not limited to these.

### 7-4-2. Step (g-4b)

Step (g-4b) can be realized by, for example, reacting compound (XXIX) with trimethyl(trifluoromethyl)silane in a solvent in the presence of a fluoride salt. In this case, ultrasonication is preferably applied to the reaction system to promote the reaction.

The solvent usable in Step (g-4b) may be any as long as it does not inhibit the reaction, and, for example, THF, MTBE and the like can be mentioned. Two or more kinds thereof may be used in combination. The amount of solvent to be used is generally 5- to 50-fold weight, preferably 8- to 20-fold weight, relative to compound (XXIX).

As the fluoride salt, for example, cesium fluoride, tetra-n-butylammonium fluoride and the like can be mentioned, with preference given to cesium fluoride. The amount of the fluoride salt to be used can be a catalyst amount, which is generally 1-5 equivalents, preferably 1-2 equivalents, relative to compound (XXIX).

The amount of trimethyl(trifluoromethyl)silane to be used is generally 0.8-5 equivalents, preferably 1-2 equivalents, relative to compound (XXIX).

Step (g-4b) is performed within the range of generally 0°C to 80°C (preferably 5°C to 40°C). The reaction completes within the above-mentioned temperature range, generally 30 min-24 hr (preferably 1 hr-16 hr).

Compound (XXX) obtained in Step (g-4b) can be isolated and purified by a conventional method. After the completion of the reaction, for example, the reaction mixture is washed with an aqueous acidic solution (e.g., hydrogen chloride, sulfuric acid etc.), and partitioned, and the obtained organic layer is concentrated to isolate compound (XXX). Furthermore, crystal precipitation may be performed, or the reaction mixture is subjected to silica gel column chromatography to purify compound (XXX), but the method is not limited to these.

### 7-4-3. Step (g-4c)

Step (g-4c) can be realized by, for example, treating compound (XXX) with a fluoride salt in a solvent. In this case, ultrasonication is preferably applied to the reaction system to promote the reaction.

The solvent usable in Step (g-4c) may be any as long as it does not inhibit the reaction, and, for example, THF, MTBE, dichloromethane, chloroform and the like can be mentioned. Two or more kinds thereof may be used in combination. The amount of solvent to be used is generally 5- to 50-fold weight, preferably 8-to 12-fold weight, relative to compound (XXX).

As the fluoride salt, for example, cesium fluoride, tetra-n-butylammonium fluoride and the like can be mentioned, with preference given to cesium fluoride. The amount of the fluoride salt to be used can be a catalyst amount, which is generally 1-5 equivalents, preferably 1-2 equivalents, relative to compound (XXX).

Step (g-4c) is performed within the range of generally 0°C to 80°C (preferably 0°C to 40°C). The reaction completes within the above-mentioned temperature range, generally 30 min-24 hr (preferably 1 hr-16 hr).

Compound (XXXI) obtained in Step (g-4c) can be isolated and purified by a conventional method. After the completion of the reaction, for example, the reaction mixture is washed with an aqueous acidic solution (e.g., hydrogen chloride, sulfuric acid etc.), and partitioned, and the obtained organic layer is concentrated to isolate compound (XXXI). Furthermore, crystal precipitation may be performed, or the reaction mixture is subjected to silica gel column chromatography to purify compound (XXXI), but the method is not limited to these.

### 7-4-4. Step (g-4d)

Step (g-4d) can be realized by, for example, treating compound (XXXI) with an acid in a solvent.

The solvent usable in Step (g-4d) may be any as long as it does not inhibit the reaction, and, for example, acetonitrile, THF, ethyl acetate, chloroform and the like can be mentioned. Two or more kinds thereof may be used in combination. The amount of solvent to be used is generally 5-to 50-fold weight, preferably 8- to 20-fold weight, relative to compound (XXXI).

As the acid, for example, strong acidic ion exchange resins (e.g., Amberlite (trademark) R-120, Amberlyst (trademark) etc.) and the like can be mentioned, with preference given to strong acidic ion exchange resin. The amount of the acid to be used is generally 0.1- to 100-fold weight, preferably 0.5- to 20-fold weight, relative to compound (XXXI).

Step (g-4d) is performed within the range of generally 0°C to 80°C (preferably 20°C to 50°C). The reaction completes within the above-mentioned temperature range, generally 30 min-24 hr (preferably 1 hr-16 hr).

### 7-5. When R⁸ is a hydrogen atom and Y is a trifluoromethyl group (hereinafter to be also referred to as Step (g-5))

When R⁸ is a hydrogen atom, R⁸ (hydrogen atom) of compound (IX) can be converted to a trifluoromethyl group by the following Steps (g-5a)-(g-5c) according to the method described in Tetrahedron Lett., 36(42), 7761-7764 (1995).

Compound (XId) can be obtained as shown in the following scheme,
Step (g-5a): compound (IXa) or a salt thereof is reacted with trimethyl(trifluoromethyl)silane to give a compound represented by the formula (XXXII) or a salt thereof;
Step (g-5b): a trimethylsilyl group of the obtained compound represented by the formula (XXXII) or a salt thereof is eliminated to give a compound represented by the formula (XXXIII) or a salt thereof;
Step (g-5c): the obtained compound represented by the formula (XXXIII) or a salt thereof is oxidized. 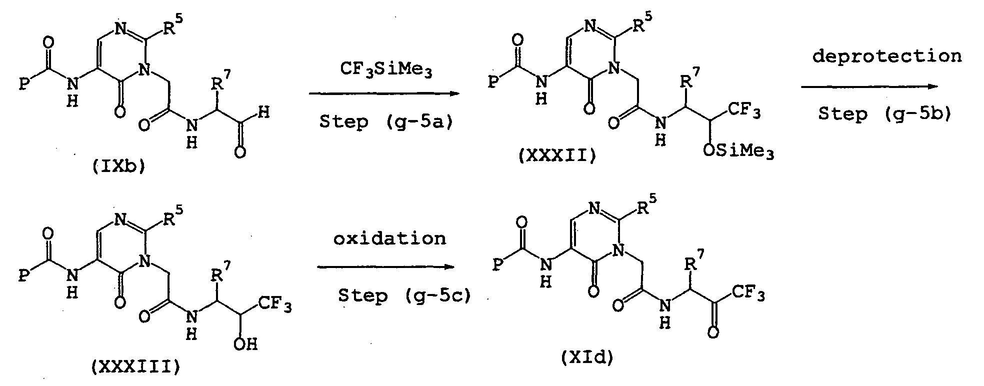 wherein each symbol is as defined above.

Step (g-5a) can be performed according to a method and conditions similar to those of Step (g-4b), Step (g-5b) can be performed according to a method and conditions similar to those of Step (g-4c), and Step (g-5c) can be performed according to a method and conditions similar to those of Step (g-2e).

Compound (XI) obtained by Steps (g-1)-(g-5) can be isolated and purified by a conventional method as mentioned above. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XI). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), acetates (e.g., ethyl acetate, butyl acetate etc.), water or a mixed solvent thereof etc.] is added to allow crystal precipitation, or the reaction mixture is subjected to silica gel column chromatography to purify compound (XI), but the method is not limited to these.

### 8. Step (h)

In Step (h), when R⁶ of compound (VI) is other than a hydrogen atom, compound (VI) or a salt thereof is converted to a carboxylic acid form, and the carboxylic acid form is condensed with compound (XII) to give compound (XI) or a salt thereof.

Step (h) is advantageous in that compound (XI) can be produced by directly condensing compound (VI) with compound (XII) in a short step.

When R⁶ of compound (VI) is other than a hydrogen atom, namely, an alkyl group or an aralkyl group, a carboxylic acid form is first obtained by a method similar to that in Step (d).

A carboxylic acid form and compound (XII) are condensed by the method of Step (d) used for condensing a carboxylic acid form with N-hydroxysuccinimide, wherein compound (XII) or a salt thereof is used instead of N-hydroxysuccinimide under similar conditions.

In a preferable embodiment, moreover, a carboxylic acid form is activated with an activating agent in a solvent and condensed with compound (XII) or a salt thereof to give compound (XI).

This embodiment is explained below but Step (h) is not limited by this embodiment.

As the activating agent, those similar to ones used in Step (d) can be mentioned, with preference given to chloroformates, particularly, ethyl chloroformate, methyl chloroformate, isobutyl chloroformate and the like.

The amount of the activating agent to be used is generally 0.9-1.5 equivalents, preferably 1-1.2 equivalents, relative to a carboxylic acid form.

During activation of a carboxylic acid form, the base may be added as necessary. As such base, a base similar to that used in Step (d) can be mentioned, with preference given to N-methylmorpholine, triethylamine and pyridine.

The amount of the base to be used is generally 0.9-2 equivalents, preferably 1-1.3 equivalents, relative to a carboxylic acid form.

The solvent to be used for activation of carboxylic acid form may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, halogen solvents (e.g., dichloromethane, dichloroethane etc.) and the like can be mentioned. These may be used in combination of two or more kinds thereof, and a single or mixed solvent of acetonitrile, THF and the like is more preferable. The amount of the solvent to be used is generally 3- to 50-fold weight, preferably 5- to 20-fold weight, relative to a carboxylic acid form.

A carboxylic acid form can be activated within the range of generally -30°C to 40°C (preferably 0°C to 30°C). The activation completes within the above-mentioned temperature range, generally 5 min-5 hr (preferably 10 min-2 hr).

After activation of a carboxylic acid form, compound (XII) is added to the reaction mixture. The order of addition may be reverse or simultaneous.

The amount of compound (XII) to be used is generally 0.9-2 equivalents, preferably 1-1.2 equivalents, relative to a carboxylic acid form.

When compound (XII) is in the form of a salt, it is preferable to concurrently add, for neutralization, a base similar to the base used for activation of a carboxylic acid form.

The amount of the base to be used is generally 0.9-2 equivalents, preferably 1-1.2 equivalents, relative to compound (XII) .

Condensation with compound (XII) is performed within the range of generally -30°C to 40°C (preferably -20°C to 30°C). The condensation completes within the above-mentioned temperature range generally for 0.5 hr-5 hr (preferably 1 hr-3 hr).

Compound (XI) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XI). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (XI), but the method is not limited to these.

Compound (XII), which is a starting material of Step (h) can be synthesized using compound (VIII) as a starting material by protecting an amino group with a benzyloxycarbonyl group, a tert-butoxycarbonyl group and the like by a known method, converting a group represented by R⁸ to a group represented by Y by a method similar to that in the above-mentioned Step (g) and deprotecting by a known method.

### 9. Step (i)

In Step (i), the 5-position P-C(=O)-, wherein P is as defined above, of pyrimidine ring of compound (IX) is deprotected to give compound (X) or a salt thereof.

For the deprotection, a method known to those of ordinary skill in the art, such as the amide-deprotecting method described in Theodora W. Grrene, Peter G. M. Wuts, Protective Groups in Organic Synthesis, 2nd ed., A Wiley Interscience Publication (1991) mentioned above, can be applied without limitation. To avoid side reactions such as decomposition and the like, mild conditions are preferable.

As a preferable embodiment of Step (i), (1) deprotection by enzyme reaction [hereafter to be also referred to as Step (i-1)] and (2) deprotection under acidic conditions [hereafter to be also referred to as Step (i-2)] can be mentioned. Step (i-1) and step (i-2) are specifically explained in the following, but Step (i) is not limited to these embodiments.

### 9-1. Step (i-1)

In Step (i-1), compound (X) can be produced by, for example, reacting compound (IX) with an enzyme in a solvent to achieve deprotection.

The enzyme to be used in Step (i-1) is not particularly limited as long as it can deprotect the 5-position P-C(=O)-, wherein P is as defined above, of the pyrimidine ring, and conventionally known enzymes derived from microorganisms such as bacteria, actinomycetes, fungi and the like, or animals or plants, such as penicillin amidase, penicillin acylase and the like, can be mentioned.

It is also preferable to use an enzyme immobilized on a resin and the like because it facilitates separation from a compound and recycled use. As these enzymes and immobilized enzymes, commercially available ones can be used.

The amount of the enzyme or a solution obtained by treating mold only needs to be an amount capable of showing the object effect (effective amount) and the effective amount can be easily determined by those of ordinary skill in the art through a simple preliminary test. In the case of, for example, a commercially available enzyme solution, it is 0.01 ml-2 ml per 1 g of compound (IX) to be a substrate.

While the protecting group of compound (IX), which is a starting material, can be appropriately determined based on the substrate characteristics of the enzyme to be used. When it is a preferable enzyme, penicillin amidase, a phenylacetyl group wherein P is benzyl is preferable.

As a solvent usable in Step (i-1), water or a mixture of hydrophilic solvent to the degree the enzyme activity is not impaired can be used, and use of water alone is preferable. As the hydrophilic solvent, one or more kinds of N,N-dimethylformamide, dimethyl sulfoxide, THF, acetone and the like can be used. The amount of the solvent to be used is generally 5- to 200-fold weight, preferably 10- to 100-fold weight, relative to compound (IX).

The solvent is preferably adjusted to an optimal pH for an enzyme (e.g., about pH 6-8) with a buffer component, such as potassium dihydrogen phosphate, dipotassium hydrogen phosphate and the like.

While the concentration of a buffer component in a solvent is not particularly limited as long as it does not inhibit the enzyme reaction, it is about 0.001M - 0.2M.

It is also possible to adjust pH by appropriately adding a base and the like during the reaction.

In Step (i-1), a surfactant may be added as necessary to promote the reaction. As such surfactant, for example, Triton X-100 (trademark), Tween (trademark) and the like can be mentioned, with preference given to Triton X-100. The amount of the surfactant to be used is generally 0.001- to 0.05-fold weight, preferably 0.01- to 0.05-fold weight, relative to compound (IX).

Step (i-1) is performed within the range of generally 20°C to 50°C (preferably 30°C to 40°C). The reaction completes within the above-mentioned temperature range, generally 30 min-1 week (preferably 1 hr-20 hr).

After the completion of the reaction, the obtained compound (X) can be separated from an enzyme by a conventional method. In the case of an immobilized enzyme, for example, a soluble solvent of compound (X) (e.g., acetonitrile etc.) is added as necessary, and the mixture is filtered to separate compound (X) in the filtrate. In the case of an unimmobilized enzyme, water is added to the reaction mixture as necessary to allow precipitation of compound (X), which is collected by filtration to separate compound (X) as a filtered substance, but the method is not limited to these methods.

To the separated compound (X) is added, for example, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] to allow crystal precipitation, or the reaction mixture is subjected to silica gel column chromatography to purify compound (X), but the method is not limited to these.

### 9-2. Step (i-2)

In Step (i-2), compound (X) can be produced by, for example, adding an acid to compound (IX) in a solvent to allow deprotection. The order of addition may be reverse or simultaneous.

The solvent usable in Step (i-2) may be any as long as it does not inhibit the reaction. When an acid is used, at least a protic solvent such as water, alcohol and the like needs to be present. As the solvent, for example, acetates (e.g., ethyl acetate, isopropyl acetate, isobutyl acetate, n-butyl acetate etc.), hydrocarbons (e.g., toluene, benzene, xylene etc.), acetonitrile, THF, alcohols (e.g., methanol, ethanol, isopropyl alcohol, n-butanol etc.), N,N-dimethylformamide and the like can be mentioned. These may be used in combination of two or more kinds thereof and methanol, ethanol, acetonitrile, isopropyl alcohol, water and the like are preferable, and a single or mixed solvent of methanol, acetonitrile, water and the like is particularly preferable. The amount of the solvent to be used is generally 3- to 50-fold weight, preferably 5- to 20-fold weight, relative to compound (IX) .

As an acid to be used in Step (i-2), inorganic acids (e.g., hydrogen chloride, sulfuric acid etc.), organic acids (e.g., methanesulfonic acid, p-toluenesulfonic acid, trifluoroacetic acid etc.) and the like can be mentioned, with preference given to hydrogen chloride and the like.

The amount of the acid to be used is generally 1-50 equivalents, preferably 3-10 equivalents, relative to compound (IX) .

The acid is preferably used in the form of a solution of the solvent to be used, wherein a solvent and an acid are added such that the amount thereof fall within the above-mentioned ranges.

Step (i-2) is performed within the range of generally from 0°C to the refluxing temperature of the solvent to be used (preferably 20°C to 60°C). The reaction completes within the above-mentioned temperature range, generally 60 min-one night (preferably 1 hr-5 hr) .

After the completion of the reaction, compound (X) is present in the reaction mixture in the form of a salt with the acid used. Thus, the acid addition salt can be isolated by concentrating the reaction mixture. The isolated acid addition salt can be purified by adding a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] thereto to allow crystal precipitation.

For isolation of a free form of compound (X), alkali addition in a protic solvent such as water, alcohol and the like is applied, or, after the completion of the reaction, the reaction mixture is washed with an aqueous alkali solution (e.g., saturated aqueous sodium hydrogen carbonate, brine etc.) and partitioned, and the obtained organic layer is concentrated, whereby compound (X) can be isolated. Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like are added to allow crystal precipitation, or the reaction mixture is subjected to silica gel column chromatography to purify a free form of compound (X), but the method is not limited to these.

Compound (X) produced in Step (i) is a novel compound and is useful as a highly versatile synthetic intermediate for producing compound (XV) and other enzyme inhibitors (J. Med. Chem., 2001, 44, 1286-1296).

For example, compound (XV) can be produced by Steps (j), (k) and (1) explained in the following.

### 10. Step (j)

In Step (j), compound (XIII) or a salt thereof can be obtained by protection of an amino group of compound (X) with a group other than an acyl group by a known method, such as the methods recited on page 309 ff. of Theodora W. Grrene, Peter G. M. Wuts, Protective Groups in Organic Synthesis, 2nd edn., A Wiley Interscience Publication, 1991.

Protection with a Cbz group, which is a preferable embodiment of Step (j), is explained in the following, which is not to be construed as limiting Step (j).

Protection with a Cbz group can be realized by, for example, reacting compound (X) with a benzyloxycarbonylation agent (hereinafter to be also referred to as Cbz agent) in a solvent.

The solvent usable in the protection reaction may be any as long as it does not inhibit the reaction, and, for example, water, acetonitrile, ethyl acetate, THF, chloroform and the like can be mentioned. Two or more kinds thereof may be used in combination. The amount of the solvent to be used is generally 5- to 50-fold weight, preferably 8- to 20-fold weight, relative to compound (X).

As the Cbz agent, for example, benzyloxycarbonyl chloride and the like can be mentioned. The amount of the Cbz agent to be used is generally 0.9-2 equivalents, preferably 1-1.3 equivalents, relative to compound (X).

In the protection reaction, a base (e.g., sodium hydroxide, triethylamine, pyridine, sodium carbonate etc.) may be added. The amount of the base to be used is generally 0.9-3 equivalents, preferably 1-1.5 equivalents, relative to compound (X) .

The protection reaction is performed within the range of generally -20°C to 40°C (preferably 0°C to 20°C). The reaction completes within the above-mentioned temperature range, generally 30 min-24 hr (preferably 1 hr-16 hr).

The obtained compound (XIII) can be isolated and purified by a conventional method. After the completion of the reaction, for example, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.) and partitioned, and the obtained organic layer is concentrated to isolate compound (XIII). Furthermore, crystal precipitation may be performed, or the reaction mixture is subjected to silica gel column chromatography to purify compound (XIII), but the method is not limited to these.

### 11. Step (k)

In Step (k), a group represented by R⁸ of compound (XIII) or a salt thereof is converted to a group represented by Y to give compound (XIV) or a salt thereof.

Step (k) can be performed by a method and reaction conditions similar to those in the above-mentioned Step (g).

### 12. Step (1)

In Step (1), a protecting group Q of compound (XIV) or a salt thereof is eliminated by a known method such as the methods recited in page 309 ff. of Theodora W. Grrene, Peter G. M. Wuts, Protective Groups in Organic Synthesis, 2nd edn., A Wiley Interscience Publication, 1991 to give compound (XV) or a salt thereof.

Deprotection of a compound wherein Q is a Cbz group, which is a preferable embodiment of Step (1), is explained in the following, which is not to be construed as limiting Step (1).

Deprotection of Cbz group can be realized by, for example, hydrogenolysis of compound (XIV) in a solvent.

The solvent usable in the hydrogenolysis may be any as long as it does not inhibit the reaction, and, for example, ethanol, methanol, isopropanol, ethyl acetate, THF and the like can be mentioned. Two or more kinds thereof may be used in combination. The amount of solvent to be used is generally 5-to 50-fold weight, preferably 8- to 20-fold weight, relative to compound (XIV).

As the catalyst to be used for the hydrogenolysis, for example, palladium on carbon, palladium hydroxide and the like can be mentioned. The amount of the catalyst to be used is generally 0.01- to 0.5-fold weight, preferably 0.05- to 0.2-fold weight, relative to compound (XIV).

In the hydrogenolysis, an acid (e.g., hydrogen chloride, methanesulfonic acid etc.) may be added to promote the reaction. The amount of the acid to be used is generally 0.05-1.5 equivalents, preferably 0.1-1 equivalent, relative to compound (XIV) .

The reaction is performed within the range of generally 10°C to 80°C (preferably 20°C to 50°C. The reaction completes within the above-mentioned temperature range, generally 30 min-24 hr (preferably 1 hr-16 hr).

The obtained compound (XV) can be isolated and purified by a conventional method. After the completion of the reaction, for example, the catalyst is removed by filtration and the like, and alkali addition is applied in a protic solvent such as water, alcohol and the like, or the reaction mixture is washed with an aqueous alkali solution (e.g., saturated aqueous sodium hydrogen carbonate, brine etc.), and partitioned, and the obtained organic layer is concentrated to isolate compound (XV). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like is added to allow crystal precipitation, or the reaction mixture is subjected to silica gel column chromatography to purify a free form of compound (XV), but the method is not limited to these.

### 13. Step (m)

In Step (m), the 5-position P-C(=O)-, wherein P is as defined above, of a pyrimidine ring of compound (XI) is eliminated to give compound (XV) or a salt thereof.

For the deprotection, a method known to those of ordinary skill in the art, such as the amide-deprotection method described in the above-mentioned Theodora W. Grrene, Peter G. M. Wuts, Protective Groups in Organic Synthesis, 2nd edn., A Wiley Interscience Publication, 1991, can be applied without limitation. To avoid side reactions, such as decomposition and the like, the reaction is preferably performed under mild conditions, such as the conditions in the above-mentioned Step (i).

As preferable embodiments of Step (m), (1) deprotection by enzyme reaction [hereinafter to be also referred to as Step (m-1)] and (2) deprotection under acidic conditions [hereinafter to be also referred to as Step (m-2)] can be mentioned as in Step (i). While Step (m-1) and Step (m-2) are specifically explained in the following but Step (m) is not limited to these embodiments.

### 13-1. Step (m-1)

In Step (m-1), compound (XV) can be produced by, for example, reacting compound (XI) and an enzyme in a solvent to allow deprotection.

As the enzyme to be used for Step (m-1), those similar as in Step (i-1) can be mentioned, with preference given to penicillin amidase, penicillin acylase and the like.

As an enzyme, those immobilized on a resin and the like are preferably used, as in Step (i-1).

The amount of the enzyme or a solution obtained by treating mold to be used only need to be in an amount capable of showing the object effect (effective amount) and the effective amount can be easily determined by those of ordinary skill in the art through a simple preliminary test. In the case of, for example, a commercially available enzyme solution, it is 0.01 ml-2 ml per 1 g of compound (XI) to be a substrate.

While the protecting group of compound (XI), which is a starting material, can be appropriately determined based on the substrate characteristics of the enzyme to be used. When it is a preferable enzyme, penicillin amidase, a phenylacetyl group wherein P is benzyl is preferable.

As a solvent usable in Step (m-1), water or a mixture of hydrophilic solvent to the degree the enzyme activity is not impaired can be used, and use of water alone is preferable. As the hydrophilic solvent, one or more kinds of dimethyl sulfoxide, acetone and the like can be used. The amount of the solvent to be used is generally 5- to 200-fold weight, preferably 10- to 100-fold weight, relative to compound (XI).

The solvent is preferably adjusted to an optimal pH for an enzyme (e.g., about pH 6-8) with a buffer component, such as potassium dihydrogen phosphate, dipotassium hydrogen phosphate and the like.

While the concentration of a buffer component in a solvent is not particularly limited as long as it does not inhibit the enzyme reaction, it is about 0.001M - 0.2M.

It is also possible to adjust pH by appropriately adding a base and the like during the reaction.

In Step (m-1), a surfactant may be added as necessary to promote the reaction. As such surfactant, for example, Triton X-100 (trademark), Tween (trademark) and the like can be mentioned, with preference given to Triton X-100. The amount of the surfactant to be used is generally 0.001- to 0.05-fold weight, preferably 0.01- to 0.05-fold weight, relative to compound (IX).

Step (m-1) is performed within the range of generally 20°C to 50°C (preferably 30°C to 40°C). The reaction completes within the above-mentioned temperature range, generally 30 min-1 week (preferably 1 hr-20 hr).

After the completion of the reaction, the obtained compound (XV) can be separated from an enzyme by a conventional method. In the case of an immobilized enzyme, for example, a soluble solvent of compound (XV) (e.g., acetonitrile etc.) is added as necessary, and the mixture is filtered to separate compound (XV) in the filtrate. In the case of an unimmobilized enzyme, water is added to the reaction mixture as necessary to allow precipitation of compound (XV), which is collected by filtration to separate compound (XV) as a filtered substance, but the method is not limited to these methods.

To the separated compound (XV) is added, for example, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] to allow crystal precipitation, or the reaction mixture is subjected to silica gel column chromatography to purify compound (X), but the method is not limited to these.

### 13-2. Step (m-2)

In Step (m-2), compound (XV) can be produced by, for example, adding an acid to compound (XI) in a solvent to allow deprotection. The order of addition may be reverse or simultaneous.

The solvent usable in Step (m-2) may be any as long as it does not inhibit the reaction. When an acid is used, at least a protic solvent such as water, alcohol and the like needs to be present. As the solvent, for example, acetates (e.g., ethyl acetate, isopropyl acetate, isobutyl acetate, n-butyl acetate etc.), hydrocarbons (e.g., toluene, benzene, xylene etc.), acetonitrile, THF, alcohols (e.g., methanol, ethanol, isopropyl alcohol, n-butanol etc.), N,N-dimethylformamide and the like can be mentioned. These may be used in combination of two or more kinds thereof and methanol, ethanol, acetonitrile, isopropyl alcohol, water and the like are preferable, and a single or mixed solvent of methanol, acetonitrile, water and the like is particularly preferable. The amount of the solvent to be used is generally 3- to 50-fold weight, preferably 5- to 20-fold weight, relative to compound (XI) .

As an acid to be used in Step (m-2), inorganic acids (e.g., hydrogen chloride, sulfuric acid etc.), organic acids (e.g., methanesulfonic acid, p-toluenesulfonic acid, trifluoroacetic acid etc.) and the like can be mentioned, with preference given to hydrogen chloride and the like.

The amount of the acid to be used is generally 1-50 equivalents, preferably 3-10 equivalents, relative to compound (XI).

The acid is preferably used in the form of a solution of the solvent to be used, wherein a solvent and an acid are added such that the amount thereof fall within the above-mentioned ranges.

Step (m-2) is performed within the range of generally from 0°C to the refluxing temperature of the solvent to be used (preferably 20°C to 60°C). The reaction completes within the above-mentioned temperature range, generally 60 min-one night (preferably 1 hr-5 hr).

After the completion of the reaction, compound (XV) is present in the reaction mixture in the form of a salt with the acid used. Thus, the acid addition salt can be isolated by concentrating the reaction mixture. The isolated acid addition salt can be purified by adding a crystal precipitation solvent [e.g., ethers (e.g.: diethyl ether, THF etc.), acetone, acetonitrile, hydrocarbon solvents (e.g., toluene, benzene, hexane, heptane etc..), halogen solvents (e.g., dichloromethane, dichloroethane etc.), alcohols (e.g., methanol, ethanol, isopropanol etc.), water or a mixed solvent thereof etc.] thereto to allow crystal precipitation.

For isolation of a free form of compound (XV), alkali addition in a protic solvent such as water, alcohol and the like is applied, or, after the completion of the reaction, the reaction mixture is washed with an aqueous alkali solution (e.g., saturated aqueous sodium hydrogen carbonate, brine etc.) and partitioned, and the obtained organic layer is concentrated, whereby compound (XV) can be purified. Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like are added to allow crystal precipitation, or the reaction mixture is subjected to silica gel column chromatography to purify a free form of compound (XV), but the method is not limited to these.

### Examples

The present invention is explained in more detail by referring to Examples, which are not to be construed as limitative.

### Reference Example 1

### 4-(N,N-dimethylaminomethylene)-2-methyl-5-oxazolinone

To N-acetylglycine (20.0 g, 171 mmol) were added phosphorus oxychloride (67.0 g, 437 mmol) and N,N-dimethylformamide (33.0 g, 451 mmol) in an ice bath, and the mixture was stirred at 45°C for 1.5 hr. The reaction mixture was concentrated under reduced pressure, and added dropwise to 28% aqueous ammonia (150 ml) while maintaining not more than 10°C. The mixture was stirred in an ice bath for 1 hr and the precipitate was collected by filtration. The obtained crystals were washed successively with water and ethanol and dried to give the title compound as crystals (20.2 g, 131 mmol).,
¹H-NMR (CDCl₃) δ: 2.21(3H, s) , 3.18(3H, m) , 3.47(3H, s) , 6.96 (1H, s).
MS (ESI) m/z [MH]+ 155.2

### Reference Example 2

### 2-benzyl-4-(N,N-dimethylaminomethylene)-5-oxazolinone

To phenaceturic acid (10.0 g, 51.8 mmol) were added chloroform (30.0 ml), phosphorus oxychloride (20.0 g, 130 mmol) and N,N-dimethylformamide (10.0 g, 137 mmol) in an ice bath and the mixture was stirred at 45°C for 1.5 hr. The reaction mixture was concentrated under reduced pressure, and added dropwise to 28% aqueous ammonia (65 ml) while maintaining not more than 10°C. The mixture was extracted with chloroform, and the organic layer was washed with water and saturated brine and concentrated to dryness. The concentrate was washed with isopropyl alcohol, and the precipitate was collected by filtration, is washed with isopropyl alcohol and dried to give the title compound as crystals (9.90 g, 43.3 mmol).
¹H-NMR(CDCl₃) δ: 3.17(3H, s), 3.48 (3H, m), 3.83(2H, s), 6.97 (1H, s), 7.23-7.36(5H, m)
MS(ESI) m/z [MH]+ 231.5

### Reference Example 3

### 2-methyl-4-(morpholinomethylene)-5-oxazolinone

To N-acetylglycine (0.50 g, 4.27 mmol) were added phosphorus oxychloride (995 µl, 10.7 mmol) and N-formylmorpholine (1.23 g, 10.7 mmol) in an ice bath, and the mixture was stirred at 45°C for 1.5 hr. The reaction mixture was concentrated under reduced pressure, and added dropwise to 28% aqueous ammonia (5 ml) and water (5 ml) while maintaining not more than 10°C. The reaction mixture was stirred in an ice bath for 1 hr and the precipitate was collected by filtration. The obtained crystals were washed successively with water and ethanol, and dried to give the title compound as crystals (0.52 g, 2.65 mmol) .
¹H-NMR(CDCl₃) δ: 2.21(3H, s), 3.47(2H, br), 3.79(4H, t, J=4.8Hz), 4.27(2H, s), 6.92 (1H, s)
MS(ESI) m/z [MH]+ 196.9

### Reference Example 4

### 2-benzyl-4-(N,N-dimethylaminomethylene)-5-oxazolinone

To phenaceturic acid (1.00 g, 5.18 mmol) were added toluene (10.0 ml) and N,N'-dicyclohexylcarbodiimide (1.07 g, 5.19 mmol) and the mixture was stirred at room temperature overnight. The precipitate was collected by filtration and N,N-dimethylformamide dimethylacetal (0.68 g, 5.71 mmol) was added. The mixture was stirred overnight. The reaction mixture was washed with saturated brine and concentrated to dryness. Isopropyl alcohol was added to the concentrate to allow crystal precipitation, and the precipitate was collected by filtration, washed with isopropyl alcohol and vacuum dried to give the title compound as crystals (0.78 g, 3.39 mmol).

### Example 1

### 4-hydroxymethylene-2-methyl-5-oxazolinone sodium salt

To a solution (50 ml) of 4-(N,N-dimethylaminomethylene)-2-methyl-5-oxazolinone (4.00 g, 26.0 mmol) in acetonitrile was added 2N aqueous sodium hydroxide solution (15 ml, 30.0 mmol) under ice-cooling and the mixture was stirred overnight. Water was evaporated and acetonitrile (30 ml) was added, and the mixture was stirred. The precipitate was collected by filtration, washed with acetonitrile and vacuum dried to give the title compound as crystals (3.65 g, 24.5 mmol).
¹H-NMR(DMSO-d₆) δ: 2.00(3H, s), 8.67(3H, s)
MS(API-ES) m/z [MH]+ 126.1

### Example 2

### 2-benzyl-4-hydroxymethylene-5-oxazolinone sodium salt· monohydrate

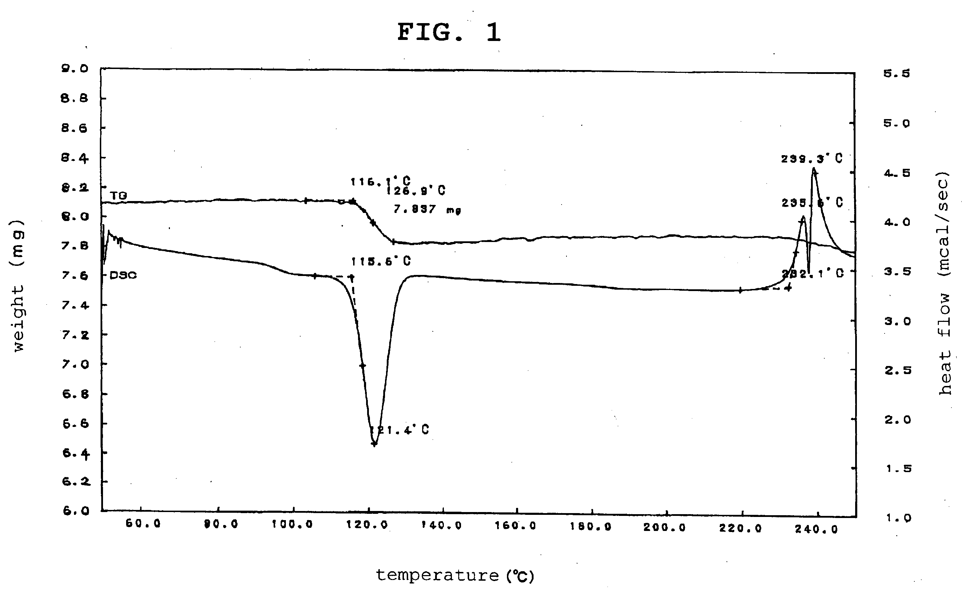

To a solution (120 ml) of 2-benzyl-4-(N,N-dimethylaminomethylene)-5-oxazolinone (10.8 g, 46.9 mmol) in acetonitrile was added 1N aqueous sodium hydroxide solution (53 ml, 53.0 mmol) under ice-cooling and the mixture was stirred overnight. Acetonitrile was evaporated to allow crystal precipitation under ice-cooling. The precipitate was collected by filtration and vacuum dried to give the title compound as crystals (5.99 g, 26.6 mmol). Differential scanning calorie (DSC) and thermal gravity (TG) were measured using a thermal analyzer (Rigaku Corporation, TAS-200). As a result, an endothermic peak appeared at 121.4°C and weight decrease was observed at 116.1-126.9°C. The chart is shown in Fig. 1.
¹H-NMR(DMSO-d₆) δ: 3.66(2H, s) , 7.22-7.32 (5H, m) , 8.71 (1H, s) MS(ESI) m/z [MH]- 202.1

### Example 3

### 2-benzyl-4-hydroxymethylene-5-oxazolinone sodium salt·anhydride

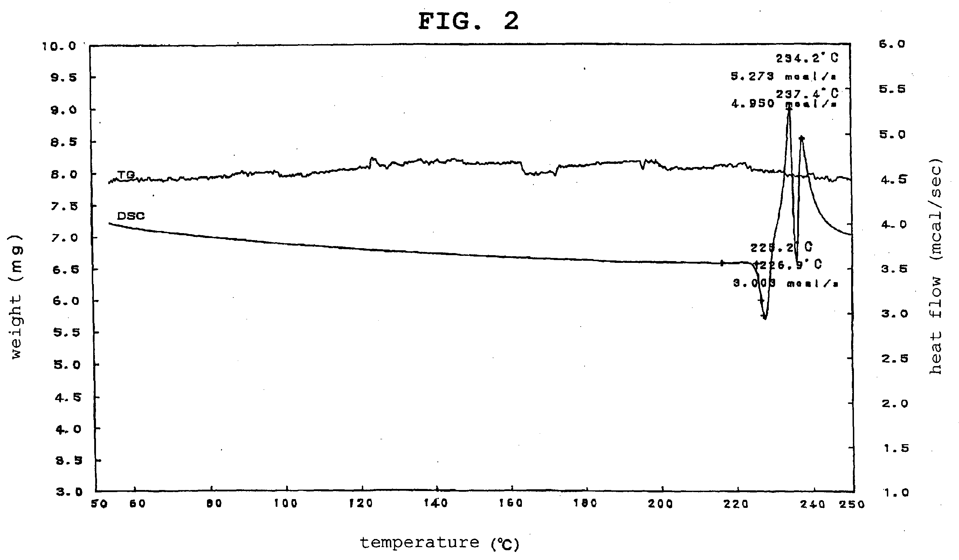

To a solution (10 ml) of 2-benzyl-4-(N,N-dimethylaminomethylene)-5-oxazolinone (1.00 g, 4.34 mmol) in acetonitrile was added 1N aqueous sodium hydroxide solution (5 ml, 5.00 mmol) under ice-cooling and the mixture was stirred overnight. The reaction mixture was concentrated to dryness. Acetonitrile (5 ml) was added and the precipitate was collected by filtration and dried at 100°C for 2 hr to give the title compound as crystals (0.90 g, 4.00 mmol). DSC and TG were measured using a thermal analyzer. As a result, an endothermic peak and clear weight change were not observed up to 220°C.
The chart is shown in Fig. 2.
¹H-NMR(DMSO-d₆) δ: 3.66(2H, s), 7.22-7.32(5H, m), 8.71 (1H, s) MS(ESI) m/z [MH]- 202.1

### Example 4

### 2-benzyl-4-hydroxymethylene-5-oxazolinone

To a solution (15 ml) of 4-N,N'-dimethylaminomethylene-2-benzyl-5-oxazolinone (350 mg, 6.65 mmol) in acetonitrile was added 2N aqueous sodium hydroxide solution (0.92 ml) under ice-cooling and the mixture was stirred overnight at room temperature. The reaction mixture was concentrated to evaporate acetonitrile. The mixture was washed with ethyl acetate (5 ml) and the aqueous layer was neutralized with 1N hydrogen chloride to pH 4 under ice-cooling. The precipitate was collected by filtration, washed with water and vacuum dried to give the title compound as crystals (1.03 g, 5.07 mmol). ¹H-NMR(DMSO-d₆) δ: 3.92 (2H, s) , 7.29-7.38 (5H, m) , 7. 68 (1H, s) MS(ESI) m/z [MH]- 202.3

### Example 5

### 4-methoxymethylene-2-methyl-5-oxazolinone

To 4-hydroxymethylene-2-methyl-5-oxazolinone sodium salt (3.00 g, 20.1 mmol) were added N,N-dimethylformamide (30 ml) and dimethyl sulfate (2.20 ml, 23.2 mmol) and the mixture was stirred at 60°C for 5 hr. The solvent was evaporated. Ethyl acetate was added and the precipitated salt was filtered off. The filtrate was concentrated under reduced pressure and the residue was purified by silica gel column chromatography to give the title compound (1.73 g, 12.2 mmol).
¹H-NMR(CDCl₃) δ: 2.28(3H, s), 4.11(3H, s), 7.13(1H, s)
MS (APCI) m/z [MH]+ 142.1

### Example 6

### 4-ethoxymethylene-2-methyl-5-oxazolinone

In the same manner as in Example 5 except that diethyl sulfate was used instead of dimethyl sulfate, the title compound was obtained.
¹H-NMR(CDCl₃) δ: 1.45 (3H, t, J=7.1Hz), 2.29 (3H, s), 4.35 (2H, q, J=7.1 Hz), 7.21(1H, s)

### Example 7

### 4-benzyloxymethylene-2-methyl-5-oxazolinone

To 4-hydroxymethylene-2-methyl-5-oxazolinone sodium salt (0.42 g, 2.81 mmol) were added N,N-dimethylformamide (5 ml) and benzyl bromide (0.53 g, 3.10 mmol) and the mixture was stirred at 80°C for 5 hr. The reaction mixture was concentrated, toluene was added and the mixture was washed successively with saturated aqueous sodium hydrogen carbonate solution and saturated brine. The organic layer was concentrated to dryness and isopropyl alcohol was added to the residue to allow crystal precipitation. The precipitate was collected by filtration, washed with isopropyl alcohol and vacuum dried to give the title compound as crystals (0.37 g, 1.70 mmol).
¹H-NMR(CDCl₃) δ: 2.27 (3H, s) , 5.26 (2H, s) , 7.19-7.38(6H, m) MS(ESI) m/z [MH]+ 218.3

### Example 8

### 2-benzyl-4-methoxymethylene-5-oxazolinone

To 2-benzyl-4-hydroxymethylene-5-oxazolinone sodium salt (3.00 g, 13.3 mmol) were added acetonitrile (30 ml) and dimethyl sulfate (1.64 ml, 17.3 mmol) and the mixture was stirred at 60°C for 4 hrs. The solvent was evaporated and ethyl acetate and n-hexane were added. The precipitated salt was filtered off. The filtrate was concentrated under reduced pressure, and the residue was purified by silica gel column chromatography to give the title compound (1.99 g, 9.16 mmol). ¹H-NMR(CDCl₃) δ: 3.86(2H, s), 4.10(3H, s), 7.15(1H, s), 7.25-7.37(5H, m)
MS (ESI) m/z [MH]+ 218.2

### Example 9

### 2-benzyl-4-ethoxymethylene-5-oxazolinone

To 2-benzyl-4-hydroxymethylene-5-oxazolinone sodium salt (3.00 g, 13.3 mmol) were added acetonitrile (30 ml) and diethyl sulfate (2.28 ml, 17.4 mmol) and the mixture was stirred at 60°C for 12 hr. Ethyl acetate was added and the mixture was washed successively with saturated aqueous sodium hydrogen carbonate solution and saturated brine. The organic layer was concentrated to dryness to give the title compound (2.60 g, 11.2 mmol).
¹H-NMR(CDCl₃) δ: 1.43 (3H, t, J=7.1Hz), 3. 86 (2H, s) , 4.28 (2H, q, J=7.1Hz), 7.21 (1H, s) , 7.24-7.35(5H, m)
MS(ESI) m/z [MH]+ 231.8

### Example 10

### 2-benzyl-4-ethoxymethylene-5-oxazolinone

To 4-hydroxymethylene-2-benzyl-5-oxazolinone (0.56 g, 2.76 mmol) were added acetonitrile (6 ml), triethylamine (0.5 ml, 3.60 mmol) and diethyl sulfate (0.47 ml, 3.60 mmol) and the mixture was stirred at 80°C for 5 hr. The reaction mixture was concentrated and ethyl acetate was added. The mixture was washed successively with saturated aqueous sodium hydrogen carbonate solution and saturated brine. The organic layer was concentrated to dryness to give the title compound (0.54 g, 2.34 mmol) .

### Example 11

### 2-benzyl-4-benzyloxymethylene-5-oxazolinone

To 2-benzyl-4-hydroxymethylene-5-oxazolinone sodium salt (0.60 g, 2.66 mmol) were added acetonitrile (6 ml) and benzyl bromide (0.48 g, 2.81 mmol) and the mixture was stirred at 80°C for 7 hr. The reaction mixture was concentrated and ethyl acetate was added. The mixture was washed successively with saturated aqueous sodium hydrogen carbonate solution and saturated brine. The organic layer was concentrated to dryness to give the title compound (0.69 g, 2.35 mmol) .
¹H-NMR(CDCl₃) δ: 3.86 (2H, s) , 5.28 (2H, s) , 7.26-7.39 (11H, m) MS(ESI) m/z [MH]+ 294.6

### Example 12

### (5-acetylamino-6-oxo-2-phenyl-1,6-dihydropyrimidin-1-yl)acetic acid

To N-(carboxymethyl)phenylamidine (0.40 g, 2.25 mmol) were added isopropyl alcohol (8 ml) and 21% solution of sodium ethoxide in ethanol (0.73 g, 2.26 mmol) and the mixture was stirred for 0.5 hr. Then, crystals of 4-benzyloxymethylene-2-methyl-5-oxazolinone (0.49 g, 2.25 mmol) were added in 4 portions at 50°C over 1 hr. The mixture was stirred overnight at 80°C, concentrated and ethyl acetate (5 ml) and water (5 ml) were added to allow partitioning. The aqueous layer was adjusted to pH 2 with hydrogen chloride and extracted with ethyl acetate. Hexane was added to the organic layer to allow crystal precipitation and the precipitate was collected by filtration and vacuum dried to give the title compound as yellow crystals (0.44 g, 1.53 mmol).
¹H-NMR(DMSO-d₆) δ: 2.15 (3H, s), 4.52(2H, s), 7.48-7. 56 (5H, m), 8.83(1H, s), 9.58(1H, s)
MS(ESI) m/z [MH]+ 288.2, [MH]- 286.1

### Example 13

### (6-oxo-2-phenyl-5-phenylacetylamino-1,6-dihydropyrimidin-1-yl)acetic acid

In the same manner as in Example 12 except that 2-benzyl-4-benzyloxymethylene-5-oxazolinone was used instead of 4-benzyloxymethylene-2-methyl-5-oxazolinone, the title compound was obtained.
¹H-NMR(DMSO-d₆) δ: 3.84(2H, s), 4.52(2H, s), 7.25-7.27 (1H, m), 7.31-7.34(4H, m), 7.47-7.55(5H, m), 8.83 (1H, s), 9.72 (1H, s) MS(ESI) m/z [MH]+ 364.0, [MH]- 361.6

### Example 14

### 1-(tert-butoxycarbonylmethyl)-6-oxo-2-phenyl-5-phenylacetylamino-1,6-dihydropyrimidine

N-(tert-Butoxycarbonylmethyl)phenylamidine hydrochloride (2.00 g, 7.39 mmol) and sodium carbonate (2.30 g, 21.7 mmol) were added to toluene (25 ml) and water (10 ml), and the mixture was stirred vigorously. The organic layer was washed with saturated brine. To this organic layer was dropwise added a solution of 4-ethoxymethylene-2-benzyl-5-oxazolinone (1.38 g, 5.97 mmol) in acetonitrile (5 ml) and the mixture was stirred overnight at 80°C. The reaction mixture was allowed to cool to room temperature, washed with 1N aqueous hydrogen chloride solution, saturated aqueous sodium hydrogen carbonate solution and saturated brine, and concentrated under reduced pressure. Hexane was added to the residue to allow crystal precipitation, and the precipitate was collected by filtration and vacuum dried to give the title compound as pale-yellow crystals (2.03 g, 4.84 mmol).
¹H-NMR(DMSO-d₆) δ: 1.40 (9H, s) , 3.81 (2H, s) , 4.58 (2H; s) , 7.33-7.44(5H, m), 7.53-7.67(5H, m), 8.02 (1H, s), 9.10(1H, s)
MS(ESI) m/z [MH]+ 420.4

### Example 15

### 1-(tert-butoxycarbonylmethyl)-6-oxo-2-phenyl-5-phenylacetylamino-1,6-dihydropyrimidine

### (1) 2-benzyl-4-methanesulfonyloxymethylene-5-oxazolinone

2-Benzyl-4-hydroxymethylene-5-oxazolinone sodium salt (100 mg, 0.444 mmol) was suspended in chloroform (3 ml) and methanesulfonyl chloride (34.3 µl, 0.443 mmol) was added. The mixture was stirred at room temperature for 6 hr. A part of the reaction mixture was sampled and analyzed by ¹H-NMR to confirm that 2-benzyl-4-methanesulfonyloxymethylene-5-oxazolinone was contained.
¹H-NMR(CDCl₃) δ: 3.67(2H, s), 3.87(1.5H, s), 3.90(1.5H, s), 7.28-7.33(6H, m)

### (2) 1-(tert-butoxycarbonylmethyl)-6-oxo-2-phenyl-5-phenylacetylamino-1,6-dihydropyrimidine

A mixture of N-(tert-butoxycarbonylmethyl)-phenylamidine hydrochloride (155 mg, 0.57 mmol), sodium carbonate (200 mg, 1.89 mmol), toluene (2 ml) and water (2 ml) was stirred for 10 min. The organic layer was washed with saturated brine. To the organic layer was added triethylamine (62 µl, 0.445 mmol) and then the reaction mixture of Example 15(1) was added, and the mixture was stirred overnight at 80°C. The reaction mixture was washed successively with 1N aqueous hydrogen chloride and saturated brine. The organic layer was concentrated and purified by silica gel chromatography (ethyl acetate/hexane) to give the title compound as crystals (84 mg, 0.20 mmol) .

### Example 16

### 1-(tert-butoxycarbonylmethyl)-6-oxo-2-phenyl-5-phenylacetylamino-1,6-dihydropyrimidine

### (1) 2-benzyl-4-trimethylsilyloxymethylene-5-oxazolinone.

2-Benzyl-4-hydroxymethylene-5-oxazolinone sodium salt (100 mg, 0.444 mmol) was suspended in chloroform (3 ml), trimethylsilyl chloride (61.9 µl, 0.498 mmol) was added and the mixture was stirred at room temperature for 2 hr. After the completion of the reaction, a part of the reaction mixture was sampled and analyzed by ¹H-NMR to confirm that 2-benzyl-4-trimethylsilyloxymethylene-5-oxazolinone was contained.
¹H-NMR (CDCl₃) δ: 0.30(9H, s), 3.82(2H, s), 7.20-7.27 (6H, m)

### (2) 1-(tert-butoxycarbonylmethyl)-6-oxo-2-phenyl-5-phenylacetylamino-1,6-dihydropyrimidine

A mixture of N-(tert-butoxycarbonylmethyl)-phenylamidine hydrochloride (155 mg, 0.57 mmol), sodium carbonate (200 mg, 1.89 mmol) , toluene (2 ml) and water (2 ml) was stirred for 10 hr. The organic layer was washed with saturated brine and the reaction mixture of Example 16(1) was further added. The mixture was stirred overnight at 80°C. The reaction mixture was washed successively with 1N aqueous hydrogen chloride and saturated brine. The organic layer was concentrated and purified by silica gel chromatography to give the title compound as crystals (121 mg, 0.288 mmol).

### Example 17

### 1-(tert-butoxycarbonylmethyl)-6-oxo-2-phenyl-5-phenylacetylamino-1,6-dihydropyrimidine

### (1) 4-acetoxymethylene-2-benzyl-5-oxazolinone

To 2-benzyl-4-hydroxymethylene-5-oxazolinone (100 mg, 0.492 mmol) was added acetic anhydride (0.5 ml, 5.29 mmol) and the mixture was stirred at room temperature for 1 hr. The reaction mixture was washed by adding cold water and then washed by adding ethyl acetate and saturated brine. The organic layer was vacuum dried to give 4-acetoxymethylene-2-benzyl-5-oxazolinone.
¹H-NMR (CDCl₃) δ: 2.36(3H, s), 4.08(2H, s) , 7.28-7.37(5H, m) , 8.11 (1H, s)
MS(ESI) m/z [MH]+ 264.1, [MH]- 261.9

### (2) 1-(tert-butoxycarbonylmethyl)-6-oxo-2-phenyl-5-phenylacetylamino-1,6-dihydropyrimidine

A mixture of N-(tert-butoxycarbonylmethyl)-phenylamidine hydrochloride (155 mg, 0.572 mmol), sodium carbonate (200 mg, 1.89 mmol) , toluene (2 ml) and water (2 ml) was stirred for 10 hr. The organic layer was washed with saturated brine and 4-acetoxymethylene-2-benzyl-5-oxazolinone obtained in Example 17(1) was added to the organic layer. The mixture was stirred overnight at 80°C. The reaction mixture was washed with 1N aqueous hydrogen chloride and saturated brine. The organic layer was concentrated and purified by silica gel column chromatography to give the title compound as crystals (70.0 mg, 0.167 mmol).

### Example 18

### 5-acetylamino-6-oxo-2-phenyl-1-(succinimidoxycarbonylmethyl)-1,6-dihydropyrimidine

(5-Acetylamino-6-oxo-2-phenyl-1,6-dihydropyrimidin-1-yl)acetic acid (300 mg, 1.04 mmol), acetonitrile (5 ml) and N-methylmorpholine (121 mg, 1.20 mmol) were mixed and ethyl chloroformate (125 mg, 1.15 mmol) was added. The mixture was stirred under ice-cooling for 30 min. N-Hydroxysuccinimide (148 mg, 1.29 mmol) was added and the mixture was stirred for 5 hr. The reaction mixture was concentrated under reduced pressure and ethyl acetate was added. The mixture was washed successively with saturated aqueous sodium hydrogen carbonate solution and saturated brine and concentrated to dryness. The residue was subjected to slurry washing with water (5 ml) and the precipitate was collected by filtration. The crystals were dried to give the title compound as pale-yellow crystals (0.31 g, 0.81 mmol).
¹H-NMR(CDCl₃) δ: 2.23(3H, s) , 2.88(4H, s), 4.94(2H, s), 7.50-7.56(5H, m), 8.04(1H, s), 9.10(1H, s)
MS(ESI) m/z [MH]+ 385.2, [MH]- 383.2

### Example 19

### (S)-2-[2-(5-acetylamino-6-oxo-2-phenyl-1,6-dihydropyrimidin-1-yl)acetylamino]-3-phenylpropionic acid

L-Phenylalanine (100 mg, 0.61 mmol) was added to 50% aqueous acetonitrile (3 ml), and the mixture was adjusted to pH 8 with 6N aqueous sodium hydroxide solution to allow dissolution. To this solution was added dropwise a solution of 5-acetylamino-6-oxo-2-phenyl-1-(succinimidoxycarbonylmethyl)-1,6-dihydropyrimidine (176 mg, 0.46 mmol) in acetonitrile (1 ml) under ice-cooling. The mixture was stirred under ice-cooling for 2 hr and further stirred at room temperature for 3 hr. The reaction mixture was concentrated and adjusted to pH 2 with aqueous hydrogen chloride solution to allow crystal precipitation. The precipitate was collected by filtration, washed and vacuum dried to give the title compound as white crystals (190 mg, 0.44 mmol).
¹H-NMR(DMSO-d₆) δ: 2.14(3H, s), 2.84-2.87(1H, dd, J1=13.8Hz, J2=5.0Hz), 3.01(1H, dd, J1=13.8Hz, J2=8.7Hz), 4.41-4.48(3H, m), 7.12-7.14(2H, d, J=7.8Hz), 7.21-7.25(3H, m), 7.43-7.52(5H, m), 8.53(1H, d, J=7.8Hz), 8.80(1H, s), 9.52(1H, s)
MS(ESI) m/z [MH]+ 435.2, [MH]- 433.3

### Example 20

### (S)-2-[(6-oxo-2-phenyl-5-phenylacetylamino-1,6-dihydropyrimidin-1-yl)acetylamino]-3-phenylpropionic acid

In the same manner as in Example 19 except that 6-oxo-2-phenyl-5-phenylacetylamino-1-(succinimideoxycarbonylmethyl)-1,6-dihydropyrimidine was used instead of 5-acetylamino-6-oxo-2-phenyl-1-(succinimidoxycarbonylmethyl)-1,6-dihydropyrimidine, the title compound was obtained.
¹H-NMR(DMSO-d₆) δ: 2.85(1H, dd, J1=14.1Hz, J2=5.1Hz), 3.02(1H, dd, J1=14.1Hz, J2=5.1Hz), 3.83(2H, s), 4.45(3H, m), 7.15-7.52(15H, m), 8.53(1H, d, J=7.8Hz), 8.80(1H, s), 9 .64 (1H, s) MS(ESI) m/z [MH]+ 511.3, [MH]- 509.4

### Example 21

### methyl (S)-2-[2-(5-acetylamino-6-oxo-2-phenyl-1,6-dihydropyrimidin-1-yl)acetylamino]-3-methylbutyrate

To (5-acetylamino-6-oxo-2-phenyl-1,6-dihydropyrimidin-1-yl)acetic acid (250 mg, 0.87 mmol) were added tetrahydrofuran (5 ml) and N-methylmorpholine (0.10 ml, 0.91 mmol) to allow dissolution. Under ice-cooling, ethyl chloroformate (85.3 µl, 0.91 mmol) was added and the mixture was stirred for 20 min. L-valine methyl ester hydrochloride (146 mg, 0.87 mmol) and N-methylmorpholine (0.10 ml, 0.91 mmol) were added and the mixture was stirred for 2 hr. Ethyl acetate was added to the reaction mixture and the mixture was washed successively with 1N aqueous hydrogen chloride, saturated aqueous sodium hydrogen carbonate solution and saturated brine. The organic layer was concentrated to dryness to give the title compound (0.18 g, 0.45 mmol) .
¹H-NMR(CDCl₃) δ: 0.92(3H, d, J=6.9Hz), 0.94(3H, d, J=6.9Hz), 2.16-2.19 (1H, m), 2.21 (3H, S), 3.75 (3H, s), 4.58(3H, m), 6.37(1H, d, J=8.6Hz), 7.43-7.50(3H, m), 7.55-7.57(2H, m), 8.01(1H, s) 9.10(1H, s)

### Example 22

### N-[2-(5-tert-butyl-1,3,4-oxadiazol-2-yl)-1-methyl-2-oxoethyl]-(6-oxo-2-phenyl-5-phenylacetylamino-1,6-dihydropyrimidin-1-yl)acetamide

To (6-oxo-2-phenyl-5-phenylacetylamino-1,6-dihydropyrimidin-1-yl)acetic acid (600 mg, 1.65 mmol) were added tetrahydrofuran (8 ml) and N-methylmorpholine (0.20 ml, 1.82 mmol) to allow dissolution. Under ice-cooling, ethyl chloroformate (0.165 ml, 1.73 mmol) was added and the mixture was stirred for 20 min. 2-Amino-1-(5-tert-butyl-1,3,4-oxadiazol-2-yl)-1-propanone hydrochloride (386 mg, 1.65 mmol) and N-methylmorpholine (0.20 ml, 1.82 mmol) were added and the mixture was stirred for 1 hr. Methyl-tert-butyl ether was added to the reaction mixture and the mixture was washed successively with 1N aqueous hydrogen chloride, saturated aqueous sodium hydrogen carbonate solution and saturated brine. The organic layer was concentrated to dryness to give the title compound (0.61 g, 1.12 mmol).
¹H-NMR (CDCl₃) δ: 1.48(9H, s) , 1.53 (3H, d, J=4.2Hz), 3.76(2H,s), 4.57(2H, s), 5.43-5.51(1H, m), 6.76(1H, d, J=6.8Hz), 7.25-7.32(5H, m), 7.43-7.53(5H, m), 8.05(1H, s), 9.09(1H, s)
MS(ESI) m/z [MH]+ 543.4, [MH]- 541.4

### Example 23

### N-[2-(5-tert-butyl-1,3,4-oxadiazol-2-yl)-1-methyl-2-oxoethyl]-(5-acetylamino-6-oxo-2-phenyl-1,6-dihydropyrimidin-1-yl)acetamide

In the same manner as in Example 22 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, the title compound was obtained.
¹H-NMR(CDCl₃) δ: 1.48(9H, s), 1.59(3H, d, J=7.3Hz), 2.23(3H, s), 4.62(2H, s), 5.48-5.54(1H, m), 6.79(1H, d, J=6.9Hz), 7.47-7.56(5H, m), 8.02 (1H, s), 9.08 (1H, s)
MS(ESI) m/z [MH]+ 467.2, [MH]- 465.4

### Example 24

### N-[2-methyl-1-(2-thiazolylcarbonyl)propyl]-(6-oxo-2-phenyl-5-phenylacetylamino-1,6-dihydropyrimidin-1-yl)acetamide

To (6-oxo-2-phenyl-5-phenylacetylamino-1,6-dihydropyrimidin-1-yl)acetic acid (400 mg, 1.10 mmol) were added tetrahydrofuran (6 ml) and N-methylmorpholine (0.133 ml, 1.21 mmol) for dissolution. Under ice-cooling, ethyl chloroformate (0.11 ml, 1.15 mmol) was added and the mixture was stirred for 20 min. 2-Amino-3-methyl-1-(2-thiazolyl)-1-butanone hydrochloride (250 mg, 1.13 mmol) and N-methylmorpholine (0.133 ml, 1.21 mmol) were added and the mixture was stirred for 1 hr. Ethyl-tert-butyl ether was added to the reaction mixture, and the mixture was washed successively with 1N aqueous hydrogen chloride, saturated aqueous sodium hydrogen carbonate solution and saturated brine. The organic layer was concentrated to dryness to give the title compound (556 mg, 1.05 mmol).
¹H-NMR(CDCl₃) δ: 0.76 (3H, d, J=6.8Hz) , 0.99(3H, d, J=6.8Hz), 2.37-2.45(1H, m), 3.75(2H, s), 4.11(1H, dd, J1=14.3Hz, J2=7.1Hz), 4.60-4.70(2H, m), 5.65-5.68(1H, dd, J1=8.9Hz, J2=4.9Hz), 7.05 (1H, d, J=8.9Hz), 7.23-7.53(10H, m), 7.69(1H, d, J=3.0Hz), 7.99(1H, d, J=3.0Hz), 8.16(1H, s), 9.08(1H, s)
MS(ESI) m/z [MH]+ 530.3, [MH]- 528.3

### Example 25

### N-[2-methyl-1-(2-thiazolylcarbonyl)propyl]-(5-acetylamino-6-oxo-2-phenyl-1,6-dihydropyrimidin-1-yl)acetamide

In the same manner as in Example 24 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, the title compound was obtained.
¹H-NMR(CDCl₃) δ: 0.84(3H, d, J=6.9Hz), 1.05(3H, d, J=6.9Hz), 2.22(3H, s), 2.48(1H, m), 4.61(1H, d, J=16Hz), 4.68(1H, d, J=16Hz), 5,67(1H, dd, J1=8.8Hz, J2=4.8Hz), 6.78(1H, d, J=8.8Hz), 7.41-7.46(3H, m), 7.54-7.56(2H, m), 7.73(1H, d, J=3.0Hz), 8.00(1H, s), 8.04(1H, d, J=3.0Hz), 9.09(1H, s)
MS(ESI) m/z [MH]+ 454.2, [MH]- 452.4

### Example 26

### (S)-N-[1-benzyl-3-chloro-2-oxopropyl]-(5-acetylamino-6-oxo-2-phenyl-1,6-dihydropyrimidin-1-yl)acetamide

In the same manner as in Example 22 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, and (S)-3-amino-1-chloro-4-phenyl-2-butanone hydrochloride was used instead of 2-amino-1-(5-tert-butyl-1,3,4-oxadiazol-2-yl)-1-propanone hydrochloride, the title compound was obtained.
¹H-NMR(CDCl₃) δ: 2.24(3H, s), 3.02-3.07 (1H, dd, J1=14.0Hz, J2=6.9Hz), 3.10-3.16 (1H, dd, J1=14.0Hz, J2=6.9Hz), 4.00(1H, d, J=16.1Hz), 4.15(1H, d, J=16.1Hz) 4.48(1H, d, J=15.6Hz), 4.56(1H, d, J=15.6Hz), 4.98-5.03(1H, m), 6.51(1H, d, J=7.2Hz), 7.10-7.12(2H, m), 7.26-7.29 (3H, m), 7.45-7.49(5H, m), 7.96 (1H, s), 9.10(1H, s)
MS(ESI) m/z [MH]+ 467.2, [MH]- 465.2

### Example 27

### (S)-2-[(5-amino-6-oxo-2-phenyl-1,6-dihydropyrimidin-1-yl)acetylamino]-3-phenylpropionic acid

To (S)-2-[(6-oxo-2-phenyl-5-phenylacetylamino-1,6-dihydropyrimidin-1-yl)acetylamino]-3-phenylpropionic acid (135 mg, 0.264 mmol) were added water (10 ml) and potassium dihydrogen phosphate (155 mg, 1.14 mmol) and the mixture was adjusted to pH 7.6 with aqueous sodium hydroxide solution. Penicillin amidase beads (23.0 mg, Sigma) were added and the mixture was stirred overnight at 37°C. Acetonitrile was added to the obtained reaction mixture and the precipitate was filtered off. The filtrate was concentrated and aqueous hydrogen chloride solution was added to adjust to pH 2.0 to allow crystal precipitation. The precipitate was collected by filtration, washed and vacuum dried to give the title compound as crystals (85.0 mg, 0.217 mmol) .
¹H-NMR(DMSO-d₆) δ: 2.83-2.88(1H, m), 3.00-3.03(1H, m), 4.33-4.47(3H, m), 5.14 (2H, br), 7.14-7.45 (10H, m), 8.44(1H, d, J=7.8Hz)
MS(ESI) m/z [MH]+ 393.3, [MH]- 391.2

### Example 28

### N-[2-methyl-1-(2-thiazolylcarbonyl)propyl]-(5-amino-6-oxo-2-phenyl-1,6-dihydropyrimidin-1-yl)acetamide

N-[2-Methyl-1-(2-thiazolylcarbonyl)propyl]-(6-oxo-2-phenyl-5-phenylacetylamino-1,6-dihydropyrimidin-1-yl)acetamide (150 mg, 0.283 mmol) was suspended in 0.05 M potassium phosphate buffer (pH 7.4, 15.0 ml) and Triton X-100 (trademark)(2.00 µl) and aqueous penicillin amidase solution (Sigma) were added. The mixture was stirred at 37°C for 5 days. The precipitate of the obtained reaction mixture was collected by filtration, washed and vacuum dried to give the title compound as crystals (114 mg, 0.277 mmol).
¹H-NMR (CDCl₃) δ: 0. 84 (3H, d, J=6.8Hz), 1.05(3H, d, J=6.82Hz), 2.45-2.50(1H, m), 4.04(2H, s), 4.58(1H, d, J=15.5Hz), 4.66(1H, d, J=15.5Hz), 5.64(1H, dd, J1=8.9Hz, J2=4.8Hz), 6.88(1H, d, J=8.9Hz), 7.40-7.52(6H,m), 7.72(1H, d, J=3.1Hz), 8.03(1H, d, J=3.1Hz)
MS(ESI) m/z [MH]+ 412.2, [MH]- 410.0

### Example 29

### N-[2-(5-tert-butyl-1,3,4-oxadiazol-2-yl)-1-methyl-2-oxoethyl]-(5-amino-6-oxo-2-phenyl-1,6-dihydropyrimidin-1-yl)acetamide

N-[2-(5-tert-Butyl-1,3,4-oxadiazol-2-yl)-1-methyl-2-oxoethyl]-(6-oxo-2-phenyl-5-phenylacetylamino-1,6-dihydropyrimidin-1-yl)acetamide (150 mg, 0.277 mmol) was suspended in 0.05 M potassium phosphate buffer (pH 7.4, 15.0 ml) and Triton X-100 (trademark) (2.00 µl) and aqueous penicillin amidase solution (Sigma) were added. The mixture was stirred overnight at 37°C. The precipitate pf the obtained reaction mixture was collected by filtration, washed and vacuum dried to give the title compound as crystals (114 mg, 0.269 mmol) .
¹H-NMR(CDCl₃) δ: 1.48(9H, s), 1.57(3H, d, J=7.3Hz), 4.48(1H, d, J=15.5Hz), 4.64(1H, d, J=15.5Hz), 4.47 (1H, m), 7.01 (1H, d, J=6.6Hz), 7.43-7.53(6H, m)
MS(ESI) m/z [MH]+ 425.3, [MH]- 423.1

### Example 30

### methyl (S)-2-[(5-amino-6-oxo-2-phenyl-1,6-dihydropyrimidin-1-yl)acetylamino]-3-methylbutyrate hydrochloride

Methyl (S)-2-[(5-acetylamino-6-oxo-2=phenyl-1,6-dihydropyrimidin-1-yl)acetylamino]-3-methylbutyrate (100 mg, 0.25 mmol) was dissolved in methanol (5 ml) and a 10% solution (0.5 ml) of hydrogen chloride in methanol was added. The mixture was stirred overnight at room temperature. The reaction mixture was concentrated to dryness and methyl-tert-butyl ether was added. The precipitate was filtered, washed with methyl-tert-butyl ether and dried to give the title compound as crystals (90.0 mg, 0.228 mmol).
¹H-NMR(DMSO-d₆) δ: 0. 80 (3H, d, J=3.0Hz), 0. 82 (3H, d, J=3.0Hz), 1.97-2.02 (1H, m), 3.64(3H, s), 4.20 (1H, dd, J1=8.4Hz, J2=6.2Hz), 4.53(2H, s), 7.37(1H, s), 7.48-7.55 (5H, m), 8.51 (1H, d, J=8.4Hz)
MS(ESI) m/z [MH]+ 359.4, [MH]- 393.1

### Example 31

### N-[2-methyl-1-(2-thiazolylcarbonyl)propyl]-(5-amino-6-oxo-2-phenyl-1,6-dihydropyrimidin-1-yl)acetamide hydrochloride

N-[2-Methyl-1-(2-thiazolylcarbonyl)propyl]-(5-acetylamino-6-oxo-2-phenyl-1,6-dihydropyrimidin-1-yl)acetamide (100 mg, 0.220 mmol) was dissolved in methanol (5 ml) and a 10% solution (0.5 ml) of hydrogen chloride in methanol was added. The mixture was stirred overnight at 60°C. The reaction mixture was concentrated to dryness and methyl-tert-butyl ether was added. The precipitate was filtered, washed with methyl-tert-butyl ether and dried to give the title compound as crystals (93.0 mg, 0.208 mmol).
¹H-NMR(DMSO-d₆) δ: 0.80 (3H, d, J=6.9Hz) , 0. 87 (3H, d, J=6.9Hz), 2.28-2.33(1H, m), 4.62(2H, s), 5.45(1H, dd, J1=8.1Hz, J2=5.5Hz), 7.51- 7.62(6H,m), 8.20(1H, d, J=3.0Hz), 8.30(1H, d, J=3.0Hz), 8.75(1H, d, J=8.1Hz)
MS(ESI) m/z [MH]+ 412.3, [MH]- 446.2

### Example 32

### (S)-N-[1-benzyl-3-chloro-2-oxopropyl]-(5-amino-6-oxo-2-phenyl-1,6-dihydropyrimidin-1-yl)acetamide hydrochloride

In the same manner as in Example 30 except that (S)-N-[1-benzyl-3-chloro-2-oxopropyl]-(5-acetylamino-6-oxo-2-phenyl-1,6-dihydropyrimidin-1-yl)acetamide was used instead of methyl (S)-2-[(5-acetylamino-6-oxo-2-phenyl-1,6-dihydropyrimidin-1-yl)acetylamino]-3-methylbutyrate, the title compound was obtained.
¹H-NMR(DMSO-d₆) δ: 2.78-2.84 (1H, m), 3.07-3.11 (1H, m), 4.43(2H, s), 4.55-4.60(3H, m), 7.13-7.23 (5H, m), 7.43-7.58(5H, m), 7.59-7.60(1H, m), 8.96(1H, d, J=7.4Hz)
MS(ESI) m/z [MH]+ 425.2, [MH]- 459.2

### Comparative Example 1

To a solution of 4-N,N-dimethylaminomethylene-2-benzyl-5-oxazolinone (242 mg, 1.05 mmol) in ethanol (8 ml) was added sodium hydroxide (50 mg, 1.25 mmol) under ice-cooling and the mixture was stirred at room temperature for 2 hr. The solvent was evaporated, water was added and the mixture was neutralized with 2N hydrogen chloride. After the completion of the reaction and after neutralization, the mixture was subjected to HPLC, MS analyses to find a ring open form, ethyl 2-phenylacetylamino-3-N,N-dimethylaminopropionate.

The present invention provides a novel compound represented by the formula (IV) and an efficient production method thereof, and further provides a method of advantageously producing the final object compound (XV) or an N-protected form thereof, which is a useful enzyme inhibitor, using the compound (IV) as a starting material compound or an intermediate compound.

In compound (VI) produced via the novel compound (IV) of the present invention, the 5-position amino group of a pyrimidine ring is protected by a protecting group that can be eliminated under mild conditions. Therefore, the protecting group can be deprotected easily without inducing decomposition of the product and the like in the final step to reach compound (XV) or an N-protected form thereof.

Therefore, unlike the conventional methods using an intermediate compound wherein the 5-position amino group of a pyrimidine ring is protected by a benzoyl group, the production method of the present invention is free of a need to eliminate a protecting group prior to amidation reaction, and as a result, suppresses side reactions caused by amidation reaction with the 5-position amino group of a pyrimidine ring being in a free form, such as dimerization and the like, and the object compound having high quality can be obtained in a high yield.

## Claims

1. A production method of a compound represented by the formula (IV) : 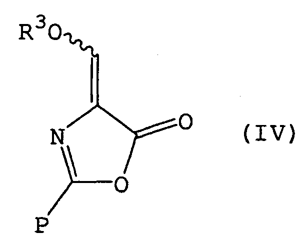 wherein P is a hydrogen atom, an alkyl group, an alkenyl group, an aralkyl group optionally having substituent(s) or a haloalkyl group, R³ is an alkyl group optionally having substituent(s), an aryl group optionally having substituent(s), an aralkyl group optionally having substituent(s), trialkylsilyl group, -COR^{4a} or -SO₂R^{4b} wherein R^{4a} and R^{4b} are the same or different and each independently is an alkyl group optionally having substituent(s) or an aryl group optionally having substituent(s) and a wavy line shows a cis form, a trans form or a mixture thereof,
which comprises hydrolyzing a compound represented by the formula (I): 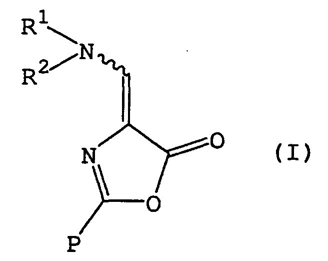 wherein R¹ and R² are the same or different and each is an alkyl group or optionally form an aliphatic heterocycle together with the adjacent nitrogen atom and a wavy line and P are as defined above, or a salt thereof, in the presence of an alkali metal hydroxide to give a compound represented by the formula (II): 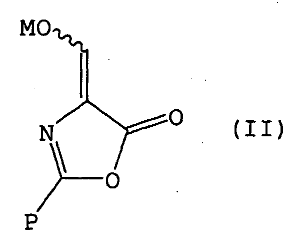 wherein M is a hydrogen atom, sodium, potassium or lithium and P and a wavy line are as defined above; and reacting the obtained compound represented by the formula (II) with a reagent represented by the formula: R³-X (III) wherein R³ is as defined above and X is a halogen atom, -OSO₃R⁴ or -OCOR^{4a}, wherein R⁴ is an alkyl group optionally having substituent(s) or an aryl group optionally having substituent(s) and R^{4a} is as defined above.

2. A production method of a compound represented by the formula (VI): 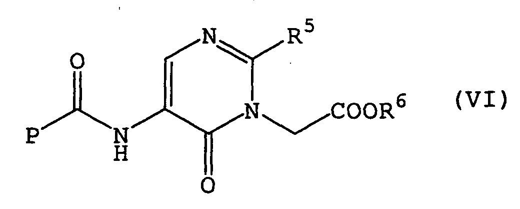 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s) and R⁶ is a hydrogen atom, an alkyl group or an aralkyl group, or a salt thereof, which comprises the following Steps (a) , (b) and (c) :
Step (a): hydrolyzing a compound represented by the formula (I): 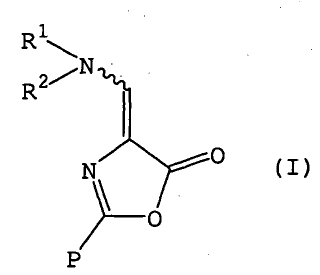 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 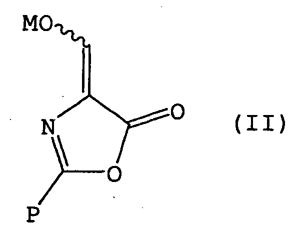 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 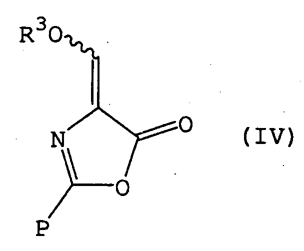 wherein each symbol and a wavy line are as defined above;
Step (c): reacting the obtained compound represented by the formula (IV) with a compound represented by the formula (V): 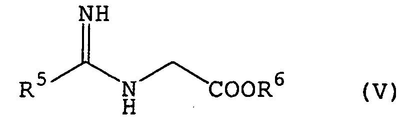 wherein each symbol is as defined above, or a salt thereof, to give said compound represented by the formula (VI) or a salt thereof.

3. A production method of a compound represented by the formula (VII): 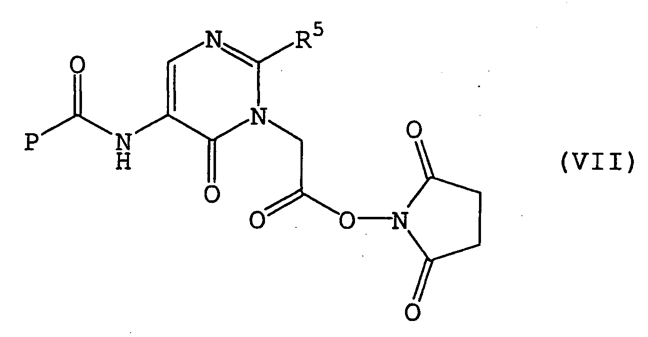 wherein P is a hydrogen atom, an alkyl group, an alkenyl group, an aralkyl group optionally having substituent(s) or a haloalkyl group and R⁵ is an alkyl group optionally having substituent(s) or a phenyl group optionally having substituent(s), which comprises the following Steps (a), (b), (c) and (d);
Step (a): hydrolyzing a compound represented by the formula (I): 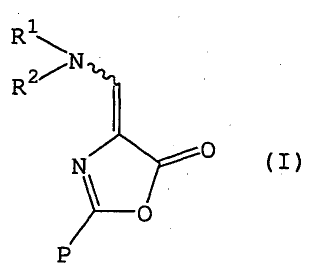 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 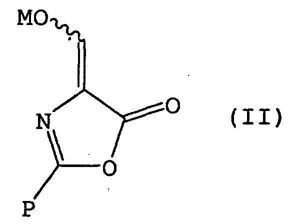 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 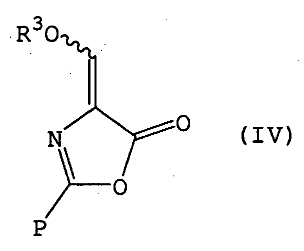 wherein each symbol and a wavy line are as defined above;
Step (c): reacting the obtained compound represented by the formula (IV) with a compound represented by the formula (V): 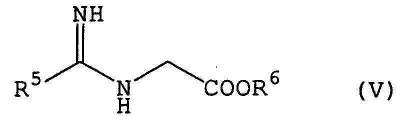 wherein R⁵ is as defined above and R⁶ is a hydrogen atom, an alkyl group or an aralkyl group, or a salt thereof, to give a compound represented by the formula (VI): 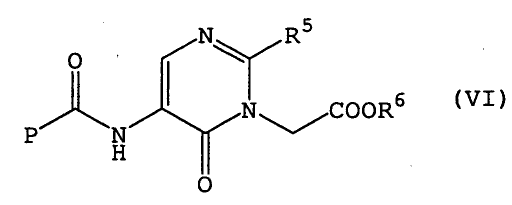 wherein each symbol is as defined above, or a salt thereof; Step (d): when R⁶ of the obtained compound represented by the formula (VI) is other than a hydrogen atom, converting R⁶ to a hydrogen atom, and condensing the compound or a salt thereof with N-hydroxysuccinimide to give said compound represented by the formula (VII).

4. The production method of any of claims 1 to 3, wherein P is a methyl group, an ethyl group or a benzyl group.

5. The production method of any of claims 1 to 3, wherein R³ is a methyl group, an ethyl group, a benzyl group, a methanesulfonyl group or a trimethylsilyl group.

6. A production method of a compound represented by the formula (IX): 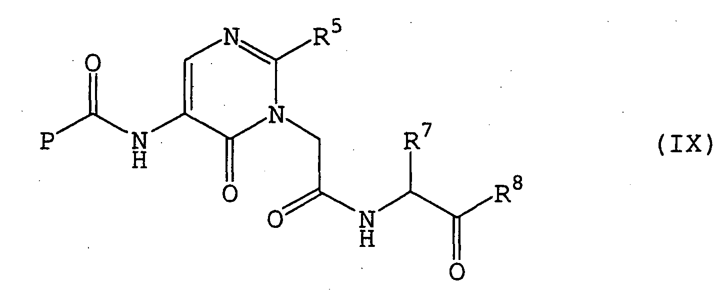 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, which comprises the following Steps (a) , (b) , (c) , (d) and (e);
Step (a): hydrolyzing a compound represented by the formula (I): 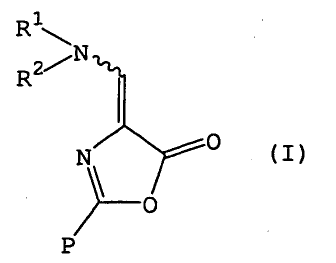 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 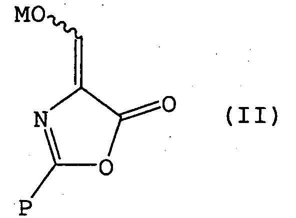 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each is an alkyl group optionally having substituent(s) or an aryl group optionally having substituent(s), to give a compound represented by the formula (IV) : 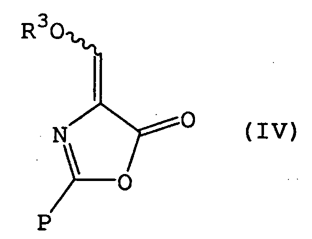 wherein each symbol and a wavy line are as defined above;
Step (c): reacting the obtained compound represented by the formula (IV) with a compound represented by the formula (V): 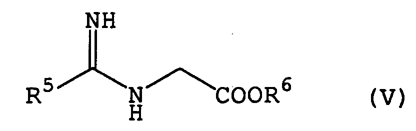 wherein R⁵ is as defined above and R⁶ is a hydrogen atom, an alkyl group or an aralkyl group, or a salt thereof, to give a compound represented by the formula (VI): 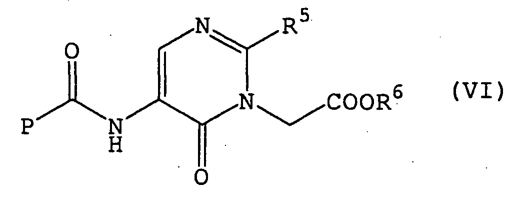 wherein each symbol is as defined above, or a salt thereof; Step (d): when R⁶ of the obtained compound represented by the formula (VI) is other than a hydrogen atom, converting R⁶ to a hydrogen atom, and condensing the compound or a salt thereof with N-hydroxysuccinimide to give a compound represented by the formula (VII): 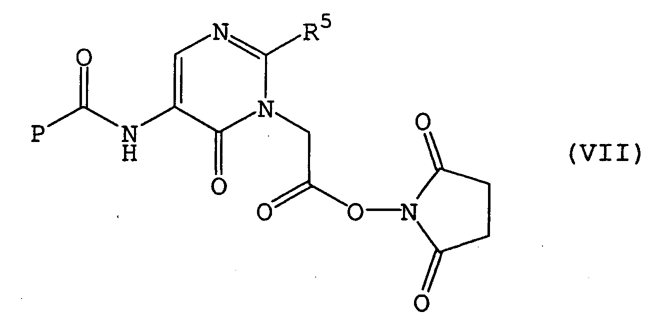 wherein each symbol is as defined above;
Step (e): reacting the obtained compound represented by the formula (VII) with a compound represented by the formula (VIII) : 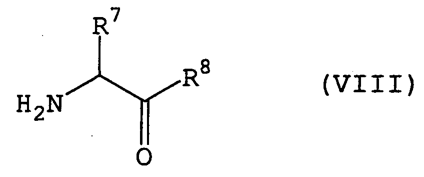 wherein each symbol is as defined above, or a salt thereof, to give said compound represented by the formula (IX) or a salt thereof.

7. A production method of a compound represented by the formula (IX): 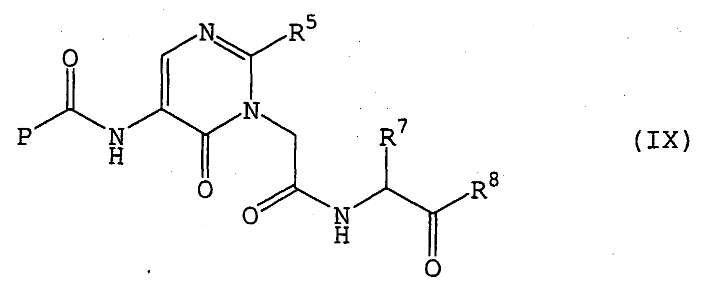 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, which comprises the following Steps (a), (b), (c) and (f) ;
Step (a): hydrolyzing a compound represented by the formula (I): 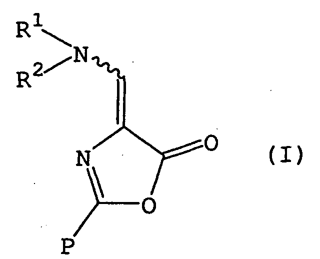 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 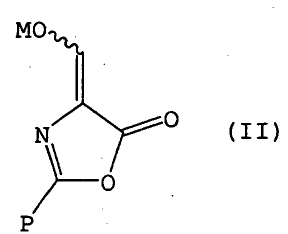 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 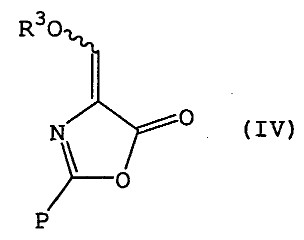 wherein each symbol and a wavy line are as defined above;
Step (c): reacting the obtained compound represented by the formula (IV) with a compound represented by the formula (V): 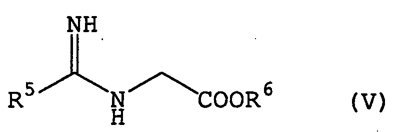 wherein R⁵ is as defined above and R⁶ is a hydrogen atom, an alkyl group or an aralkyl group, or a salt thereof, to give a compound represented by the formula (VI): 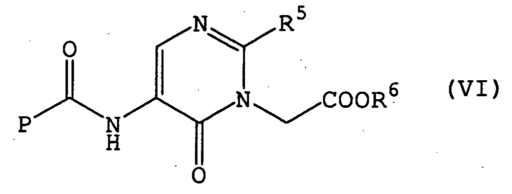 wherein each symbol is as defined above, or a salt thereof; Step (f): when R⁶ of the obtained compound represented by the formula (VI) is other than a hydrogen atom, converting R⁶ to a hydrogen atom, and condensing the compound or a salt thereof with a compound represented by the formula (VIII): 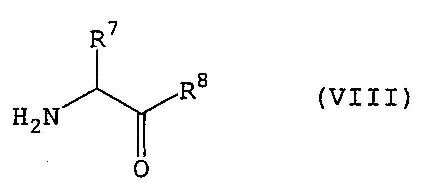 wherein each symbol is as defined above, or a salt thereof, to give said compound represented by the formula (IX) or a salt thereof.

8. The production method of claim 6 or 7, wherein P is a methyl group, an ethyl group or a benzyl group.

9. The production method of claim 6 or 7, wherein R³ is a methyl group, an ethyl group, a benzyl group, a methanesulfonyl group or a trimethylsilyl group.

10. A production method of a compound represented by the formula (X): 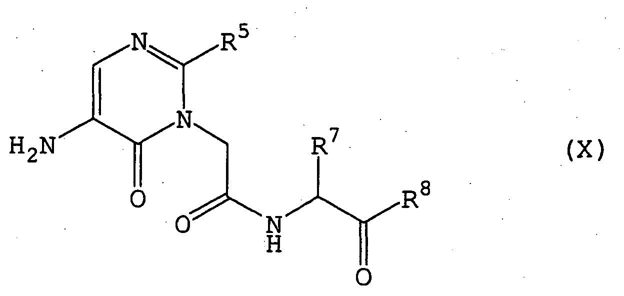 wherein R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, which comprises deprotecting a compound represented by the formula (IX) : 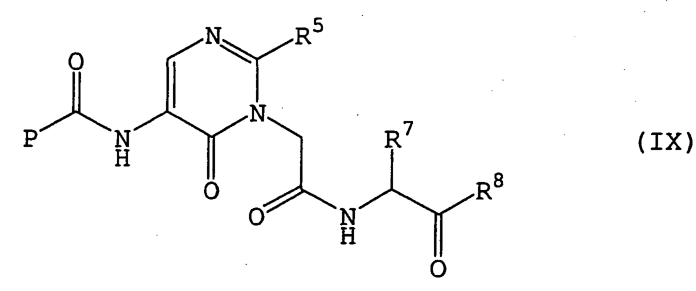 wherein P is a hydrogen atom, an alkyl group, an alkenyl group, an aralkyl group optionally having substituent(s) or a haloalkyl group and other symbols are as defined above, or a salt thereof, obtained by a production method of any of claims 6 to 9.

11. The production method of claim 10, which comprises deprotection by an enzyme reaction.

12. The production method of claim 10, which comprises deprotection under acidic conditions.

13. A production method of a compound represented by the formula (XV): 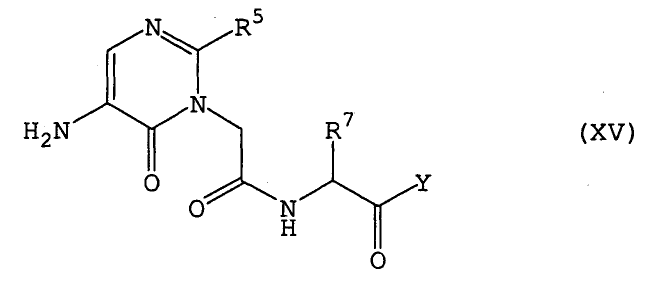 wherein R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and Y is a heterocyclic group optionally having substituent(s), an acyl group optionally having substituent(s) or a haloalkyl group, or a salt thereof,
which comprises the following Steps (j), (k) and (l);
Step (j): protecting an amino group of a compound represented by the formula (X): 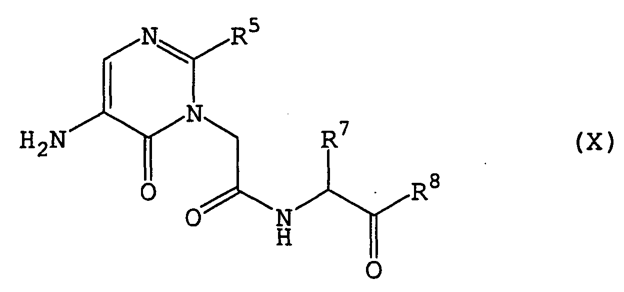 wherein R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, obtained by a method of any of claims 10 to 12 to give a compound represented by the formula (XIII): 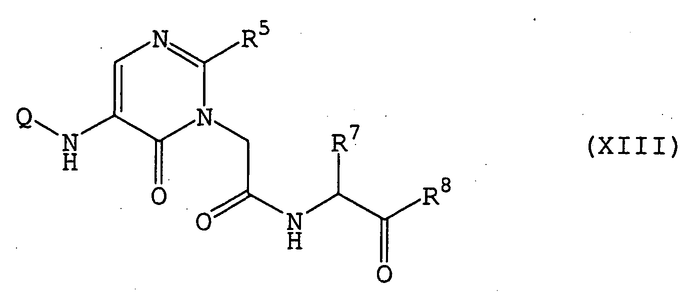 wherein Q is an amino-protecting group other than an acyl group and other symbols are as defined above, or a salt thereof;
Step (k): converting a group represented by R⁸ of the obtained compound represented by the formula (XIII) or a salt thereof to a group represented by Y to give a compound represented by the formula (XIV): 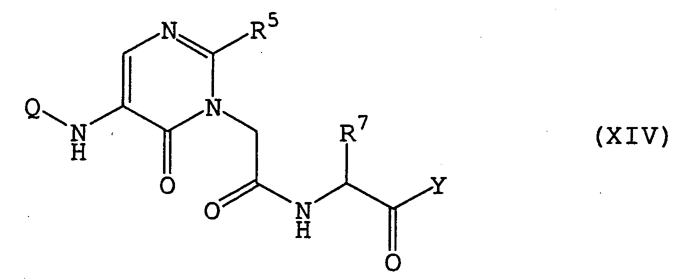 wherein each symbol is as defined above, or a salt thereof; Step (1): deprotecting the obtained compound represented by the formula (XIV) or a salt thereof to give said compound represented by the formula (XV) or a salt thereof.

14. A production method of a compound represented by the formula (XI): 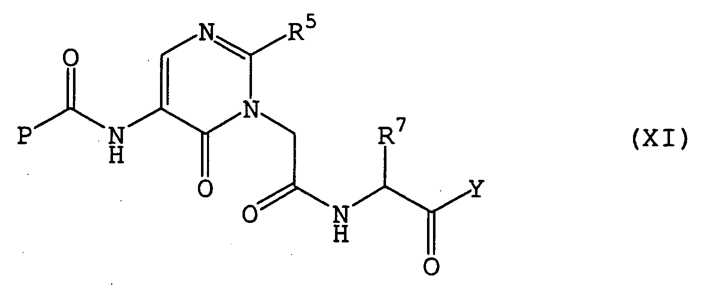 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and Y is a heterocyclic group optionally having substituent(s), an acyl group optionally having substituent(s) or a haloalkyl group, or a salt thereof, which comprises the following Steps (a), (b), (c), (d), (e) and (g);
Step (a): hydrolyzing a compound represented by the formula (I) : 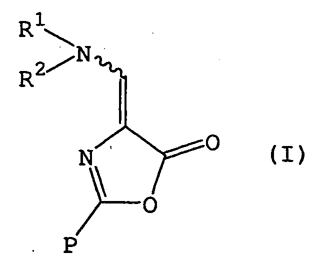 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 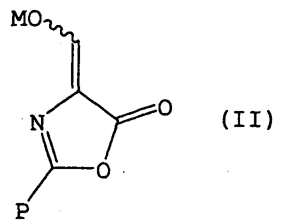 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 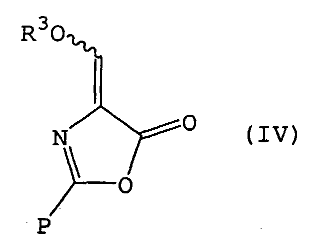 wherein each symbol and a wavy line are as defined above;
Step (c): reacting the obtained compound represented by the formula (IV) with a compound represented by the formula (V): 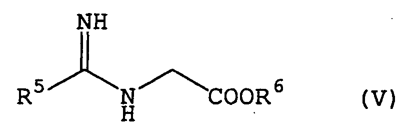 wherein R⁵ is as defined above and R⁶ is a hydrogen atom, an alkyl group or an aralkyl group, or a salt thereof, to give a compound represented by the formula (VI): 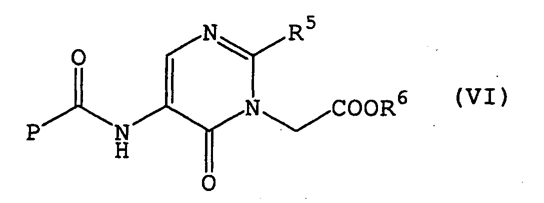 wherein each symbol is as defined above, or a salt thereof;
Step (d): when R⁶ of the obtained compound represented by the formula (VI) is other than a hydrogen atom, converting R⁶ to a hydrogen atom, and condensing the compound or a salt thereof with N-hydroxysuccinimide to give a compound represented by the formula (VII) : 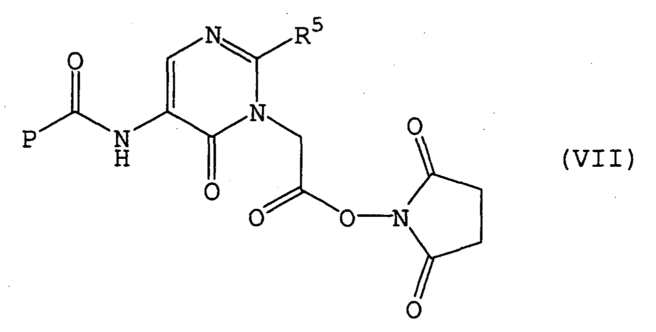 wherein each symbol is as defined above;
Step (e): reacting the obtained compound represented by the formula (VII) with a compound represented by the formula (VIII): 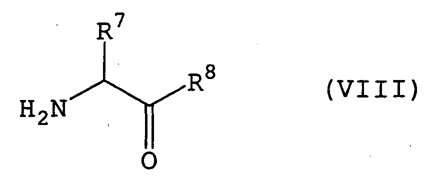 wherein R⁷ is as defined above and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, to give a compound represented by the formula (IX): 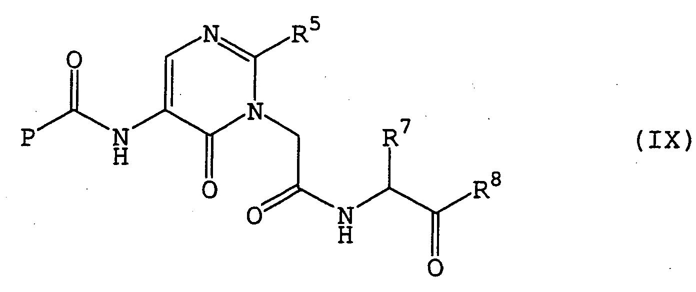 wherein each symbol is as defined above, or a salt thereof;
Step (g): converting a group represented by R⁸ of the obtained compound represented by the formula (IX) or a salt thereof to a group represented by Y to give said compound represented by the formula (XI) or a salt thereof.

15. A production method of a compound represented by the formula (XI): 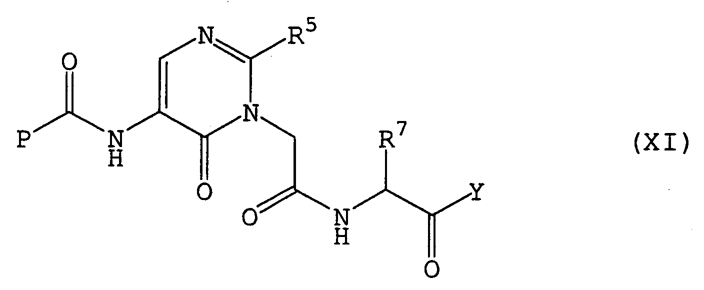 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and Y is a heterocyclic group optionally having substituent(s), an acyl group optionally having substituent(s) or a haloalkyl group, or a salt thereof, which comprises the following Steps (a), (b), (c), (f) and (g);
Step (a): hydrolyzing a compound represented by the formula (I): 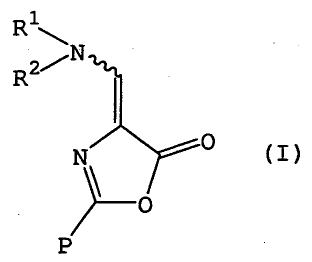 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 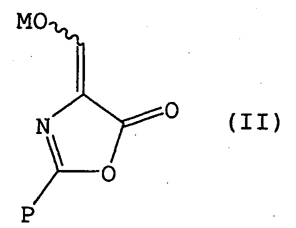 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 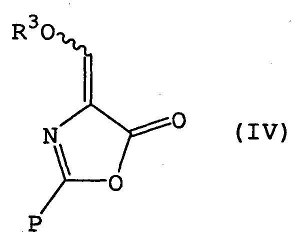 wherein each symbol and a wavy line are as defined above;
Step (c): reacting the obtained compound represented by the formula (IV) with a compound represented by the formula (V): 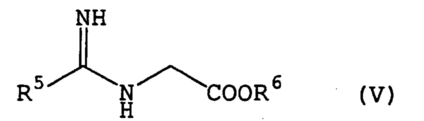 wherein R⁵ is as defined above and R⁶ is a hydrogen atom, an alkyl group or an aralkyl group, or a salt thereof, to give a compound represented by the formula (VI): 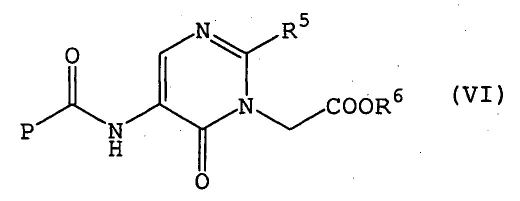 wherein each symbol is as defined above, or a salt thereof;
Step (f): when R⁶ of the obtained compound represented by the formula (VI) is other than a hydrogen atom, converting R⁶ to a hydrogen atom, and condensing the compound or a salt thereof with a compound represented by the formula (VIII): 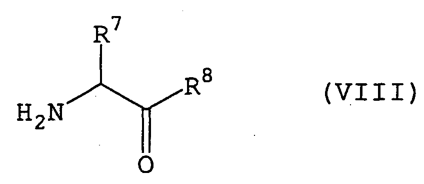 wherein R⁷ is as defined above and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, to give a compound represented by the formula (IX): 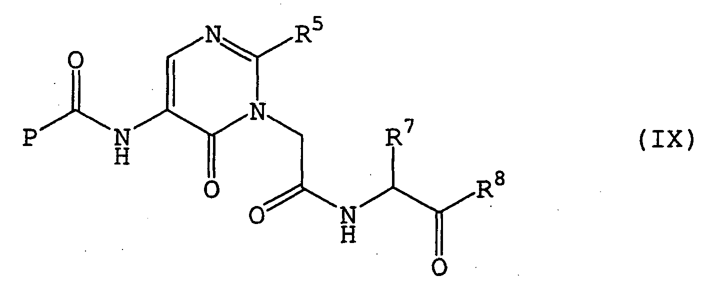 wherein each symbol is as defined above, or a salt thereof;
Step (g): converting a group represented by R⁸ of the obtained compound represented by the formula (IX) or a salt thereof to a group represented by Y to give said compound represented by the formula (XI) or a salt thereof.

16. A production method of a compound represented by the formula (XI): 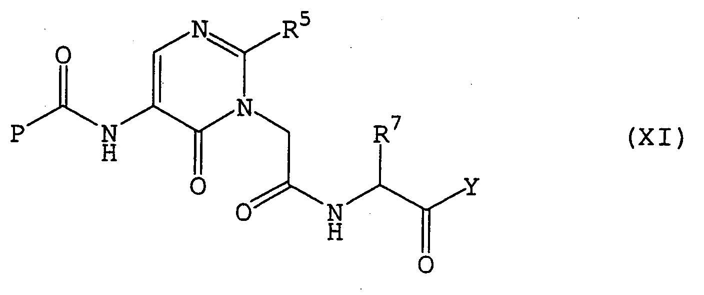 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and Y is a heterocyclic group optionally having substituent(s), an acyl group optionally having substituent(s) or a haloalkyl group, or a salt thereof, which comprises the following Steps (a), (b), (c) and (h);
Step (a): hydrolyzing a compound represented by the formula (I): 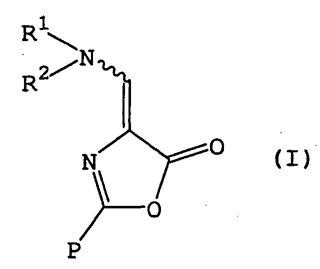 wherein R¹ and R² are the same or different and each is an alkyl group or optionally form an aliphatic heterocycle together with the adjacent nitrogen atom, a wavy line shows a cis form, a trans form or a mixture thereof and P is as defined above, or a salt thereof, in the presence of an alkali metal hydroxide to give a compound represented by the formula (II): 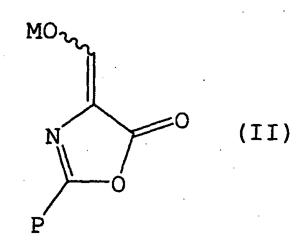 wherein M is a hydrogen atom, sodium, potassium or lithium and P and a wavy line are as defined above;
Step (b): reacting the obtained compound represented by the formula (II) with a reagent represented by the formula: R³-X (III) wherein R³ is an alkyl group optionally having substituent(s), an aryl group optionally having substituent(s), an aralkyl group optionally having substituent(s), trialkylsilyl group, -COR^{4a} or -SO₂R^{4b} and X is a halogen atom, -OSO₃R⁴ or -OCOR^{4a} wherein R⁴, R^{4a} and R^{4b} are the same or different and each independently is an alkyl group optionally having substituent(s) or an aryl group optionally having substituent(s), to give a compound represented by the formula (IV) : 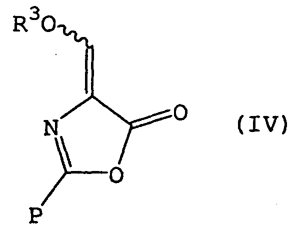 wherein each symbol and a wavy line are as defined above;
Step (c): reacting the obtained compound represented by the formula (IV) with a compound represented by the a compound represented by the formula (V): 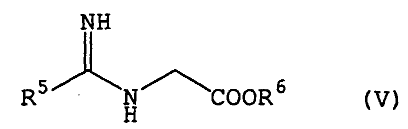 wherein R⁵ is as defined above and R⁶ is a hydrogen atom, an alkyl group or an aralkyl group, or a salt thereof, to give a compound represented by the formula (VI): 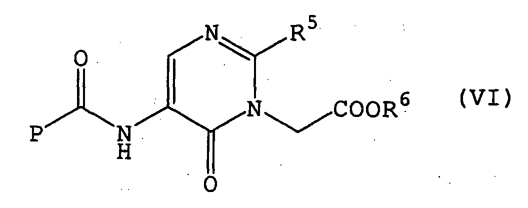 wherein each symbol is as defined above, or a salt thereof;
Step (h): when R⁶ of the obtained compound represented by the formula (VI) is other than a hydrogen atom, converting R⁶ to a hydrogen atom, and condensing the compound or a salt thereof with a compound of the formula (XII): 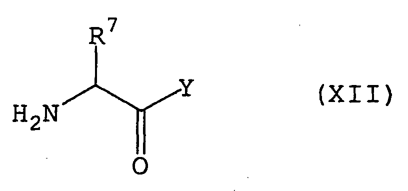 wherein each symbol is as defined above, or a salt thereof, to give said compound represented by the formula (XI) or a salt thereof.

17. The production method of any of claims 14 to 16, wherein P is a methyl group, an ethyl group or a benzyl group.

18. The production method of any of claims 14 to 16, wherein R³ is a methyl group, an ethyl group, a benzyl group, a methanesulfonyl group or a trimethylsilyl group.

19. A production method of a compound represented by the formula (XV): 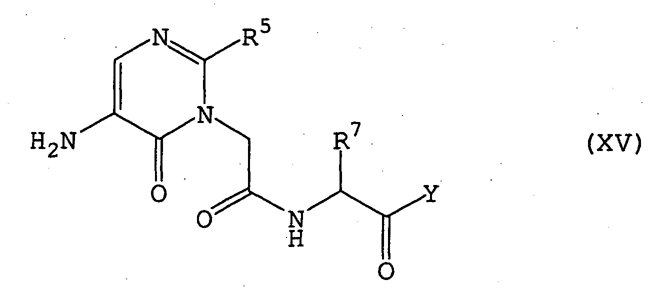 wherein R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and Y is a heterocyclic group optionally having substituent(s), an acyl group optionally having substituent(s) or a haloalkyl group, or a salt thereof, which comprises deprotecting a compound represented by the formula (XI) : 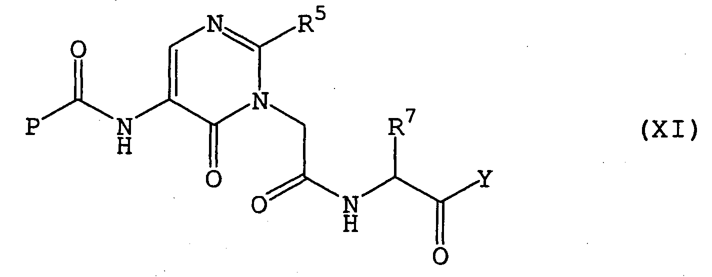 wherein P is a hydrogen atom, an alkyl group, an alkenyl group, an aralkyl group optionally having substituent(s) or a haloalkyl group, and other symbols are as defined above, or a salt thereof, obtained by a production method of any of claims 14 to 18.

20. The production method of claim 19, which comprises deprotection by an enzyme reaction.

21. The production method of claim 19, which comprises deprotection under acidic conditions.

22. A compound represented by the formula (IV'): 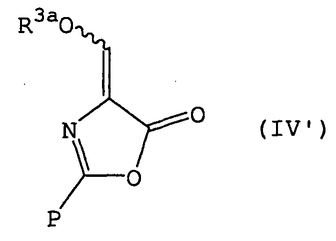 wherein P is a hydrogen atom, an alkyl group, an alkenyl group, an aralkyl group optionally having substituent(s) or a haloalkyl group, R^{3a} is an alkyl group optionally having substituent(s), provided that P is not an alkyl group and a benzyl group, an aryl group optionally having substituent(s), an aralkyl group optionally having substituent(s), a trialkylsilyl group, -COR^{4a} or -SO₂R^{4b} wherein R^{4a} and R^{4b} are the same or different and each independently is an alkyl group optionally having substituent(s) or an aryl group optionally having substituent(s) and a wavy line shows a cis form, a trans form or a mixture thereof.

23. The compound of claim 22, wherein P is a methyl group, an ethyl group or a benzyl group and R^{3a} is a methyl group, an ethyl group, a benzyl group, a methanesulfonyl group or a trimethylsilyl group.

24. A compound represented by the formula (VII): 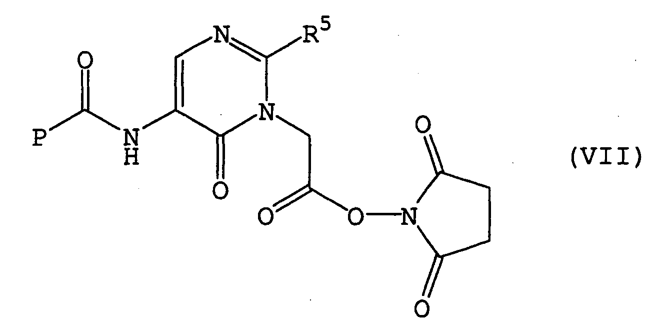 wherein P is a hydrogen atom, an alkyl group, an alkenyl group, an aralkyl group optionally having substituent(s) or a haloalkyl group and R⁵ is an alkyl group optionally having substituent(s) or a phenyl group optionally having substituent(s).

25. The compound of claim 24, wherein P is a methyl group, an ethyl group or a benzyl group, and R⁵ is a methyl group or a phenyl group optionally having substituent(s).

26. A compound represented by the formula (IX): 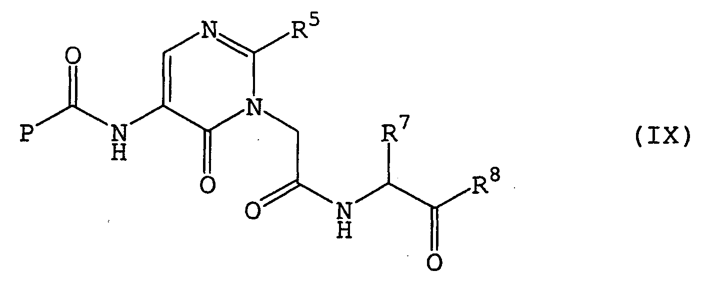 wherein P is a hydrogen atom, an alkyl group, an alkenyl group, an aralkyl group optionally having substituent(s) or a haloalkyl group, R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof.

27. The compound claim 26, wherein P is a methyl group, an ethyl group or a benzyl group, R⁵ is a methyl group or a phenyl group optionally having substituent(s), R⁷ is a methyl group, an isopropyl group or a benzyl group, and R⁸ is a hydrogen atom, a hydroxyl group, a methoxy group, an ethoxy group or -N(Me)-OMe, or a salt thereof.

28. A compound represented by the formula (X): 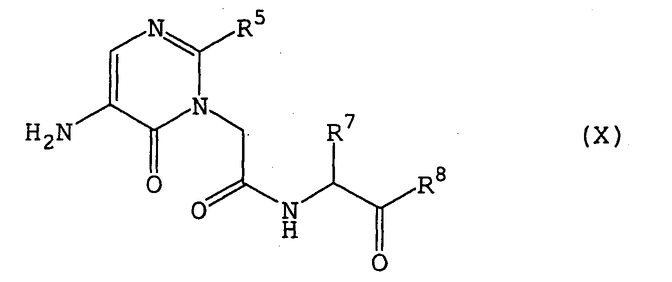 wherein R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof.

29. The compound of claim 28, wherein R⁵ is a methyl group or a phenyl group optionally having substituent(s), R⁷ is a methyl group, an isopropyl group or a benzyl group, R⁸ is a hydrogen atom, a hydroxyl group, a methoxy group, an ethoxy group or -N(Me)-OMe, or a salt thereof.

30. A production method of a compound represented by the formula (X): 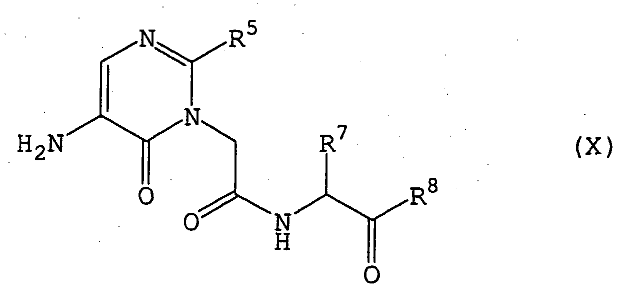 wherein R⁵ is an alkyl group optionally having substituent(s) or a phenyl group optionally having substituent(s), R⁷ is a hydrogen atom, an alkyl group optionally having substituent(s), an aralkyl group optionally having substituent(s) or an aryl group optionally having substituent(s) and R⁸ is a hydrogen atom, a hydroxyl group, an alkoxy group, an aralkyloxy group or -N(R⁹)-OR¹⁰ wherein R⁹ and R¹⁰ are the same or different and each independently is an alkyl group, or a salt thereof, which comprises deprotecting a compound represented by the formula (IX) : 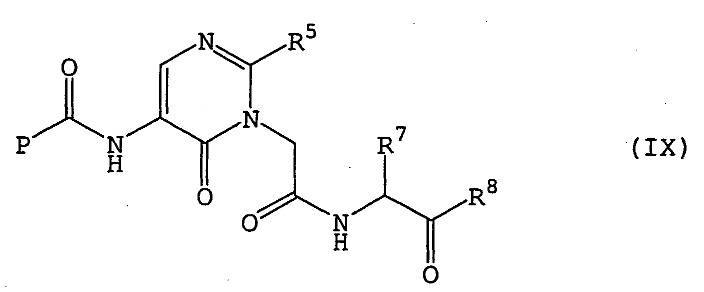 wherein P is a hydrogen atom, an alkyl group, an alkenyl group, an aralkyl group optionally having substituent(s) or a haloalkyl group and other symbols are as defined above, or a salt thereof, by an enzyme reaction.

31. A production method of a compound represented by the formula (XV): 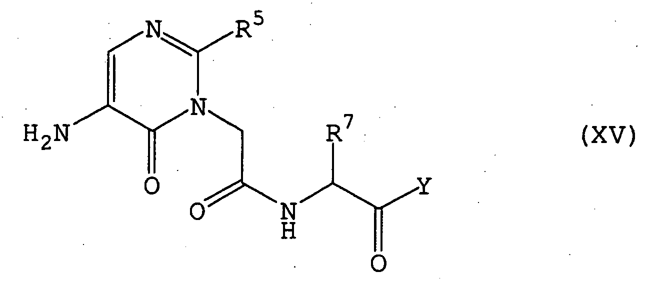 wherein R⁵ is an alkyl group optionally having substituent(s) or a phenyl group optionally having substituent(s), R⁷ is a hydrogen atom, an alkyl group optionally having substituent (s), an aralkyl group optionally having substituent(s) or an aryl group optionally having substituent(s) and Y is a heterocyclic group optionally having substituent(s), an acyl group optionally having substituent(s) or a haloalkyl group, or a salt thereof, which comprises deprotecting a compound represented by the formula (XI): 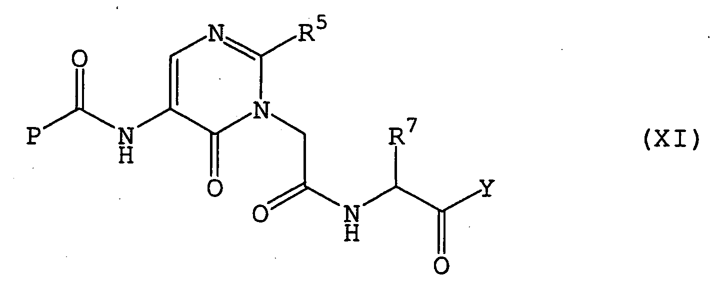 wherein P is a hydrogen atom, an alkyl group, an alkenyl group, an aralkyl group optionally having substituent(s) or a haloalkyl group and other symbols are as defined above, or a salt thereof, by an enzyme reaction.

32. The production method of claim 30 or 31, wherein P is a benzyl group optionally having substituent(s).

33. The production method of claim 30 or 31, wherein the enzyme is penicillin amidase.
